# EUROPEAN PATENT SPECIFICATION

(11) **EP 1 827 108 B1**
(45) Date of publication and mention of the grant of the patent: **08.04.2015**
(21) Application number: 05810540.4
(22) Date of filing: 29.11.2005
(51) Int. Cl.: A01N 63/00, A01N 65/00, A01N 37/18, A61K 38/00

(54) **INDUCTION OF NEUROGENESIS AND STEM CELL THERAPY IN COMBINATION WITH COPOLYMER 1**
INDUKTION DER NEUROGENESE UND STAMMZELLENTHERAPIE IN KOMBINATION MIT COPOLYMER 1
INDUCTION DE NEUROGENESE ET THERAPIE PAR CELLULES SOUCHES EN COMBINAISON AVEC LE COPOLYMERE 1

(30) Priority: 29.11.2004 US 631163 P; 15.06.2005 US 690498 P
(43) Date of publication of application: 05.09.2007
(73) Proprietor: YEDA RESEARCH AND DEVELOPMENT CO., LTD., 76100 Rehovot (IL)
(72) Inventor: EISENBACH-SCHWARTZ, Michal, 76100 Rehovot (IL); ARNON, Ruth, 76100 Rehovot (IL); BUTOVSKY, Oleg, Boston, MA 02215 (US); ZIV, Yaniv, Givatayim, 53419 (IL); KIPNIS, Jonathan, 71700 Modiin (IL); RON, Noga, 37861 Katzir (IL); EILAM, Raya, 93117 Jerusalem (IL); AHARONI, Rina, 76346 Rehovot (IL)
(74) Representative: Vossius & Partner
(86) International application number: PCT/IL2005/001275
(87) International publication number: WO 2006/057003

(56) References cited:
- US-A1- 2002 037 848
- US-A1- 2003 003 082
- US-A1- 2004 248 802
- US-B2- 6 844 314
- BENNER E J ET AL: "COPAXONE IMMUNIZATION PROTECTS DOPAMINERGIC NEURONS IN AN MPTP MOUSE MODEL OF PARKINSON'S DISEASE" ANNUAL MEETING OF THE SOCIETY OF NEUROSCIENCE, XX, XX, vol. 2003, 8 November 2003 (2003-11-08), page 440.01, XP001205929
- BENNER E J ET AL: "THERAPEUTIC IMMUNIZATION PROTECTS DOPAMINERGIC NEURONS IN A MOUSE MODEL OF PARKINSON'S DISEASE" PROCEEDINGS OF THE NATIONAL ACADEMY OF SCIENCES OF USA, NATIONAL ACADEMY OF SCIENCE, WASHINGTON, DC, US, vol. 101, no. 25, 22 June 2004 (2004-06-22), pages 9435-9440, XP001205934 ISSN: 0027-8424
- AHARONI RINA ET AL: "Neurogenesis and neuroprotection induced by peripheral immunomodulatory treatment of experimental autoimmune encephalomyelitis" JOURNAL OF NEUROSCIENCE, vol. 25, no. 36, September 2005 (2005-09), pages 8217-8228, XP002544640 ISSN: 0270-6474
- JOSHI D ET AL: "Neuronal stem cells." NEUROLOGY INDIA SEP 2003, vol. 51, no. 3, September 2003 (2003-09), pages 323-328, XP002544641 ISSN: 0028-3886
- SILANI V. ET AL.: 'Stem-cell therapy for amyotrophic lateral sclerosis' LANCET vol. 364, no. 9429, 2004, pages 200 - 202, XP005070196
- CHEN ET AL.: 'GLATIRAMER ACETATE-REACTIVE T CELLS PRODUCE BRAIN-DERIVED NEUROTROPHIC FACTOR' J. NEUROL. SCIENCE vol. 215, 2003, pages 37 - 44, XP008076627
- ZIEMSSEN ET AL.: 'Glatiramer acetate-specific T-helper 1- and 2-type cell lines produce BDNF: implications for multiple sclerosis therapy' BRAIN vol. 125, 2002, pages 2381 - 2391, XP003007488
- MURARO ET AL.: 'Using stem cells multiple sclerosis therapies' CYTOTHERAPY vol. 6, no. 6, 2004, pages 615 - 620, XP008076596
- KIPNIS ET AL.: 'DUAL ACTION OF GLATIRAMER ACETATE (COP-1) IN THE TREATMENT OF CNS AUTOIMMUNE AND NEURODEGENERATIVE DISORDERS' TRENDS IN MOL. MED. vol. 8, 2002, pages 319 - 323, XP001205939
- KREITMAN ET AL.: 'ON THE HORIZON: POSSIBLE NEUROPROTECTIVE ROLE FOR GLATIRAMER ACETATE' MULTIPLE SCLEROSIS vol. 10, 2004, pages S81 - S89, XP009045420
- PENCEA ET AL.: 'Infusion of Brain-Derived Neurotrophic Factor into the Lateral Ventricle of the Adult Rat Leads to New Neurons in the Parenchyma of the Striatum, Septum, Thalamus, and Hypothalamus' J. NEUROSCI. vol. 21, 2001, pages 6706 - 6717, XP003007489
- MCTIGUE ET AL.: 'Neurotrophin-3 and Brain-Derived Neurotrophic Factor Induce Oligodendrocyte Proliferation and Myelination of Regenerating Axons in the Contused Adult Rat Spinal Cord' J. NEUROSCI. vol. 18, 1998, pages 5354 - 5365, XP003007490

## Description

### FIELD OF THE INVENTION

The present invention relates to methods and compositions, in particular using Copolymer 1, for induction and/or enhancement of endogenous neurogenesis and/or oligodendrogenesis and for stem cell therapy in injuries, diseases, disorders or conditions, in particular those associated with the central nervous system (CNS) or peripheral nervous system (PNS).

**Abbreviations:** Aβ, β-amyloid; AD, Alzheimer's disease; BDNF, brain-derived neurotrophic factor; BMS, Basso motor score; BrdU, 5-bromo-2'-deoxyuridine; CFA, complete Freund's adjuvant; CNS, central nervous system; Cop 1, Copolymer 1, same as GA; DCX, doublecortin; DG, dentate gyrus; EAE, experimental autoimmune encephalomyelitis; EGF, epidermal growth factor; FCS, fetal calf serum; FGF, fibroblast growth factor; i.c.v., intracerebroventricular; GA, glatiramer acetate; GFAP, glial fibrillary acidic protein; GFP, green fluorescent protein; IB4, isolectin B4; IFA, incomplete Freund's adjuvant; IGF-I, insulin-like growth factor 1; IFN, interferon; IL, interleukin; LPS, lipopolysaccharide; MBP, myelin basic protein; MG, microglia; MHC-II, class II major histocompatibility complex molecules; MOG, myelin oligodendrocyte glycoprotein; MS, multiple sclerosis; MWM, Morris water maze; NeuN, neuronal nuclear antigen; NPC, neural stem/progenitor cell; OB, olfactory bulb; PBS, phosphate-buffered saline; PDL, poly-D-lysine; PNS, peripheral nervous system; RMS, rostral migratory stream; SCI, spinal cord injury; SGZ, subgranular zone; SVZ, subventricular zone; TGF-β, transforming growth factor-β; TNF, tumor necrosis factor.

### BACKGROUND OF THE INVENTION

The central nervous system (CNS) is particularly vulnerable to insults that result in cell death or damage in part because cells of the CNS have a limited capacity for repair. Since damaged brain tissue does not regenerate, recovery must come from the remaining intact brain.

Poor recovery from acute insults or chronic degenerative disorders in the CNS has been attributed to lack of neurogenesis, limited regeneration of injured nerves, and extreme vulnerability to degenerative conditions. The absence of neurogenesis was explained by the assumption that soon after birth the CNS reaches a permanently stable state, needed to maintain the equilibrium of the brain's complex tissue network. Research during the last decade showed, however, that the brain is capable of neurogenesis throughout life, albeit to a limited extent (Morshead et al., 1994). In the inflamed brain, neurogenesis is blocked (Ekdahl et al., 2003; Monje et al., 2003). This latter finding strengthened the traditional view that local immune cells in the CNS have an adverse effect on neurogenesis. Likewise, the limited regeneration and excessive vulnerability of CNS neurons under inflammatory conditions or after an acute insult were put down to the poor ability of the CNS to tolerate the immune-derived defensive activity that is often associated with local inflammation and cytotoxicity mediated, for example, by tumor necrosis factor (TNF)-α or nitric oxide (Merrill et al., 1993). More recent studies have shown, however, that although an uncontrolled local immune response indeed impairs neuronal survival and blocks repair processes, a local immune response that is properly controlled can support survival and promote recovery (Hauben and Schwartz, 2003; Schwartz et al., 2003). It was further shown that after an injury to the CNS, a local immune response that is well controlled in time, space, and intensity by peripheral adaptive immune processes (in which CD4⁺ helper T cells are directed against self-antigens residing at the site of the lesion) is a critical requirement for post-traumatic neuronal survival and repair (Moalem et al., 1999; Butovsky et al., 2001; Schwartz et al., 2003; Shaked et al., 2004). These and other results led the inventor M Schwartz and colleagues to formulate the concept of 'protective autoimmunity' (Moalem et al., 1999).

Neurogenesis occurs throughout life in adult individuals, albeit to a limited extent. Most of the newly formed cells die within the first 2-3 weeks after proliferation and only a few survive as mature neurons. Little is known about the mechanism(s) underlying the existence of neural stem/progenitor cells (NPCs) in an adult brain and why these cells are restricted in amount and limited to certain areas. Moreover, very little is known about how neurogenesis from an endogenous NPC pool can be physiologically increased. Knowledge of the factors allowing such stem cells to exist, proliferate, and differentiate in the adult individual is a prerequisite for understanding and promoting the conditions conducive to CNS repair. This in turn can be expected to lead to the development of interventions aimed at boosting neural cell renewal from the endogenous stem-cell pool or from exogenously applied stem cells.

Experiments with rat and mouse models in our laboratory have shown that well-controlled implantation of specifically activated blood-borne macrophages (Rapalino et al., 1998) or dendritic cells (Hauben et al., 2003) promotes recovery from spinal cord injury (SCI). Other studies showed that the well-controlled activity of autoimmune T cells reactive to CNS antigens residing in the lesioned site can promote recovery from axonal insults (Hauben et al., 2000; Moalem et al., 1999). It was also shown that neuroprotection, mediated by T cells directed specifically to CNS-related autoantigens, is the body's physiological response to CNS injury (Yoles et al., 2001a, 2001b).

Under normal conditions in the adult brain, new neurons are formed in the neurogenic niches of the subventricular zone of the lateral ventricles and the subgranular zone of the hippocampal dentate gyrus (Kempermann et al., 2004). Under pathological conditions some neurogenesis can also be induced in non-neurogenic brain areas. Several studies have demonstrated, for example, that injury to the CNS in animals is followed by recruitment of endogenous NPCs, which can undergo differentiation to neurons and glia at the injured site (Nakatomi et al., 2002; Imitola et al., 2004a). However, this injury-triggered cell renewal from endogenous progenitors is limited in extent and is not sufficient for full replacement of the damaged tissue. To overcome the deficit, scientists are currently seeking ways to promote recovery by transplanting cultured adult NPCs (aNPCs) (McDonald et al., 2004). Exogenous aNPCs might contribute to recovery by acting as a source of new neurons and glia in the injured CNS (Cummings et al., 2005; Lepore and Fischer, 2005) or by secreting factors that directly or indirectly promote neuroprotection (Lu et al., 2005) and neurogenesis from endogenous stem-cell pools (Enzmann et al., 2005).

Current opinions concur that neurogenesis persists in the adult brain, where it may contribute to repair and recovery after injury. Brain insults such as cerebral ischemia (Jin et al., 2003), apoptosis (Magavi et al., 2000) or autoimmune inflammatory demyelination (Picard-Riera et al., 2002) enhance neurogenesis. Hence, multipotent cells located in the hippocampus hilus and the subventricular zone (SVZ) of the lateral ventricle manifest increased proliferation and migration in pathological situations. Moreover, progenitor cells from the SVZ that migrate through the rostral migratory stream (RMS) to the olfactory bulb (OB) can be triggered to differentiate into astrocytes and neurons (Picard-Riera et al., 2004). Nevertheless, the therapeutic significance of self-neurogenesis in CNS pathology is limited, as it fails to regenerate functional neurons that compensate the damage.

In multiple sclerosis (MS) and its animal model experimental autoimmune encephalomyelitis (EAE), the immune system provokes the detrimental process via autoimmune inflammatory mechanisms (Hellings et al., 2002; Behi et al., 2005). Still, neuronal and axonal degeneration, initiated at disease onset and revealed when compensatory CNS resources are exhausted, are the major determinant of the irreversible neurological disability (Bjartmar et al., 2003), particularly in the myelin oligodendrocyte glycoprotein (MOG) induced model (Hobom et al., 2003). Current treatments for MS are effective in ameliorating the immune inflammatory process, but their ability to enhance the intrinsic CNS repair mechanism and to induce effective neuroprotection and neurogenesis has not been shown.

A potential approach for treatment of CNS damage includes the use of adult neural stem cells or any type of stem cells. The adult neural stem cells are progenitor cells present in the mature mammalian brain that have the ability of self-renewal and, given the appropriate stimulation, can differentiate into brain neurons, astrocytes and oligodendrocytes. Stem cells (from other tissues) have classically been defined as pluripotent and having the ability to self-renew, to proliferate, and to differentiate into multiple different phenotype lineages. Hematopoietic stem cells are defined as stem cells that can give rise to cells of at least one of the major hematopoietic lineages in addition to producing daughter cells of equivalent potential. Three major lineages of blood cells include the lymphoid lineage, e.g. B-cells and T-cells, the myeloid lineage, e.g. monocytes, granulocytes and megakaryocytes, and the erythroid lineage, e.g. red blood cells. Certain hematopoietic stem cells are capable of differentiating to other cell types, including brain cells.

Transplantation of multipotent (stem) precursor cells is a promising strategy for the therapy of various disorders caused by loss or malfunction of single or few cell types. These include neurological disorders such as spinal cord injury, subcortical neurodegenerations e.g. Huntington and Parkinson, and demyelinatindg diseases e.g. MS, as well as other pathological conditions such as diabetes, myocardial infarction of cardiac failure, tissue injury and insufficient wound healing. Particularly in MS and its animal model EAE, stem cells differentiating into oligodendrocytes and neurons may lead to repair of myelin damage and replace degenerating neurons. However, hitherto stem cell transplantation in these systems resulted in poor therapeutic outcome. Thus, stem cells transplanted as such into EAE mice were found mainly around the injection site (in cases of local administration) or in perivascular position (when systemic administration was employed), and their proliferation, migration and differentiation were insufficient to compensate for the damage inflicted by the disease (Goldman, 2005; Pluchino and Martini, 2005). A clinical trial in which stem cells were transplanted into demyelinating brain areas of MS patients was discontinued in 2003, as no evidence of stem cell survival was found in the implanted patients (Pluchino and Martini, 2005). These outcomes were related to the chronic inflammatory processes that destroy the transplanted as well as the resident cells. It has been suggested that stem cell-based therapeutic strategies, especially those intended for MS, will require disease modification adjuncts to cell delivery. Stem cell therapy is also considered for many other medical applications, not related to neurological disorders.

Separation and cloning of neural stem cell lines from both the murine and human brain have been reported. Human CNS neural stem cells, like their rodent homologues, when maintained in a mitogen-containing (typically epidermal growth factor or epidermal growth factor plus basic fibroblast growth factor), serum-free culture medium, grow in suspension culture to form aggregates of cells known as neurospheres. Upon removal of the mitogens and provision of a substrate, the stem cells differentiate into neurons, astrocytes and oligodendrocytes. When such stem cells are reintroduced into the developing or mature brain, they can undergo through division, migration and growth processes, and assume neural phenotypes, including expression of neurotransmitters and growth factors normally elaborated by neurons. Thus, use of neural stem cells may be advantageous for CNS damage recovery in at least two ways: (1) by the stem cells partially repopulating dead areas and reestablishing neural connections lost by CNS damage, and (2) by secretion of important neurotransmitters and growth factors required by the brain to rewire after CNS damage.

Recently, a renewable source of neural stem cells was discovered in the adult human brain. These cells may be a candidate for cell-replacement therapy for nervous system disorders. The ability to isolate these cells from the adult human brain raises the possibility of performing autologous neural stem cell transplantation. It has been reported that clinical trials with adult human neural stem cells have been initiated for treatment of Parkinson's disease patients. If adult neural stem cells are to be used in clinical trials they must be amenable to expansion into clinically significant quantities. Unfortunately, these cells seem to have a limited life-span in the culture dish and it remains to be determined whether they are stable at later passages and capable of generating useful numbers of neurons.

The brain has long been viewed as an immune-privileged site. However, autoimmune T cells (controlled with respect to the onset, duration, and intensity of their activity) were recently shown to exert a beneficial effect on neuronal survival after CNS injury (Schwartz et al., 2003), as well as in cases of mental dysfunction (Kipnis et al., 2004). Moreover, in-depth understanding of the mechanisms underlying the beneficial effect of T cells for degenerative neural tissue has has pointed out that the T cells instruct the microglia, at the injured area, to acquire a phenotype supportive of neural tissue. In addition, it appeared that several immune-based intervention can boost this protective response, all of which converts to microglial activation (Shaked et al., 2004). The type of damage does not determine the choice of the approach, it is the site which determines it. Some antigens cross-react with numerous antigens and thus can overcome tissue specificity barrier. According to the present invention, we show that the same manipulation that leads to neuronal survival leads to neurogenesis and oligodendrogenesis. It appears that the same microglia, activated by T cells or by their cytokines, not only support neuronal survival but also support oligodendrogenesis and neurogenesis. These results indicate that T-cell-based manipulation will create conditions in damaged neural tissue that favor cell renewal not only from endogenous stem cell resources but also from exogenously applied stem cells.

Copolymer 1 or Cop 1, a non-pathogenic synthetic random copolymer composed of the four amino acids: L-Glu, L-Lys, L-Ala, and L-Tyr. Glatiramer acetate (GA), one form of Cop 1, is currently an approved drug for the treatment of multiple sclerosis under the name of Copaxone® (a trademark of Teva Pharmaceutical Industries Ltd., Petach Tikva, Israel). It exerts a marked suppressive effect on EAE induced by various encephalitogens, in several species (Arnon and Sela, 2003).

Cop 1 is a very well tolerated agent with only minor adverse reactions and high safety profile. Treatment with Cop 1 by ingestion or inhalation is disclosed in US 6,214,791.

Recently it was found that in animal models Cop 1 provides a beneficial effect for several additional disorders. Thus, Cop 1 suppresses the immune rejection manifested in graft-versus-host disease (GVHD) in case of bone marrow transplantation (Schlegel et al., 1996; Aharoni et al., 1997; US 5,858,964), as well as in graft rejection in case of solid organ transplantation (Aharoni et al., 2001, 2004) and in the applicant's patent applications WO 00/27417 and WO/009333A2)

WO 01/52878 and WO 01/93893 of the same applicants disclose that Cop 1, Cop 1-related peptides and polypeptides and T cells activated therewith protect CNS cells from glutamate toxicity and prevent or inhibit neuronal degeneration or promote nerve regeneration in the CNS and in the PNS. Thus, for example, Cop 1 is under evaluation as a therapeutic vaccine for neurodegenerative diseases such as optic neuropathies and glaucoma (Kipnis and Schwartz, 2002).

Cop 1 has been shown to act as a low-affinity antigen that activates a wide range of self-reacting T cells, resulting in neuroprotective autoimmunity that is effective against both CNS white matter and grey matter degeneration (Schwartz and Kipnis, 2002). The neuroprotective effect of Cop 1 vaccination was demonstrated in animal models of acute and chronic neurological disorders such as optic nerve injury (Kipnis et al., 2000), head trauma (Kipnis et al., 2003), glaucoma (Schori et al., 2001; Bakalash et al., 2003), amyotrophic lateral sclerosis (Angelov et al., 2003) and in the applicant's patent applications WO 01/52878, WO 01/93893 and WO 03/047500.

The use of Copolymer 1 for treatment of prion-related diseases is disclosed in WO 01/97785. Gendelman and co-workers disclose that passive immunization with splenocytes of mice immunized with Cop 1 confers dopaminergic neuroprotection in MPTP-treated mice (Benner et al., 2004).

Cop 1 and related copolymers and peptides have been disclosed in WO 00/05250 (Aharoni et al., 2000) for treating autoimmune diseases and in WO 2004/064717 for treatment of Inflammatory Bowel Diseases (Aharoni et al, 2004).

The immunomodulatory effect of GA was attributed to its ability to induce Th2/3 cells that secrete high levels of anti-inflammatory cytokines (Aharoni et al., 1998; Duda et al., 2000). These cells cross the blood brain barrier (BBB), accumulate in the CNS (Aharoni et al., 2000, 2002), and express *in situ* interleukin-10 (IL-10), transforming growth factor-β (TGF-β), as well as Brain Derived Neurotrophic Factor (BDNF) (Aharoni et al., 2003). Furthermore, the GA-specific cells induce bystander effect on neighboring CNS cells to express these beneficial factors and reduce interferon (IFN)- y expression. A key issue in the capability of GA to counteract the pathological process is its effect on the neuronal system, which is the actual target of the pathological process.

None of the above-mentioned references discloses specifically that GA induces neurogenesis in the CNS and no data nor protocol is disclosed for testing GA effect in the induction of neurogenesis in the CNS.

### SUMMARY OF THE INVENTION

The present invention relates to the embodiments characterized in the claims. Thus, it relates to the following items:
1. A neuroprotective agent selected from Copolymer 1, a Copolymer 1-related polypeptide, a Copolymer 1-related peptide, and activated T cells which have been activated by Copolymer 1, a Copolymer 1-related polypeptide, or a Copolymer 1-related peptide, in combination with stem cell therapy with the proviso that said stem cells are not human embryonic stem cells, for use in treating an injury of the central nervous system (CNS) or peripheral nervous system (PNS), a Parkinsonian disorder such as Parkinson's disease, Huntington's disease, Alzheimer's disease, multiple sclerosis, or amyotrophic lateral sclerosis (ALS), wherein said Copolymer 1-related polypeptide/Copolymer 1-related peptide functionally cross-reacts with myelin basic protein (MBP) and is able to compete with MBP on the MHC class II in the antigen presentation.
2. The neuroprotective agent for use according to item 1, wherein said neuroprotective agent is for administration to a patient before, concomitantly or after transplantation of the stem cells to said patient.
3. The neuroprotective agent for use according to items 1 or 2, wherein said neuroprotective agent is Copolymer 1 or a pharmaceutically acceptable salt thereof.
4. The neuroprotective agent for use according to any one of items 1 to 3, wherein the stem cells are adult stem cells, embryonic stem cells with the proviso that said embryonic stem cells are not human embryonic stem cells , umbilical cord blood stem cells, hematopoietic stem cells, peripheral blood stem cells, mesenchimal stem cells, multipotent stem cells, neural stem cells, neural progenitor cells, stromal stem cells, progenitor cells, or precursors thereof, or genetically-engineered stem cells.
5. The neuroprotective agent for use according to any one of items 1 to 3, wherein said CNS injury is selected from spinal cord injury, closed head injury, blunt trauma, penetrating trauma, hemorrhagic stroke, ischemic stroke, cerebral ischemia, optic nerve injury, myocardial infarction or injury caused by tumor excision.

Moreover, there is disclosed a method for inducing and enhancing neurogenesis and/or oligodendrogenesis from endogenous as well as from exogenously administered stem cells, which comprises administering to an individual in need thereof an agent selected from the group consisting of Copolymer 1, a Copolymer 1-related polypeptide, a Copolymer 1-related peptide, and activated T cells which have been activated by Copolymer 1, a Copolymer 1-related polypeptide, or a Copolymer 1-related peptide.

There is further disclosed a method of stem cell therapy comprising transplantation of stem cells in combination with a neuroprotective agent to an individual in need thereof, wherein said neuroprotective agent is selected from the group consisting of Copolymer 1, a Copolymer 1-related polypeptide, a Copolymer 1-related peptide, and activated T cells which have been activated by Copolymer 1, a Copolymer 1-related polypeptide, or a Copolymer 1-related peptide.

The invention also relates to the use of a neuroprotective agent selected from the group consisting of Copolymer 1, a Copolymer 1-related polypeptide, a Copolymer 1-related peptide, and activated T cells which have been activated by Copolymer 1, a Copolymer 1-related polypeptide, or a Copolymer 1-related peptide, for the preparation of a pharmaceutical composition for inducing and enhancing neurogenesis and/or oligodendrogenesis from endogenous as well as from exogenous stem cells administered to a patient.

In a preferred embodiment, the agent is Copolymer 1 for use in combination with stem cell therapy.

### BRIEF DESCRIPTION OF THE FIGURES

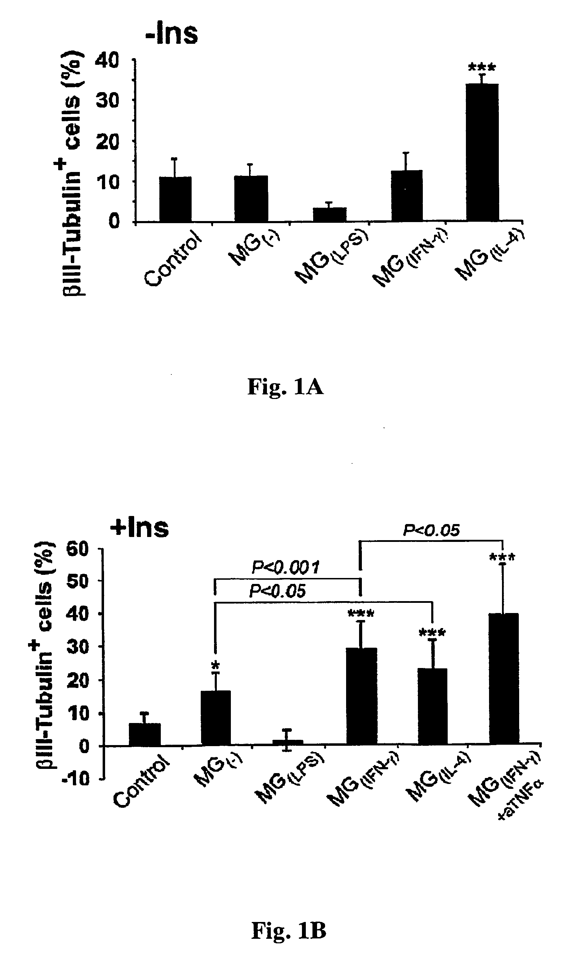

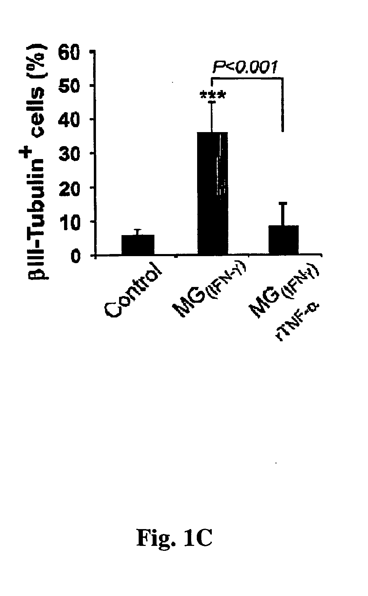

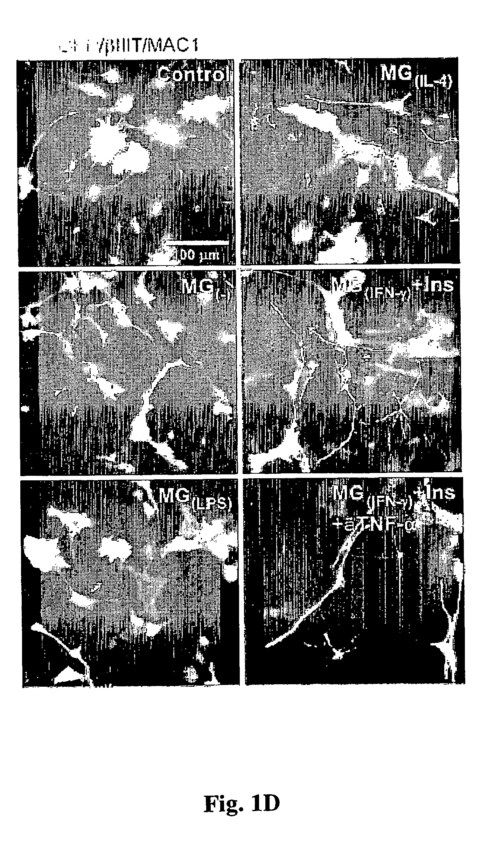

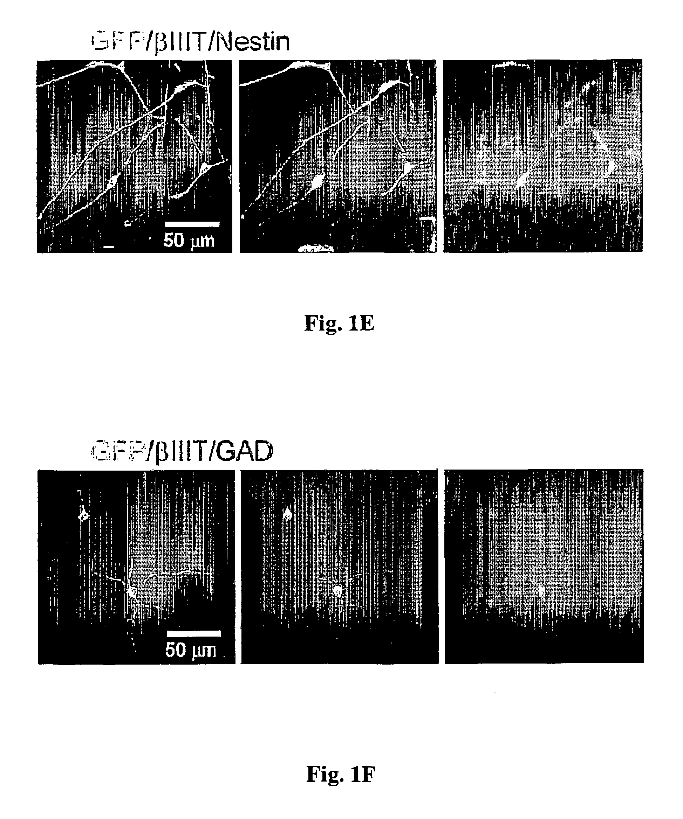

**Figs. 1A-1F** show that differentiation of NPCs into neurons can be either induced or blocked by microglia, depending on how they are activated. Green fluorescent protein (GFP)-expressing NPCs (green) were co-cultured with differently activated microglia from mice for 5 days. Quantification of β-III-tubulin⁺ cells (expressed as a percentage of GFP⁺ cells) obtained from confocal images, without (-Ins) or with insulin (+Ins), is summarized in Figs. 1A and 1B, respectively. Fig. 1C shows results of the effect of rTNF-α on the number of β-III-tubulin⁺ cells, expressed as a percentage of GFP⁺ cells, in co-cultures of NPCs and MG_{(IFN-γ)} in the presence of insulin. Error bars represent means ± SD. Data are from one of at least three independent experiments in replicate cultures. Asterisks above bars express differences relative to untreated (Control) NPCs (* *P* < 0.05; *** *P <* 0.001; ANOVA). Fig. 1D shows representative confocal images of GFP-expressing NPCs (green), in the absence of insulin without microglia (Control); with untreated microglia (MG₍₋₎); with LPS-activated microglia (MG_{(LPS)}); with IL-4-activated microglia (MG_{(IL-4)}); and in the presence of insulin with IFN-γ-activated microglia (MG_{(IFN-γ)}+Ins) or IFN-γ-activated microglia (MG_{(IFN-γ)}+Ins) and ãTNF. Fig. 1E shows GFP-expressing NPCs co-expressing β-III-tubulin and Nestin. Fig. 1F shows that newly formed neurons from NPCs are positively stained for glutamic acid decarboxylase (GAD) 67 (β-III-tubulin⁺/GFP⁺/GAD⁺). Note, confocal channels are presented separately.

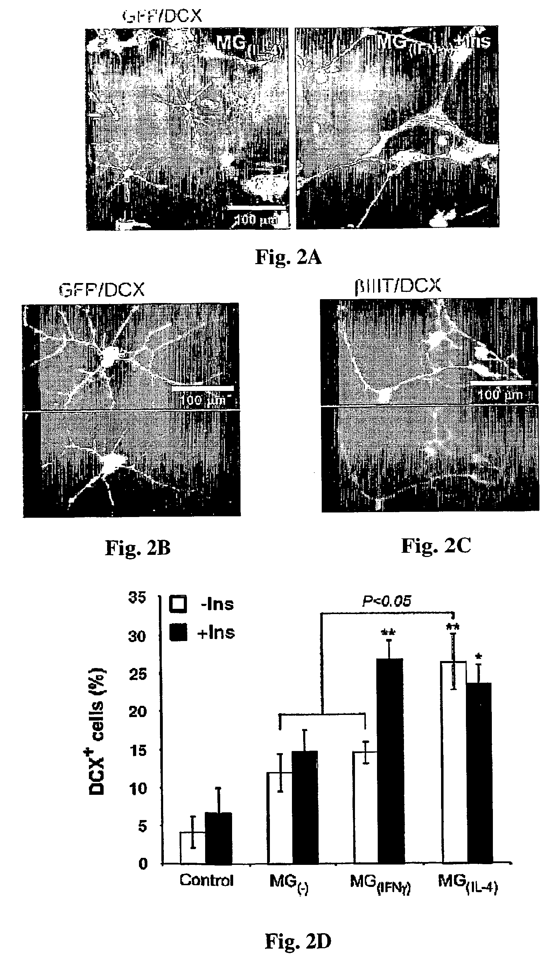

**Figs. 2A-2D** show that microglia activated with IFN-γ or IL-4 induce differentiation of NPCs into doublecortin (DCX)-expressing neurons with different morphology. GFP-expressing NPCs (green) were co-cultured with differently activated microglia as described in Fig. 1, and stained for the neuronal marker DCX. Fig. 2A depicts two representative confocal images of GFP-expressing NPCs (green) co-cultured for 5 days with MG_{(IL-4)} in the absence of insulin (left panel) or with MG_{(IFN-γ)} in the presence of insulin (MG_{(IFN-γ)+}Ins, right panel). Fig. 2B shows representative confocal images of GFP-expressing NPCs co-expressing DCX. Fig. 2C shows representative confocal images of β-III-tubulin⁺ cells co-expressing DCX. Note, confocal channels are presented separately. Fig. 2D shows quantification of DCX⁺ cells (expressed as a percentage of GFP⁺ cells) obtained from confocal images, without or with insulin. Error bars represent means ± SD. Data are from one of at least three independent experiments in replicate cultures. Asterisks above bars express differences relative to untreated (control) NPCs (^{*} P < 0.05;^{**} P < 0.01; ^{***} P < 0.001; ANOVA).

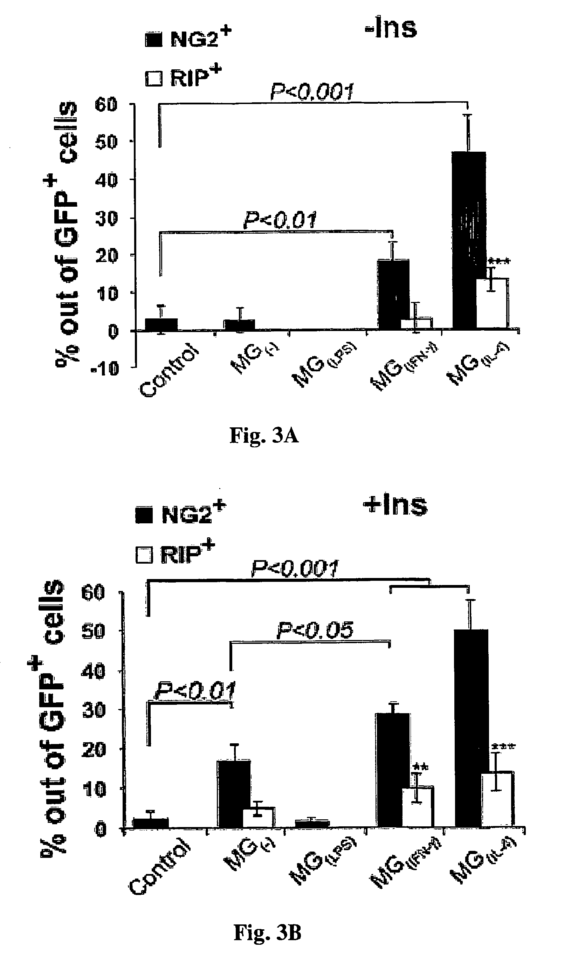

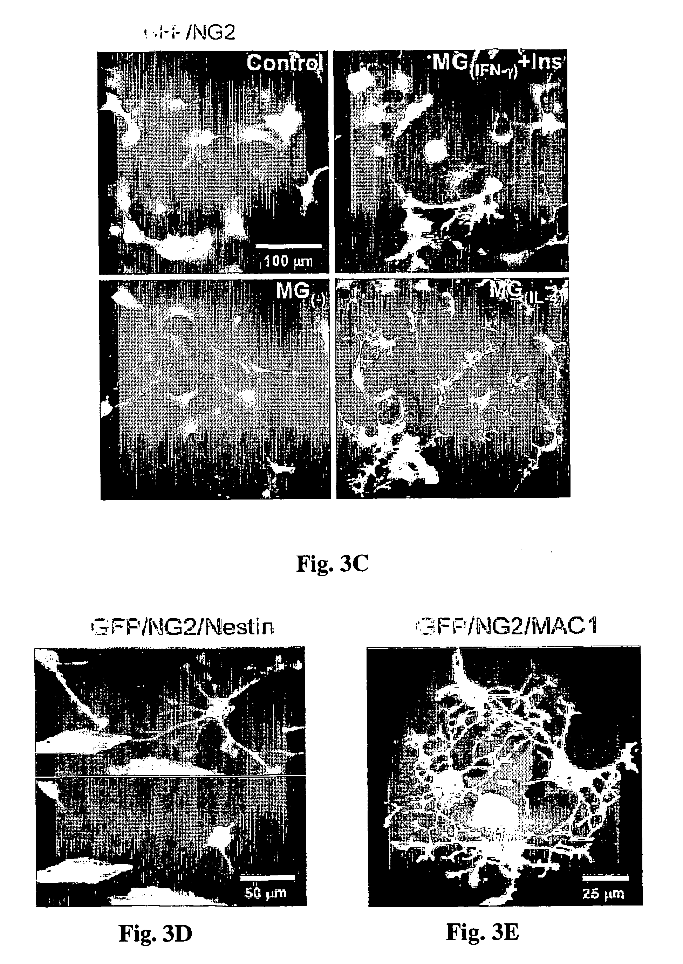

**Figs. 3A-3E** show that differentiation of NPCs into oligodendrocytes can be either induced or blocked by microglia, depending on how they are activated. GFP-expressing NPCs (green) were cultured alone (Control) or co-cultured with differently activated microglia as described in Fig. 1. Histograms showing quantification of NG2⁺ or RIP⁺ cells (expressed as a percentage of GFP⁺ cells) obtained from confocal images, co-cultures after 5 days (3A) in insulin-free medium (-Ins) or (3B) in insulin-containing medium (+Ins). The data shown are from one of three independent experiments in replicate cultures, with bars representing means ± SD. Asterisks above bars express differences relative to untreated (Control) NPCs (** *P* < 0.01; *** *P* < 0.001; ANOVA). Fig. 3C shows 4 representative confocal images of GFP-expressing NPCs (green) and NG2⁺ (red) cells: without microglia (Control); co-cultured with untreated microglia (MG₍₋₎); co-cultured with IFN-γ-activated microglia in the presence of insulin (MG_{(IFN-γ)+}Ins); co-cultured with IL-4-activated microglia (MG_{(IL-4)}), for 5 days. Fig. 3D shows confocal images showing co-localization of GFP, NG2 and Nestin cells. Note, confocal channels are presented separately. Fig. 3E shows that NG2⁺ cells are seen adjacent to MAC1⁺ cells.

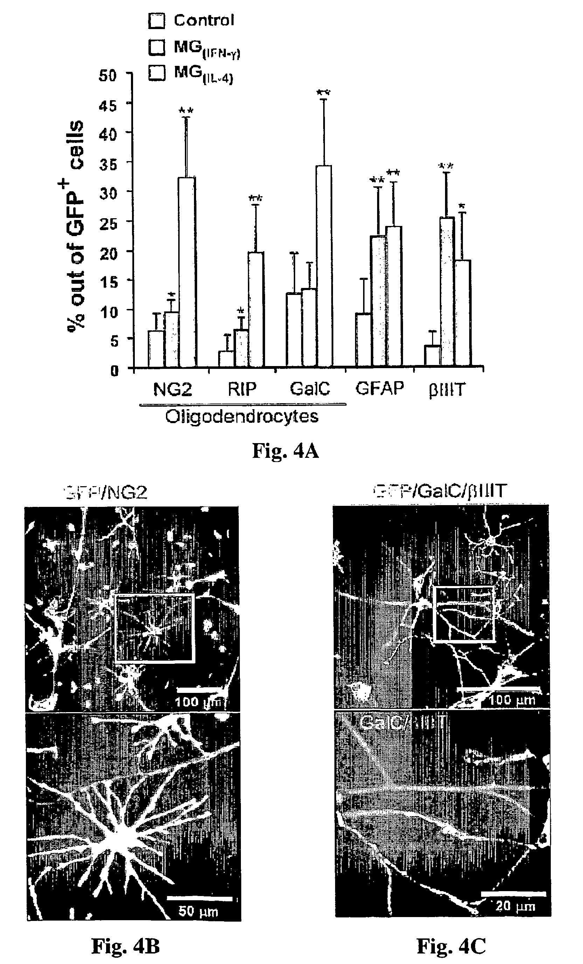

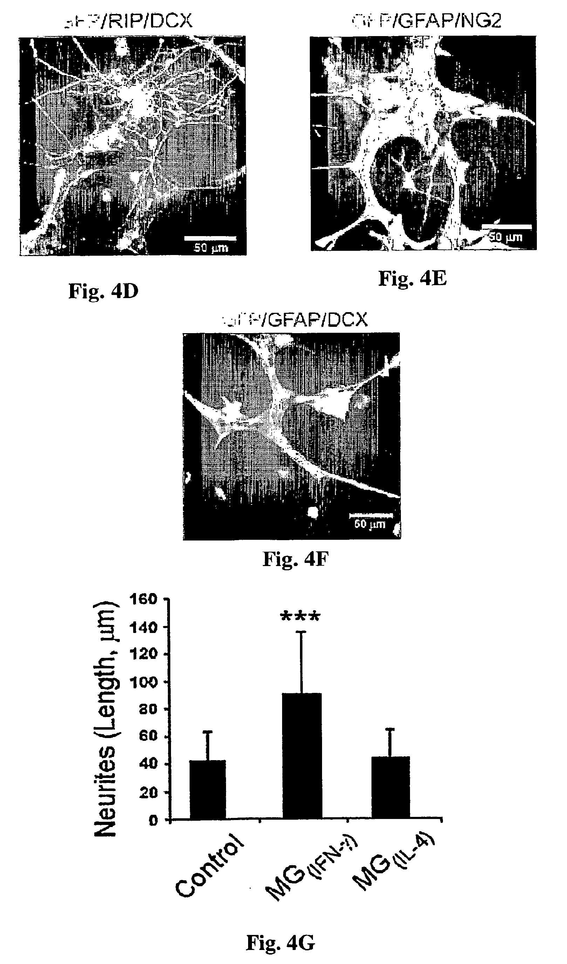

**Figs. 4A-4G** show differentiation and maturation of NPCs in the presence of MG_{(IFN-γ)} or MG_{(IL-4)} after 10 days in culture. Cultures of untreated NPCs (Control) or of NPCs co-cultured with MG_{(IFN-γ)} or MG_{(IL-4)} were analyzed after 10 days. Fig. 4A depict the numbers of NG2⁺, RIP⁺, GalC⁺, GFAP⁺ or β-III-tubulin⁺ cells expressed as percentages of GFP⁺ cells. Values are means ± SD (^{*} *P* < 0.05; ^{**} *P <* 0.01; *** *P* < 0.001; ANOVA). Figs. 4B-4F are representative confocal images of NPCs in the presence of MG_{(IL-4)} after 10 days in culture. Fig. 4B shows that increased branching of processes stained with NG2 was seen after 10 days (compare Fig. 4B with Fig. 3C, MG_{(IL-4)}). Contact is seen to be formed between an NG2⁺ process and an adjacent cell (high magnification of boxed area). Fig. 4C shows that staining of the same cultures for mature oligodendrocytes (GalC⁺) and neurons β-III-tubulin⁺) shows contacts between neurons and highly branched oligodendrocytes (high magnification of boxed area). Fig. 4D shows that no overlapping is seen between labeling for neurons (DCX⁺) and for oligodendrocytes (RIP⁺). Figs. 4E and 4F show that no overlapping is seen between GFAP and NG2 labeling or between GFAP and DCX labeling, respectively. Fig. 4G shows neurites length of MG_{(IFN-γ)} and MG_{(IL-4)} cells. Values are means ± SD (*** *P* < 0.001; ANOVA).

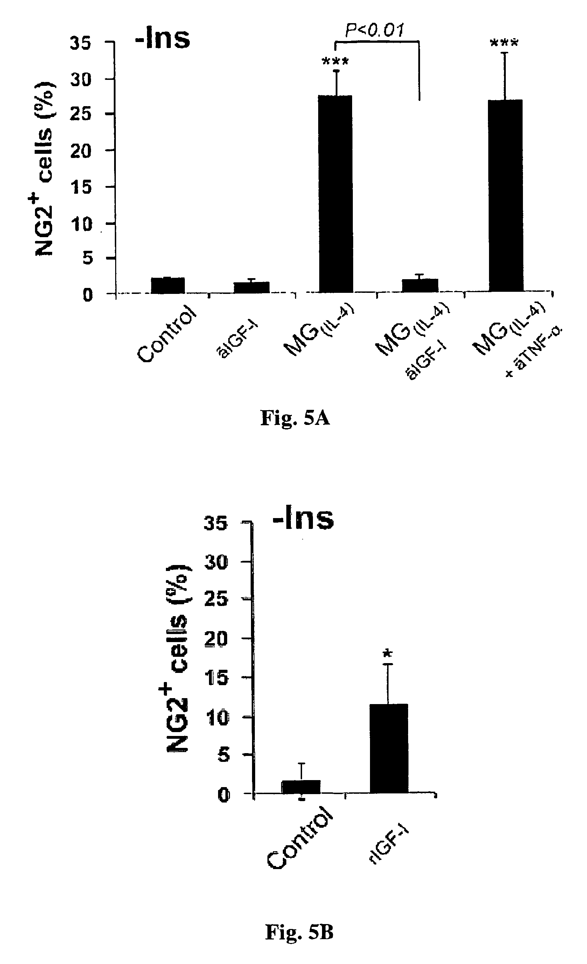

**Figs. 5A-5D** show the role of IGF-I and TNF-α in induction of oligodendrogenesis by IL-4- and IFN-γ-activated microglia. (5A) GFP-expressing NPCs (green) were cultured alone (control), in the presence of ãIGF-I, in co-cultures with MG_{(IL-4)} in the absence or presence of ãIGF-I (5 µg/ml), or in the presence of ãTNF-α (1 ng/ml). (5B) In an independent experiment, NPCs were cultured in the presence of rIGF-I (500 ng/ml). In Figs. 5A and 5B no insulin was added to the media. (5C) NPCs were cultured alone (control), with ãTNF-α (1 ng/ml), or with MG_{(IFN-γ)} in the absence or presence of ãTNF-α. (5D) In an independent experiment, NPCs were cultured with MG_{(IFN-γ)} in the presence of insulin and rTNF-α (10 ng/ml). Error bars represent means ± SD. Asterisks above bars express differences relative to untreated (control) NPCs (* *P* < 0.05; ** *P <* 0.01; *^{***} P <* 0.001; ANOVA).

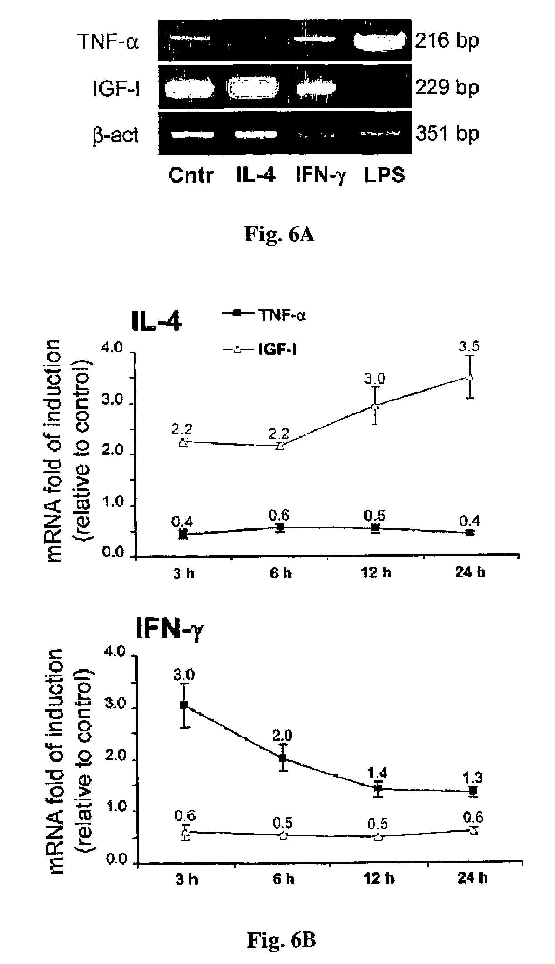

**Figs. 6A-6C** show that IFN-γ, unlike IL-4, transiently induced TNF-α and reduced IGF-I expression in microglia. (6A) Microglia treated with IL-4 (10 ng/ml), IFN-γ (20 ng/ml), or LPS (100 ng/ml) for 24 h were analyzed for TNF-α and IGF-I mRNA by semi-quantitative RT-PCR. Representative results of one of three independent experiments are shown. (6B) Time courses of TNF-α and IGF-I mRNA expression by MG_{(IL-4)} and MG_{(IFN-γ)}. PCR at each time point was performed with the same reverse-transcription mixtures for all cDNA species. Values represent relative amounts of amplified mRNA normalized against β-actin in the same sample, and are represented as fold of induction relative to control (means ± SD). The linear working range of amplifications was ascertained before the experiments were carried out. Each sample was tested in three replicates, and similar results were obtained in three different microglial cultures. (6C) Statistical analysis of IGF-I expression demonstrates fluorescence intensity per cell, calculated as a percentage of increased intensity relative to MG₍₋₎ (control) (means ± SD; obtained in two independent experiments, each repeated four times). Note, relative to the untreated control, MG_{(IL-4)} showed a significant increase in IGF-I. Asterisks above bar express differences relative to MG₍₋₎ (* *P* < 0.05; ** *P* < 0.001; two-tailed Student's *t*-test).

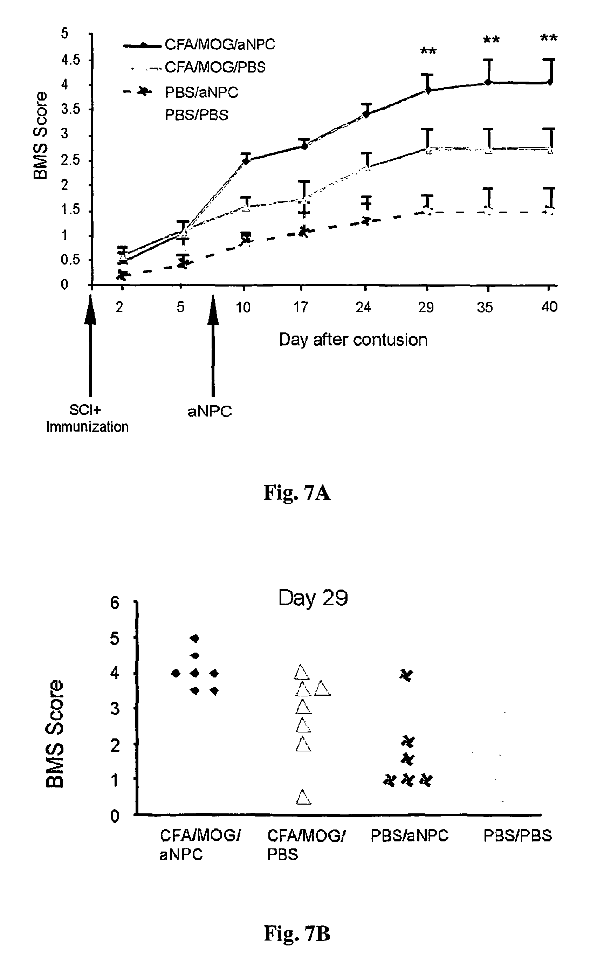

**Figs. 7A-7H** show that a myelin-specific autoimmune response operates synergistically with transplanted aNPC transplantation in promoting functional recovery from spinal cord injury (SCI). Recovery of motor function after SCI (200 kdynes for 1 s) in male C57B1/6J mice (n = 6-9 in each group). (7A) Mice were immunized with MOG peptide or PBS emulsified in CFA containing 1% *Mycobacterium tuberculosis* (MOG-CFA and PBS-CFA, respectively). One week after SCI, aNPCs were transplanted into their lateral ventricles (MOG-CFA/aNPC or PBS-CFA/aNPC). The lateral ventricles of mice in similarly injured and immunized control groups were treated with PBS (MOG-CFA/PBS or PBS-CFA/PBS). Values of the Basso motor score (BMS) rating scale are presented. (7B) BMS scores of individual mice described in (7A) on day 28 of the experiment. (7C) Recovery of motor function after SCI (200 kdynes for 1 s) in male C57BI/6J mice (n = 6-9 in each group) immunized with MOG peptide 45D emulsified in CFA containing *2*.*5*%*Mycobacterium tuberculosis.* One week after SCI, aNPCs were transplanted into the lateral ventricles. Similarly injured and immunized control groups, instead of being transplanted with aNPCs, were injected with PBS. BMS values are presented. (7D) Recorded BMS scores of individual mice described in (7C) on day 28 of the experiment. (7E) Injury and aNPC transplantation were as in (7A), but immunization was carried out 7 days prior to SCI and the mice were immunized with MOG-IFA or injected with PBS (control). (7F) Injury and aNPC transplantation were as in (7A), but immunization was carried out 7 days prior to SCI and the mice were immunized with OVA/CFA. (7G, 7H) Injury and aNPC transplantation were as in (7A), but immunization was carried out 7 days prior to SCI and the mice were immunized with MOG peptide and CFA containing 2.5% *Mycobacterium tuberculosis.* Results in all groups are means ± SEM. Asterisks show differences at the indicated time points, analyzed by two-tailed Student's t-test. (*, *p <* 0.05; **, *p* < 0.01; ***, *p* < 0.001).

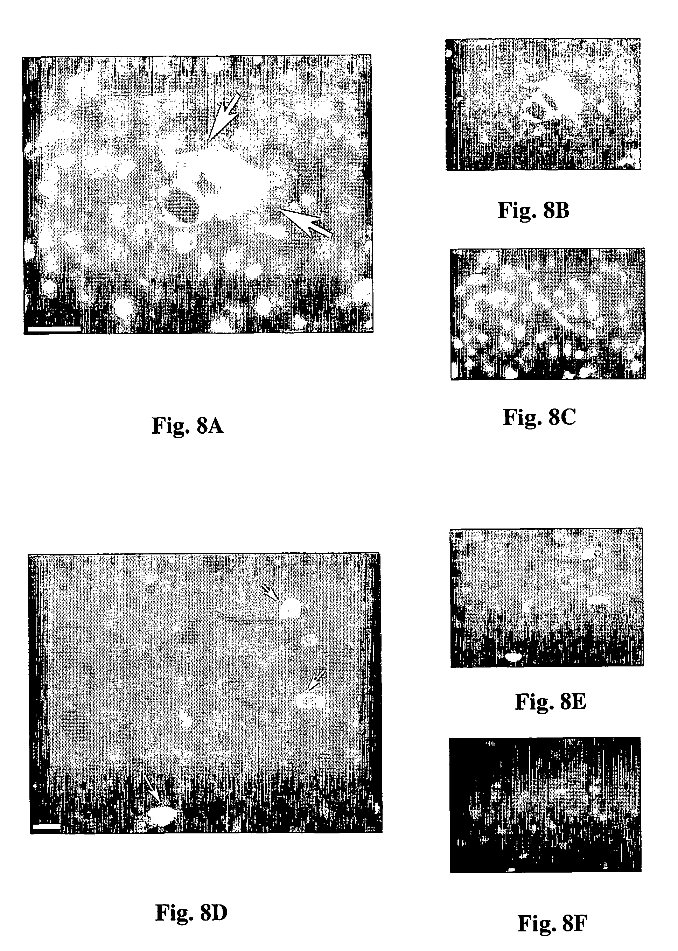

**Figs. 8A-8F** show that GFP-labeled aNPCs are found in the parenchyma of the spinal cord after dual treatment with MOG immunization and aNPC transplantation. Immunohistochemical staining of longitudinal paraffin sections of spinal cords excised 7 or 60 days after transplantation of aNPCs to the lateral ventricles. Sections were stained with anti-GFP antibody and counterstained with Hoechst to detect nuclei. They were then scanned by fluorescence microscopy for the presence of GFP+ cells. Representative micrographs of GFP-immunolabeled cells in areas adjacent to the lesion site 7 days after transplantation (8A-8F) and 60 days after transplantation of aNPCs (8A-8F) are shown.

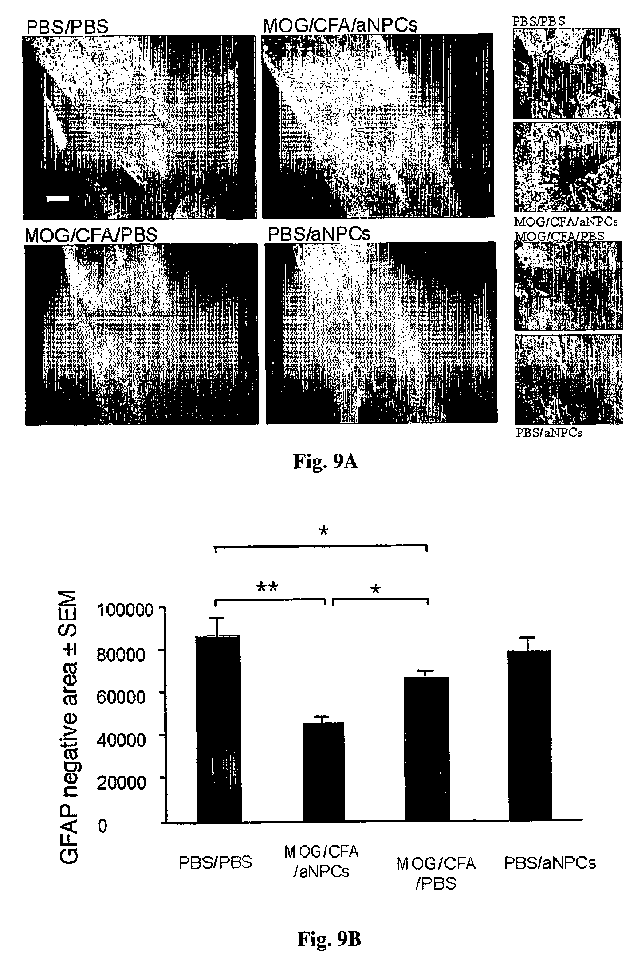

**Figs. 9A-9F** show histological analysis of spinal cords from injured C57B1/6J mice after dual treatment with MOG/CFA immunization and aNPC transplantation. Spinal cords were excised 1 week after cell transplantation. SCI C57B1/6J mice (n = 3-4 in each group) were subjected to SCI (200 kdynes for 1 s) and immunized on the day of SCI with MOG peptide emulsified in CFA containing 1% *Mycobacterium tuberculosis.* One week after SCI, the lateral ventricles of MOG-CFA-immunized mice transplanted aNPCs or injected PBS. (9A, 9B), GFAP staining of longitudinal sections of injured spinal cords shows significantly smaller areas of scar tissue after treatment with MOG-CFA/aNPC than in any of the other groups. (9A) Representative micrographs of spinal cords from mice treated with MOG-CFA/aNPC, MOG-CFA/PBS, PBS-CFA/aNPC, or PBS-CFA/PBS are shown. (9B) Quantification of the area delineated by GFAP staining (* p <0.05, ** p <0.01, *** p <0.001, two-tailed Student's t-test; n = 4 analyzed slices from each mouse). (9C, 9D) Longitudinal paraffin sections of spinal cords excised and stained for IB4 7 days after cell transplantation and 14 days after contusive SCI show significantly less staining in MOG/CFA/aNPC-treated mice than in any of the other groups. (9D) Quantification of area occupied by IB4 staining (* p <0.05, ** p <0.01 ***p <0.001, two-tailed Student's t-test). (9E, 9F) Staining with anti-CD3 antibody to identify infiltrating T cells at the site of injury. (9E) Representative micrographs of spinal cords from mice treated with MOG-CFA/aNPC, MOG-CFA/PBS, PBS-CFA/aNPC, or PBS-CFA/PBS. Manual counting of cells in four slices from each mouse, obtained from four areas surrounding the site of injury, disclosed significantly more CD3+ cells in the group treated with MOG-CFA/aNPC than in any of the other groups (* p <0.05, ** p <0.01 ***p <0.001, two-tailed Student's t-test).

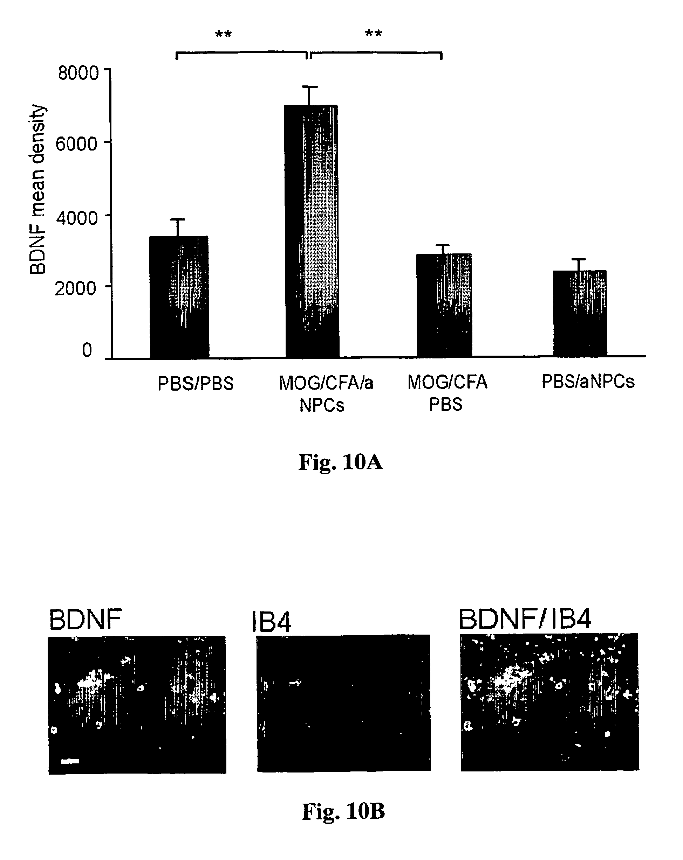

**Figs. 10A-10D** show histological analysis of BDNF and noggin expression in spinal cords from injured C57B1/6J mice after dual treatment with MOG/CFA immunization and aNPC transplantation. C57B1/6J mice were subjected to SCI (n = 3-4 in each group) and immunized with pMOG 35-55 emulsified in CFA (1% *Mycobacterium tuberculosis)* on the day of SCI. One week after SCI, the lateral ventricles of MOG-CFA-immunized mice transplanted with aNPCs or injected PBS. Longitudinal sections of spinal cords excised 7 days after cell transplantation and 14 days after SCI (n = 3-4 in each group) were stained for BDNF. (10A) Quantification of area stained for BDNF (* p <0.05, ** p <0.01, ***p <0.001, two-tailed Student's t-test). Staining for BDNF is significantly more intense in mice treated with MOG-CFA/aNPC than in any of the other groups. (10B) Double staining for BDNF and IB4 shows that IB4+ microglia/macrophages are a major source of BDNF. (10C) Significantly more intense staining for noggin was found in mice treated with MOG/CFA/aNPCs than in any of the other groups. (10D) Quantification of area stained with noggin (* p <0.05, ** p <0.01 ***, p <0.001, two-tailed Student's t-test). Double staining for noggin and IB4 shows that IB4+ microglia are a major source of noggin.

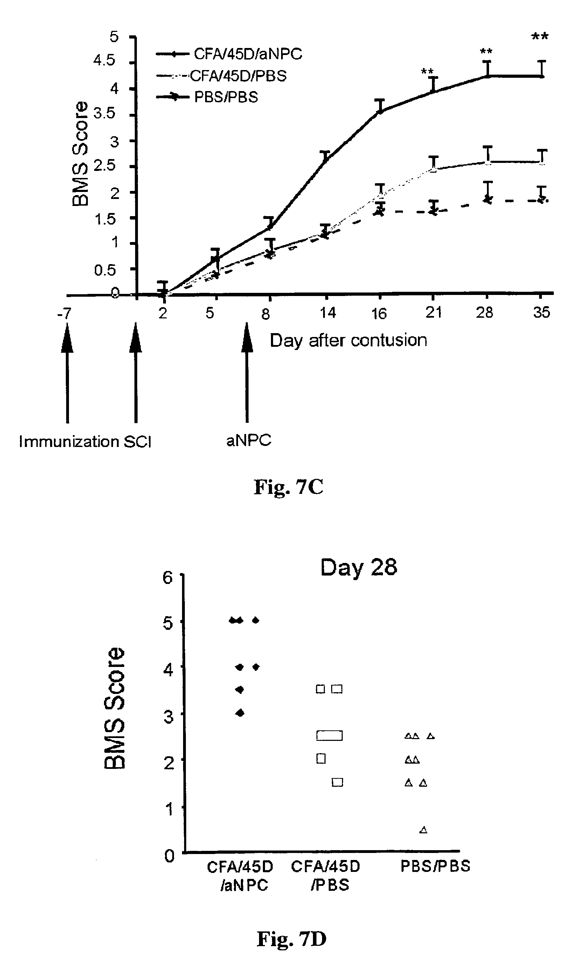

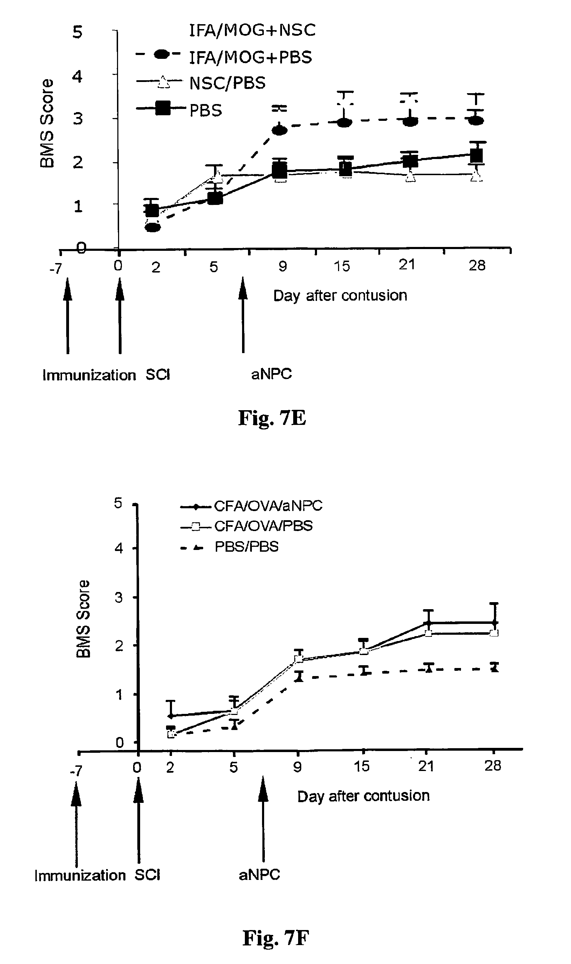

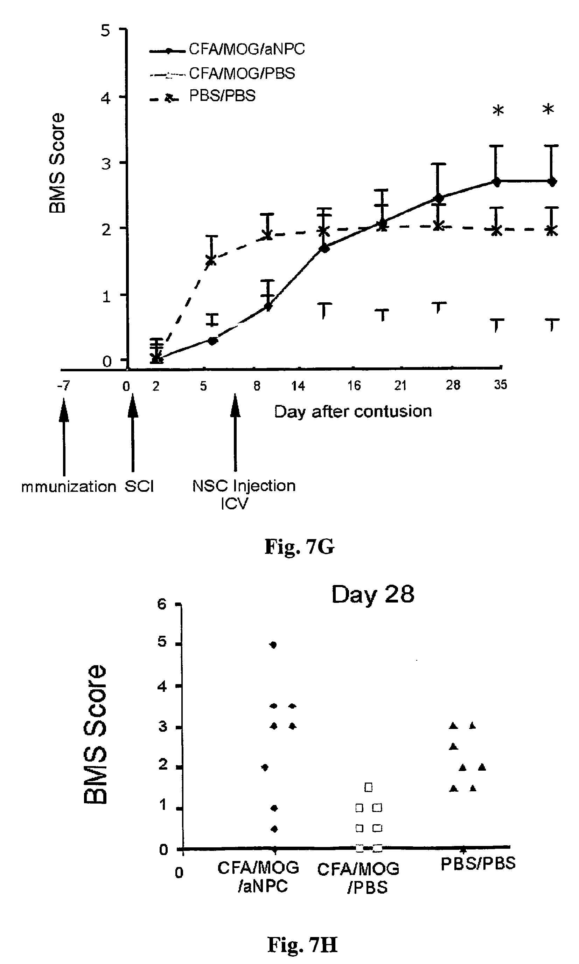

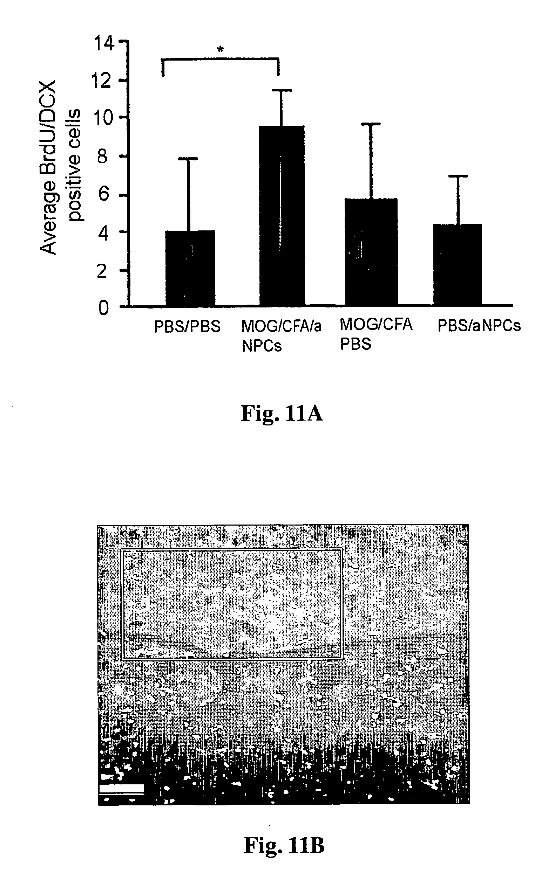

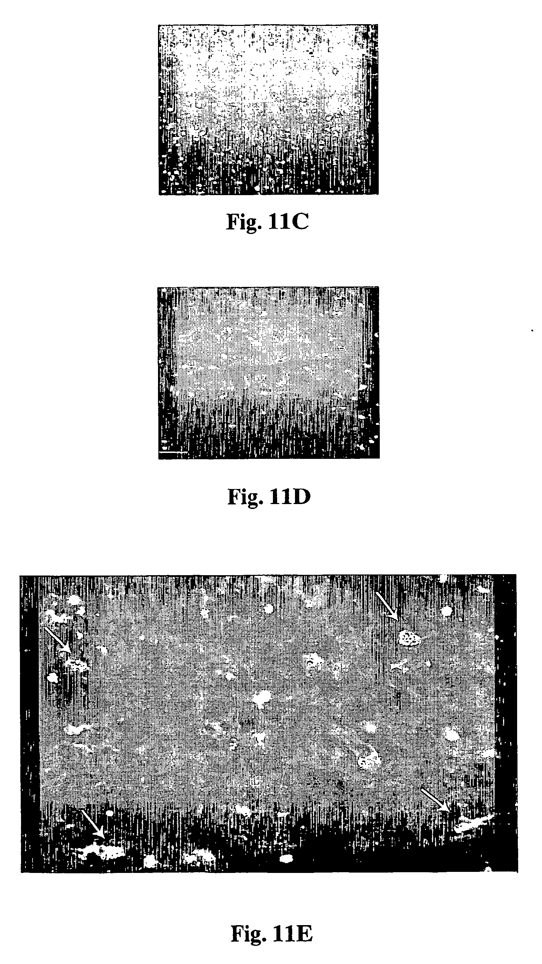

**Figs. 11A-11E** show increase in BrdU/DCX double staining in the vicinity of the site of injury after dual treatment with immunization and aNPC transplantation. SCI and aNPCs transplantation as in Fig. 7. One week after aNPCs transplantation mice were injected twice daily for 3 days with BrdU. Longitudinal sections of spinal cords excised 14 days after cell transplantation and 28 days after contusive SCI (n = 3-4 in each group) were stained for BrdU and DCX. Significantly more BrdU+/DCX+ cells were found in mice treated with MOG-CFA/aNPC than in any of the other groups.

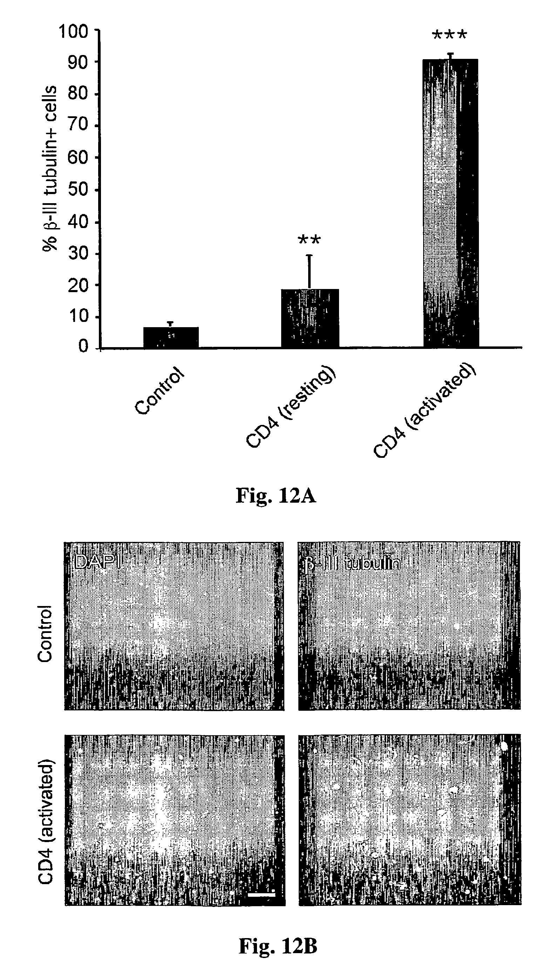

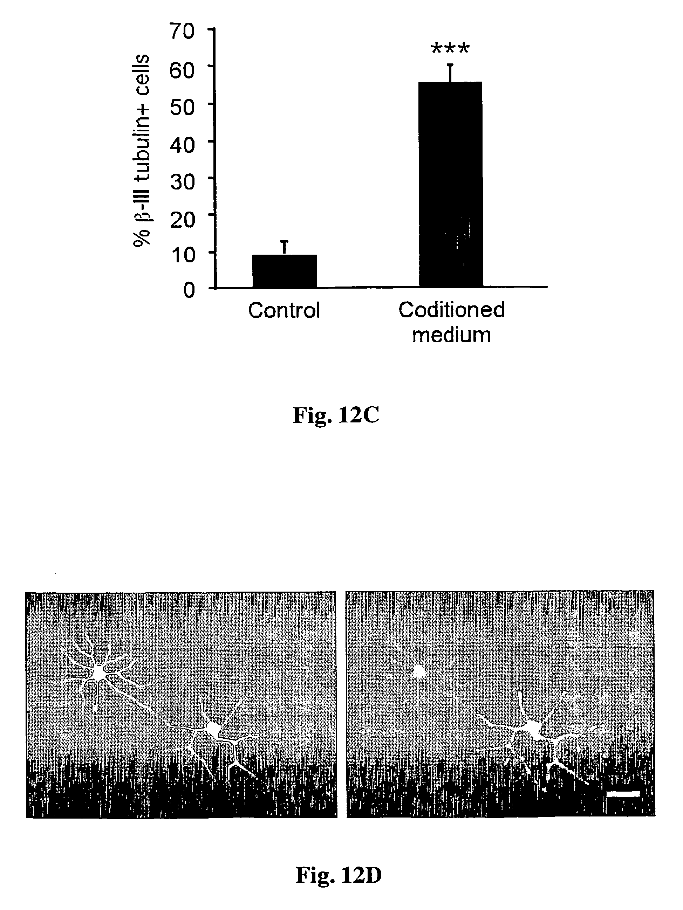

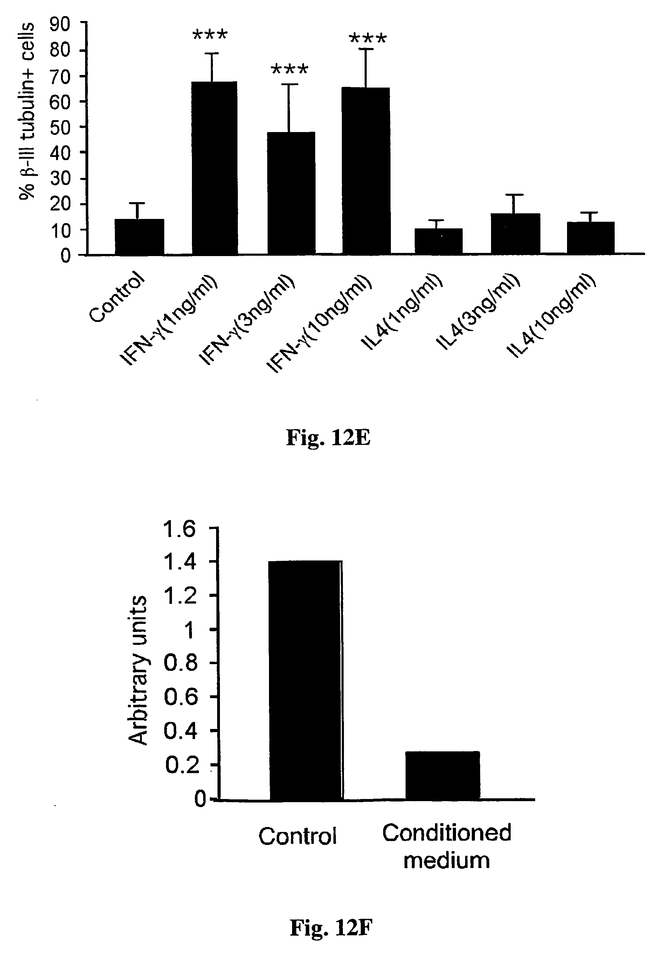

**Figs. 12A-12F** show that T cells induce neuronal differentiation from aNPCs *in vitro.* (Fig. 12A) Quantification of β-III-tubulin+ cells (expressed as a percentage of DAPI cells) after 5 days in culture alone (control), or in co-culture with pre-activated CD4+ T cells, or with resting CD4+ T cells (** *p* < 0.01; ****p* < 0.001; ANOVA). (Fig. 12B) Representative images showing β-III-tubulin expression in aNPCs after 5 days in culture alone (control), or in co-culture with pre-activated CD4+ T cells. (Fig. 12C) Quantification of β-III-tubulin+ cells (expressed as a percentage of DAPI cells) after 5 days in culture of aNPCs in the presence of medium conditioned by activated T cells. (Fig. 12D) Representative images showing branched, elongating β-III-tubulin lebeled fibers. (Fig. 12E) Quantification of β-III-tubulin+ cells in aNPCs after 5 days in culture with different concentrations of IFN-γ or IL-4 (****p* < 0.001; ANOVA). (Fig. 12F) Quantitative RT-PCR showing a fivefold reduction in Hes-5 expression in aNPCs cultured with medium conditioned for 24 h by activated CD4+ T-cells.

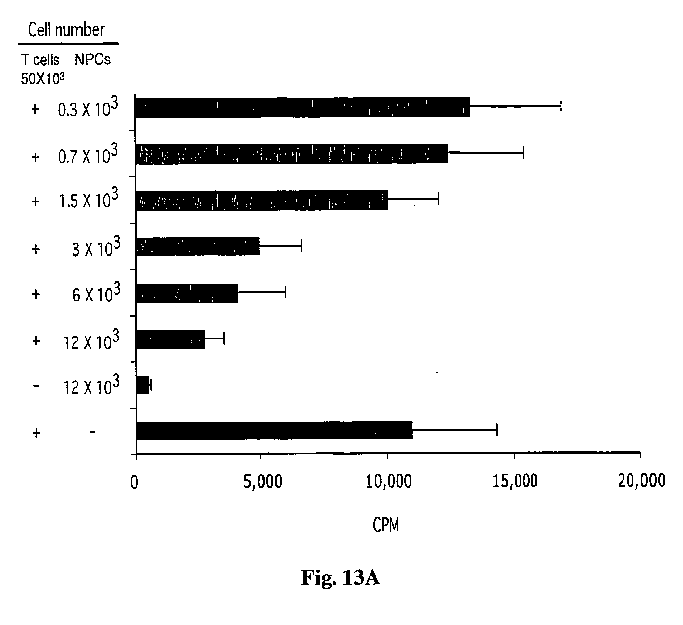

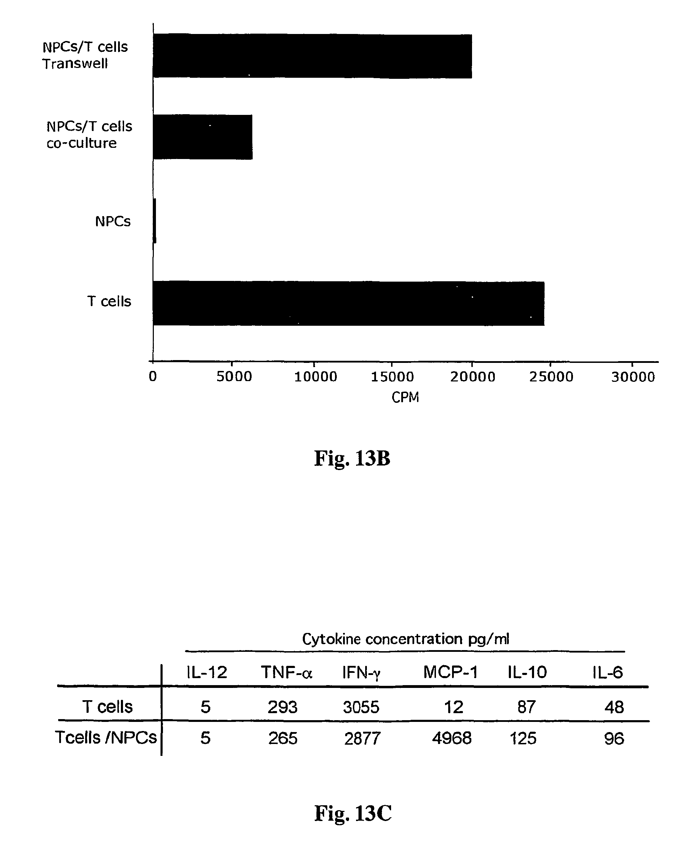

**Figs. 13A-13C** show that aNPCs inhibit T-cell proliferation and modulate cytokine production. (Fig. 13A) Proliferation was assayed 96 h after activation by incorporation of [³H]-thymidine into CD4+ T cells co-cultured with aNPCs. Recorded values are from one of three representative experiments and are expressed as means ± SD of four replicates. (Fig. 13B) Proliferation of CD4+ T cells cultured alone, or in the presence of aNPCs (co-culture), or with aNPCs in the upper chamber of a transwell. (Fig. 13C) Cytokine concentrations (pg/ml) in the growth medium 72 h after activation of CD4+ T cells alone or in co-culture with aNPCs.

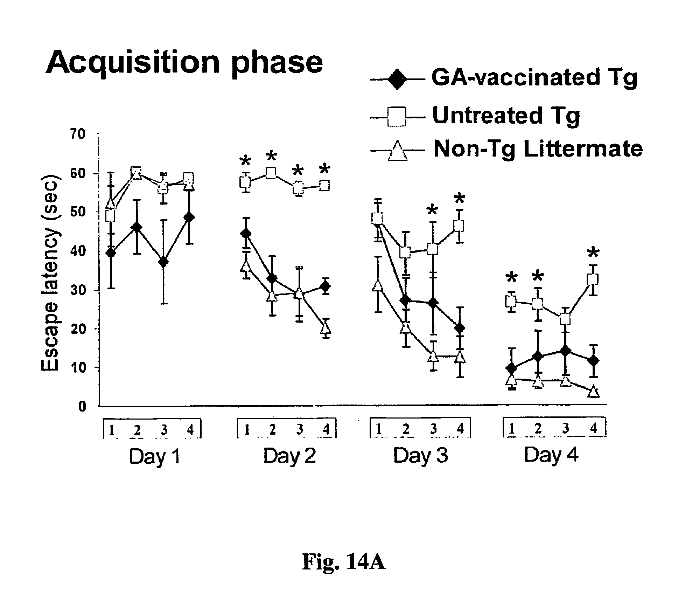

**Figs. 14A-14C** show that glatiramer acetate (GA) vaccination counteracts cognitive loss in the APP/PS1 Tg mouse model of Alzheimer's disease (AD). Hippocampal-dependent cognitive activity was tested in the MWM. (Figs. 14A-14C) GA-vaccinated Tg mice (*diamond; n=6*) showed significantly better learning/memory ability than untreated Tg mice (*square; n=7*) during the acquisition and reversal phases but not the extinction phase of the test. Untreated Tg mice showed consistent and long-lasting impairments in spatial memory tasks. In contrast, performance of the MWM test by the GA-vaccinated Tg mice was rather similar, on average, to that of their age-matched naïve non-Tg littermates (*triangle; n* = *6*) (3-way ANOVA, repeated measures: groups, df (2,16), *F =* 22.3*, P* < 0.0002; trials, df (3,48), *F=* 67.9, *P* < 0.0001; days, df (3,48), *F =* 3.1, *P* < 0.035, for the acquisition phase; and groups, df (2,16), *F* = 14.9, *P* < 0.0003; trials, df (3,48), *F* = 21.7, *P* < 0.0001; days, df (1,16), *F* = 16.9, *P* < 0.0008, for the reversal phase).

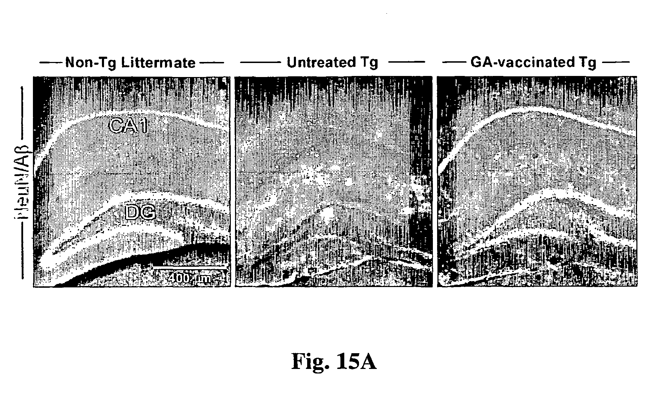

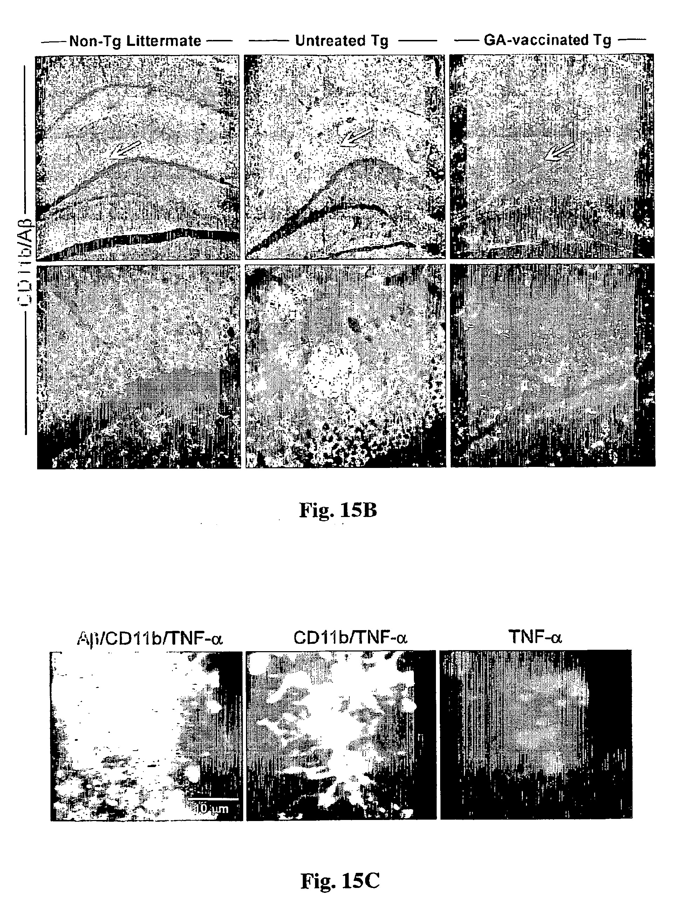

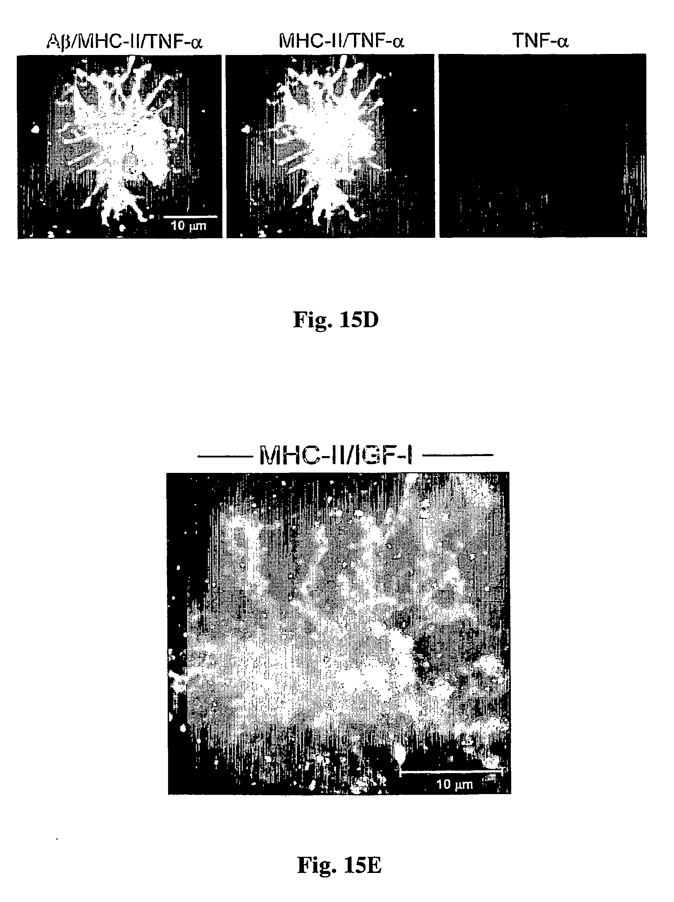

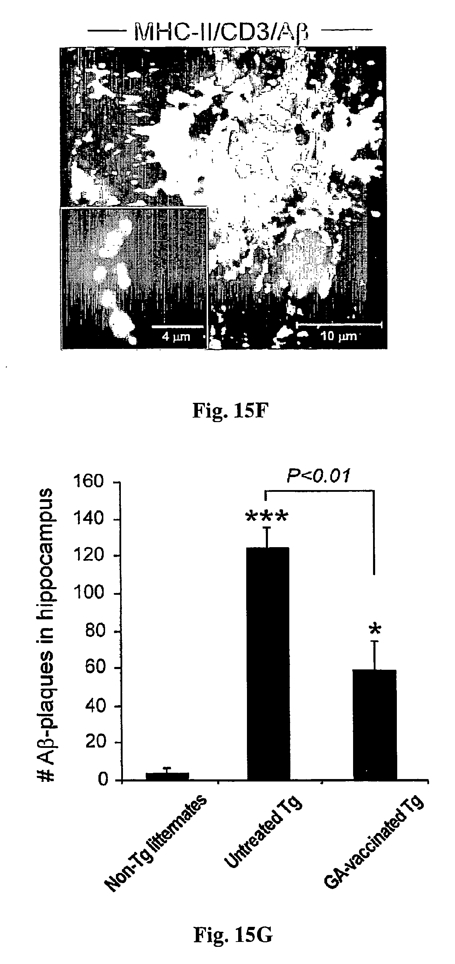

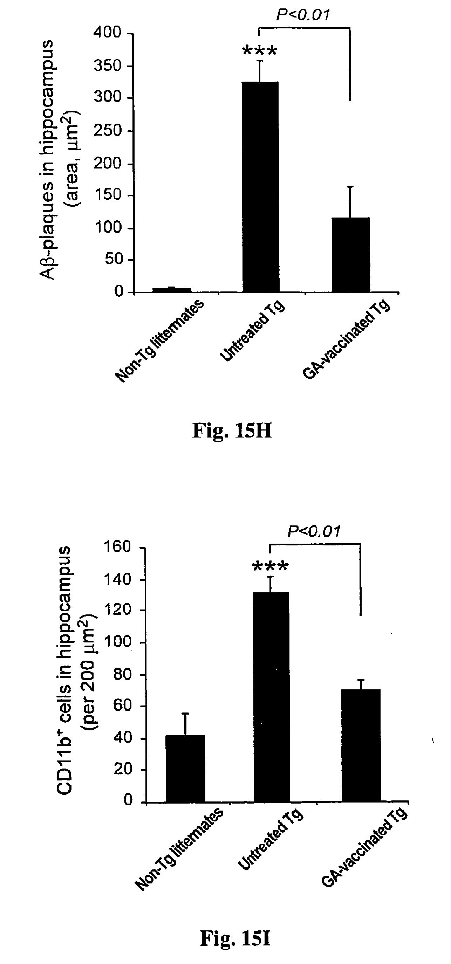

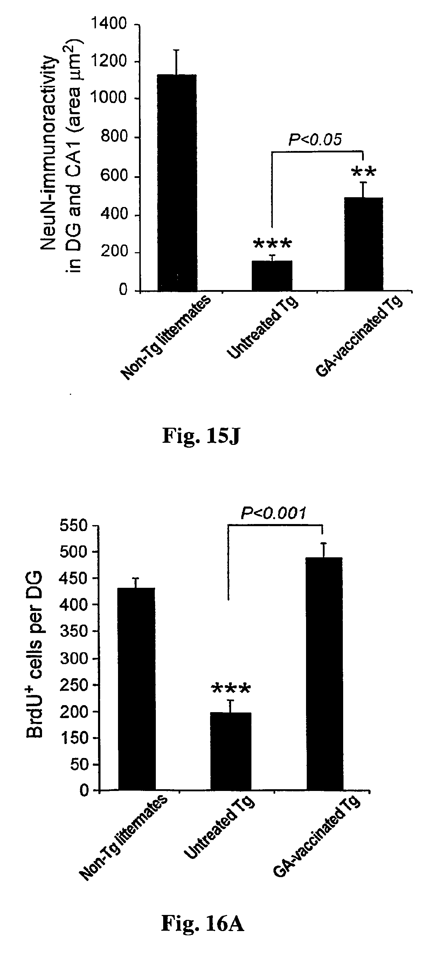

**Figs 15A-15J** show that T cell-based vaccination with GA leads to a reduction in β-amyloid (Aβ) and counteracts hippocampal neuronal loss in the brains of Tg mice: key role of microglia. (Fig. 15A) Representative confocal microscopic images of brain hippocampal slices from non-Tg, untreated-Tg, and GA-vaccinated Tg littermates stained for NeuN (mature neurons) and human Aβ. The non-Tg mouse shows no staining for human Aβ. The untreated Tg mouse shows an abundance of extracellular Aβ plaques, whereas in the GA-vaccinated Tg mouse Aβ-immunoreactivity is low. Weak NeuN⁺ staining is seen in the hippocampal CA1 and DG regions of the untreated Tg mouse relative to its non-Tg littermate, whereas NeuN⁺ staining in the GA-vaccinated Tg mouse is almost normal. (Fig. 15B) Staining for activated microglia using anti-CD11b antibodies. Images at low and high magnification show a high incidence of cells double-immunostained for Aβ and CD11b in the CA1 and DG regions of the hippocampus of an untreated Tg mouse, but only a minor presence of CD11b⁺ microglia in the GA-vaccinated Tg mouse. Arrows indicate areas of high magnification, shown below. (Fig. 15C) CD11b⁺ microglia, associated with an Aβ-plaque, expressing high levels of TNF-α in an untreated Tg mouse. (Fig. 15D) Staining for MHC-II (a marker of antigen presentation) in a cryosection taken from a GA-vaccinated Tg mouse in an area that stained positively for Aβ shows a high incidence of MHC-II⁺ microglia and almost no TNF-α⁺ microglia. (Fig. 15E) MHC-II⁺ microglia in the GA-vaccinated mouse co-express IGF-I. (Fig. 15F) CD3⁺ T cells are seen in close proximity to MHC-II⁺ microglia associated with Aβ-immunoreactivity. Boxed area shows high magnification of an immunological synapse between a T cell (CD3⁺) and a microglial cell expressing MHC-II. (Fig. 15G) Histogram showing the total number of Aβ-plaques (in a 30-µm hippocampal slice). (Fig. 15H) Histogram showing the total stained Aβ-immunooreactive cells. Note, the significant differences between GA-vaccinated and untreated Tg mice, and verifies the decreased presence of Aβ-plaques in the vaccinated Tg mice. (Fig. 15I) Histogram showing a remarkable reduction in cells stained for CD11b, indicative of activated microglia and inflammation, in the GA-vaccinated Tg mice relative to untreated Tg mice. Note the increase in CD11b⁺ microglia with age in the non-Tg littermates. (Fig. 15J) Histogram showing increased survival rate of NeuN⁺ neurons in the DGs of GA-vaccinated Tg mice relative to untreated Tg mice. Error bars indicate means ± SEM. Asterisks above bars express the significance of differences in the immunostaining (* *P* < 0.05; ***P* < 0.01; **** P* < 0.001; two-tailed Student's *t*-test). Note, all the mice in this study were included in the analysis (6-8 sections per mouse).

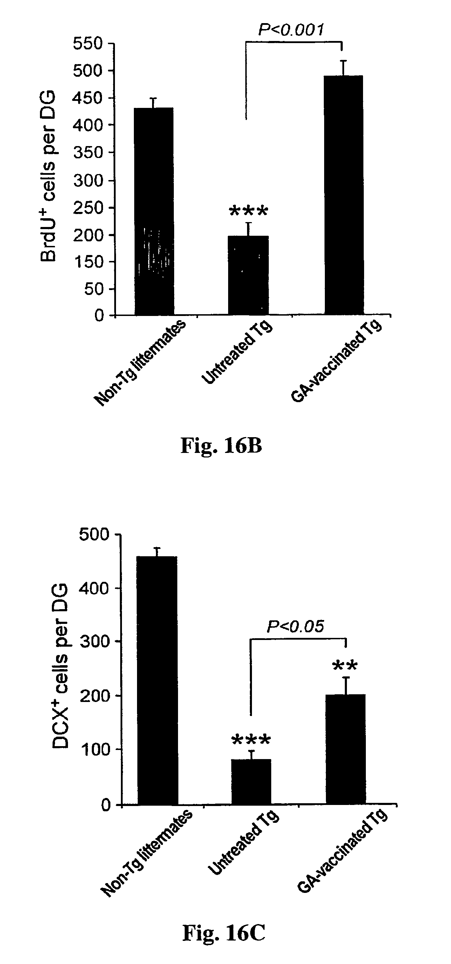

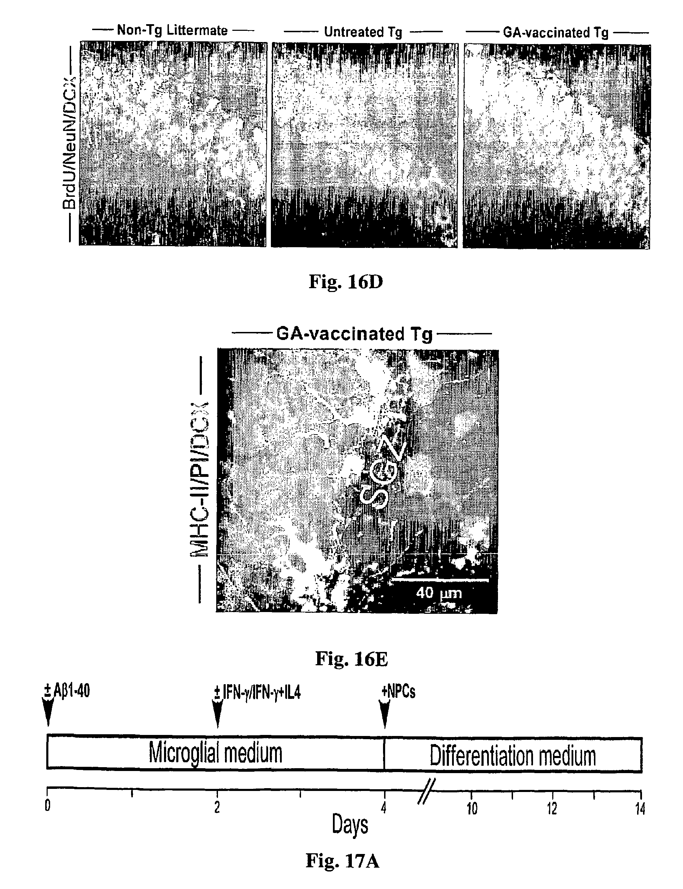

**Figs. 16A-16E** show enhanced cell renewal induced by T cell-based vaccination with glatiramer acetate (GA) in the hippocampus of adult Tg mice. Three weeks after the first GA vaccination, mice in each experimental group were injected i.p. with BrdU twice daily for 2.5 days. Three weeks after the last injection their brains were excised and the hippocampi analyzed for BrdU, DCX, and NeuN. (Fig. 16A-16C) Histograms showing quantification of the proliferating cells (BrdU⁺) (Fig. 16A), newly formed mature neurons (BrdU⁺/NeuN⁺) (Fig. 16B), and all pre-mature (DCX⁺-stained) neurons (Fig. 16C). Numbers of BrdU⁺, BrdU⁺/NeuN⁺, and DCX⁺ cells per DG, calculated from six equally spaced coronal sections (30 µm) from both sides of the brains of all the mice tested in this study. Error bars represent means ± SEM. Asterisks above bars denote the significance of differences relative to non-Tg littermates (** *P* < 0.01; *** *P* < 0.001; two-tailed Student's *t*-test). Horizontal lines with *P* values above them show differences between the indicated groups (ANOVA). (Fig. 16D) Representative confocal microscopic images of the DG showing immunostaining for BrdU/DCX/NeuN in a GA-vaccinated Tg mouse and in a non-Tg littermate relative to that in an untreated Tg mouse. (Fig. 16E) Branched DCX⁺ cells are found near MHC-II⁺ microglia located in the subgranular zone of the hippocampal DG of a GA-vaccinated Tg mouse.

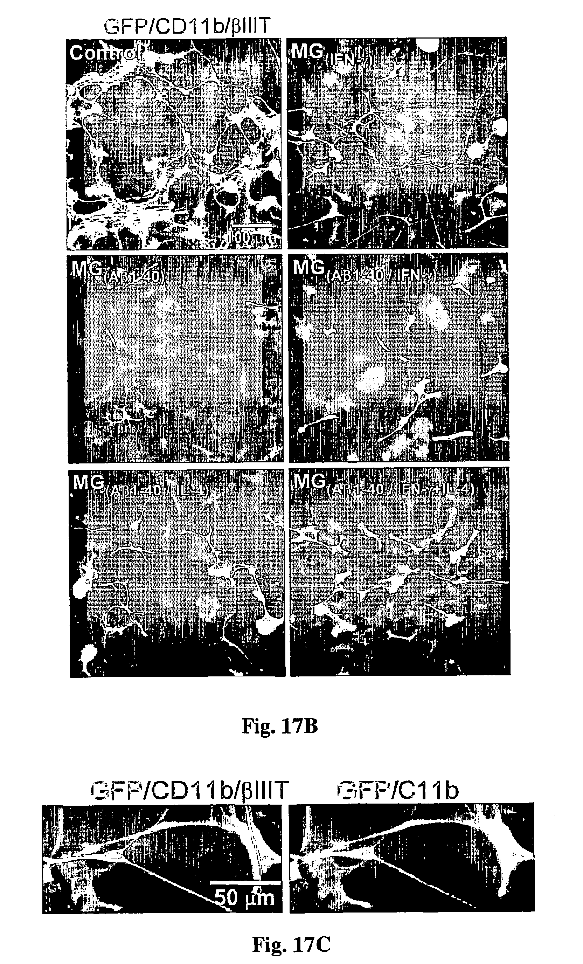

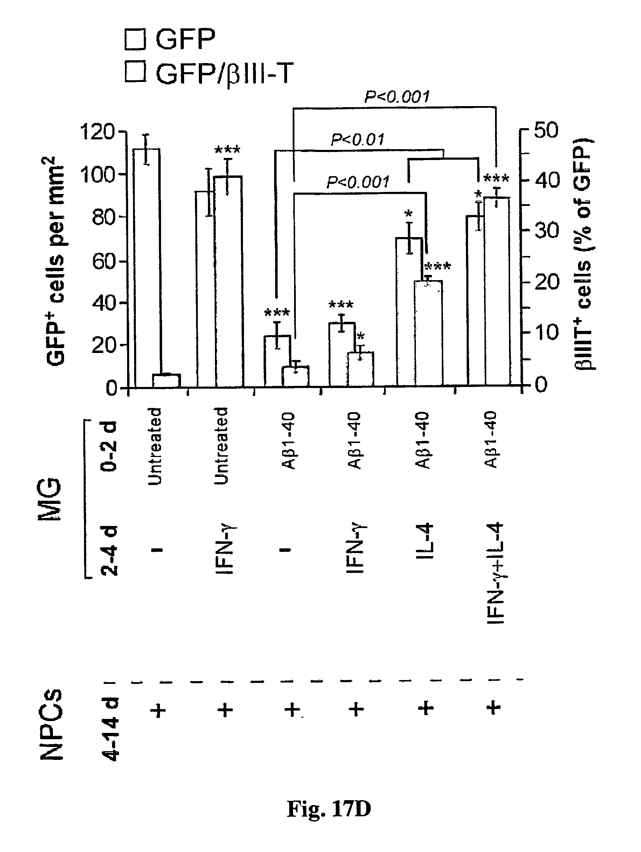

**Figs. 17A-17D** show that IL-4 can counteract the adverse effect of aggregated Aβ on microglial toxicity and promotion of neurogenesis. (Fig. 17A) *In-vitro* treatment paradigm. (Fig. 17B) Representative confocal images of NPCs expressing GFP and β-III-tubulin, co-cultured for 10 days without microglia (control), or with untreated microglia, or with microglia that were pre-activated with Aβ₍₁₋₄₀₎ (5 µM) (MG_{(Aβ1-40)}) for 48 h and subsequently activated with IFN-γ (10 ng/ml) (MG_{(Aβ1-40/IFNγ,10ng/ml)}), or with IL-4 (10 ng/ml) (MG_{(Aβ1-40/IL-4)}), or with both IFN-γ (10 ng/ml) and IL-4 (10 ng/ml) (MG_{(Aβ1-40 / IFNγ+IL-4)}). Note, aggregated Aβ induces microglia to adopt an ameboid-like morphology, but after IL-4 was added these microglia exhibited a ramified-like structure. (Fig. 17C) Separate confocal images of NPCs co-expressing GFP and β-III-tubulin adjacent to CD11b⁺ microglia. (Fig. 17D) Quantification of cells double-labeled with GFP and β-III-tubulin (expressed as a percentage of GFP⁺ cells) obtained from confocal images. Results are of three independent experiments in replicate cultures; bars represent means ± SEM. Asterisks above bars denote the significance of differences relative to untreated (control) NPCs (^{*} *P* < 0.05; *^{***} P* < 0.001; two-tailed Student's t-test). Horizontal lines with *P* values above them show differences between the indicated groups (ANOVA).

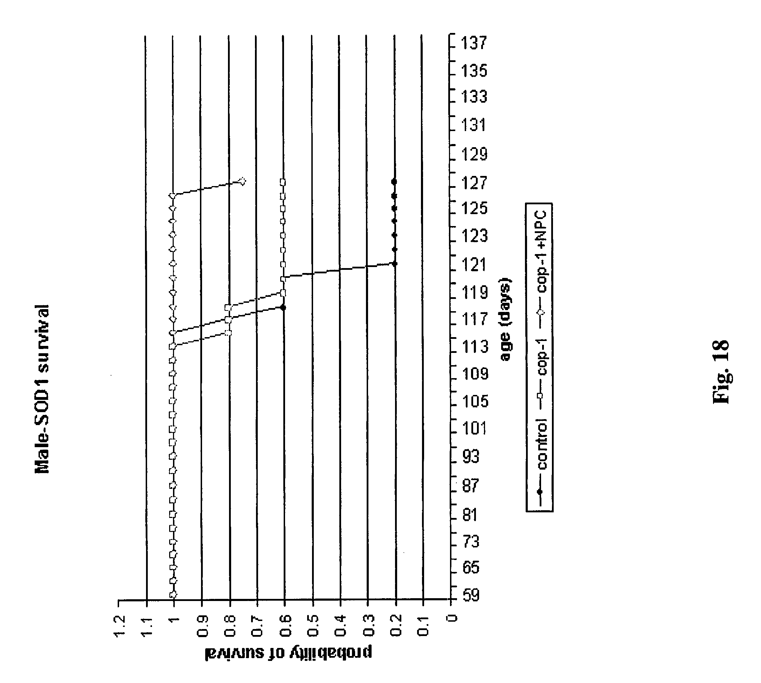

**Fig. 18** shows the effect of the administration of stem cells in combination with glatiramer acetate to a mice model of amyotrophic lateral sclerosis (ALS).

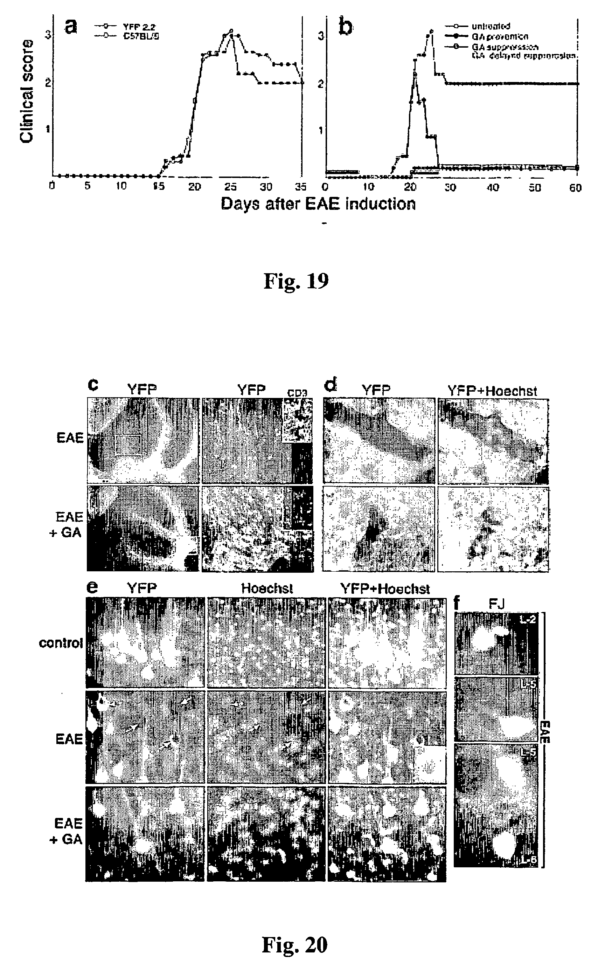

**Figs. 19A-19B** show clinical manifestations of EAE induced by MOG peptide 35-55. (Fig. 19A) In C57BL/6 and YFP2.2 mice. (Fig. 19B) The effect of GA treatment in C57BL/6 mice treated by 5-8 daily injections of GA in different stages of the disease i.e. starting immediately after disease induction - prevention treatment, starting after the appearance of disease manifestations at day 20 - suppression treatment, or during the chronic phase 6 weeks after disease appearance - delayed suppression. The injection period of each treatment is illustrated along the x axis (n=6).
**Figs. 20A-20E** show histological manifestations of EAE induced by MOG peptide 35-55. (Figs. 20A-20D) The effect of GA treatment in sagital brain sections of YFP2.2 mice expressing YFP (green) on their neuronal population: (Fig. 20A) Deterioration and transaction of YFP expressing fibers in the cerebellum and correlation with perivascular infiltration. Inserts indicate area with perivascular infiltrations, demonstrated by staining with antibodies for the T-cell marker - CD3. (Fig. 20B) Elimination of fibers in lesions in the striatum. (Fig. 20C) Typical morphology of pyramidal cells in layer 5 of the cerebral cortex. Arrows and insert indicate abnormal neuronal cell bodies with marginalized nuclei in EAE mice. Considerably less damages were found in brains of EAE+GA mice than in brains of untreated EAE mice i.e. less deteriorating fibers, reduced number of lesions with smaller magnitude, and less swollen cell nuclei. Note the thin layer of YFP positive fiber, frequently found over the lesions in GA treated mice. (Fig. 20D) Staining with Fluoro-Jade B (green), which binds to degenerating neurons, in the cortex of C57BL/6 mice, 25 days after disease induction. Scale bar indicates: 500 µm in (Fig. 20A) 50 µm in (Figs. 20B, 20C) and 20 µm in (Fig. 20E). L-2, L-5 and L-6, layer two, five and six of the cerebral cortex.

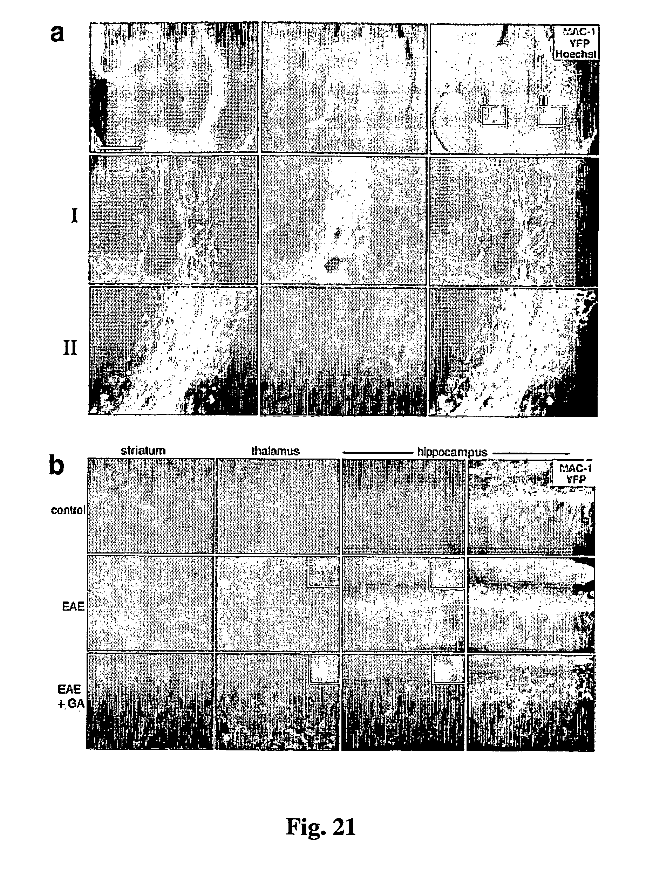

**Figs. 21A-21B** show microglial activation in EAE YFP2.2 mice. (Fig. 21A) Correlation of the expression of the microglia and macrophage marker MAC-1 (red) with deterioration and injury of YFP expressing fiber (green) in the white matter of the cerebellum. In box I, highly activated microglia cells are observed, accompanied by reduction in fiber density, whereas, in nearby area in box II low MAC-1 expression and normal fiber appearance are present. (Fig. 21B) The effect of GA on MAC-1 expression and on microglial cell morphology in various brain regions of EAE mice: striatum, thalamus (dorsal lateral geniculate nucleus) and hippocampus (granular and molecular layers). Increased MAC-1 staining and cell morphology typical for activated microglia were displayed in brains of EAE mice (inserts). In contrast, MAC-1 expression in brains of EAE+GA mice was extensively reduced exhibiting cell morphology similar to that of unactivated microglia in naive mice. EAE was induced in YFP2.2 mice, 35 days before perfusion. GA treatment was applied by 8 daily injections, starting immediately after EAE induction. Sagital sections. Scale bar indicates: (Fig. 21A) 500 µm, (Fig. 21B) 100 µm in the striatum and thalamus, 50µm in the hippocampus.

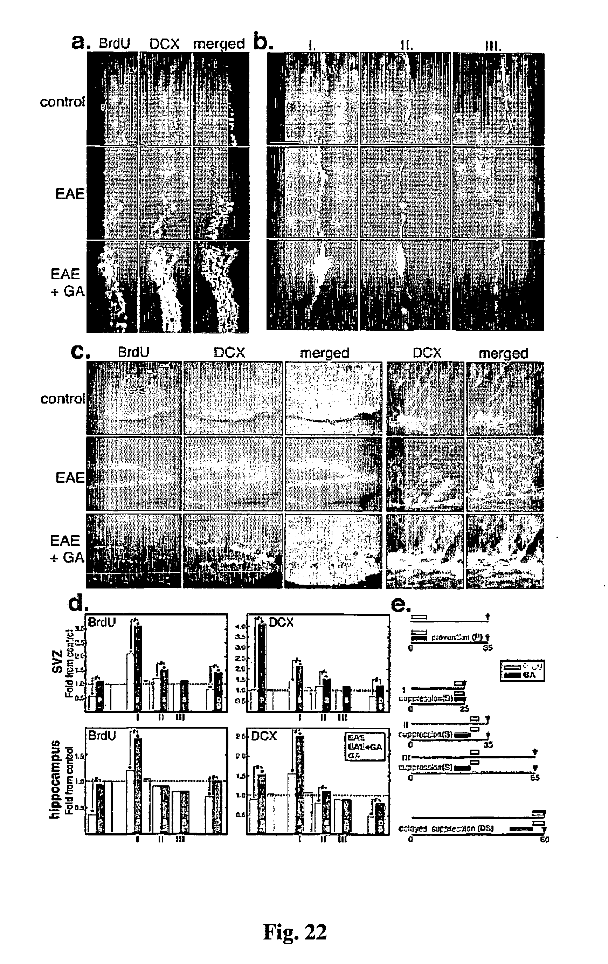

**Figs. 22A-22E** show proliferation of newly generated neurons visualized by immunostaining for the proliferation marker BrdU (red) and the immature neuronal marker DCX (green) in the neuroproliferative zones of C57BL/6 mice. Increased expression of BrdU and DCX in EAE mice and to a greater extent in EAE+GA mice, (Fig. 22A) in the SVZ, confocal images and (Fig. 22C) in the hippocampal SGZ, 25 days after EAE induction, 1 day after last GA injection. Note the DCX⁺ cells in the hippocumpus that migrated into the GCL and manifest dense and branched dendritic tree. (Fig. 22B) DCX expression in the SVZ at different times points: 1 day (I), 10 (II) and 30 (III) days after the last GA injection. Neuroproliferation declined with time, still, DCX expression in GA treated mice was higher than in EAE mice, 1 and 10 days after treatment. Coronal sections. Scale bar indicates: 50 µm in (Fig. 22A), 200 µm in (Fig. 22B) and (Fig. 22C), and 20 µm in the right panels of (Fig. 22C). st, striatum; LV, lateral ventricle; SGZ, subgranular zone; GCL, granular cell layer; IML, OML, inner and outer molecular layer. (Fig. 22D) Quantitative analysis of BrdU incorporation and DCX expression in EAE (red) and EAE+GA (blue) mice, at various time points after EAE induction and GA treatment. Increased neuronal proliferation is observed in both neuroproliferative zones following disease appearance; subsequent decline below that of naive mice, and augmentation of neuroproliferation by the various schedules of GA treatment. Quantification was performed in the SVZ by counting BrdU positive cells, (those with BrdU/DCX dual staining), and measuring the DCX stained area, starting at the level of the medial septum and 640 µm backward, and in the hippocampal DG by counting BrdU⁺/DCX⁺ cells (in both blades), and DCX⁺ cells (in the upper blade of the dentete), through its septo-temporal axis. The number of BrdU/DCX stained cells for each brain structure was averaged from 8 unilateral levels per mouse, 80 µm apart, 3-4 mice for treatment group. Results are expressed as change fold from naive controls. Control values for BrdU incorporation: in the SVZ 211±31 and 23±6, in the hippocampus 45±13 and 17+8, BrdU/DCX⁺ cells, one day and one month after the last BrdU injection respectively; for DCX staining: in the SVZ 19,464±3550µm² and in the hippocampus 78±12µm² positive cells averaged from 10 naïve mice. Statistical analysis was performed by ANOVA followed by Fisher's LSD when appropriate. * significant effect over naive control, # significant effect over EAE untreated mice, (p <0.05). (Fig. 22E) Schedule of experiments: time length from EAE induction (day 0) till perfusion; GA injections as prevention (P), suppression (S) or delayed suppression (DS) treatments, and BrdU inoculation - concurrently or immediately following GA treatment.

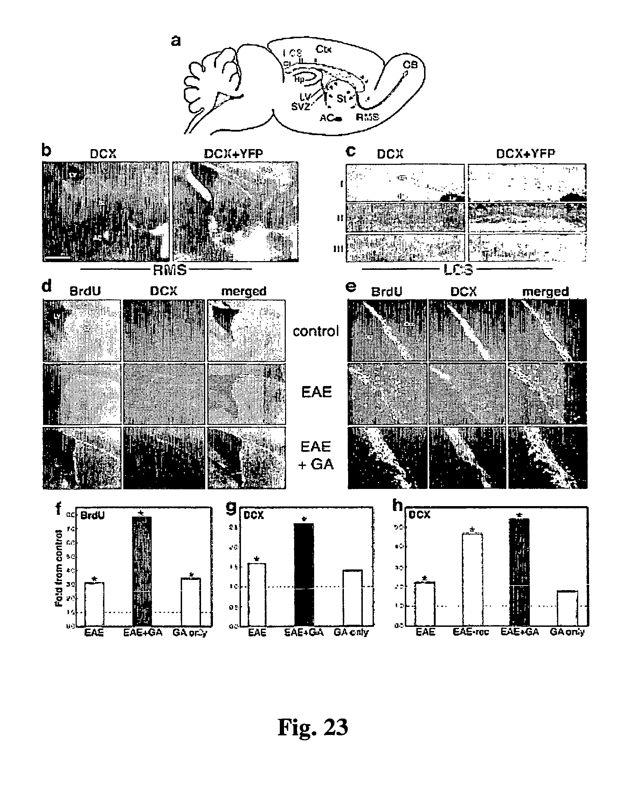

**Figs. 23A-23H** show promoted mobilization and migration of neuronal progenitor cells in EAE mice treated with GA through migratory streams. (Fig. 23A) Schematic sagital representation of the migratory routes from the subventricular zone (SVZ) through both the rostral migratory stream (RMS - in red) and the lateral cortical stream (LCS - in yellow). (Fig. 23B) Sagital section through the RMS showing the route of DCX-positive cells (red) from the SVZ to the OB. (Fig. 23C) neuroprogenitors in the LCS, generally functional in the embryonic forebrain, and reappear after GA treatment in EAE adult mice. DCX-positive cells (red) migrate alongside the YFP expressing fibers (green) of the interface between the hippocampus and the corpus callosum, towards various cortical regions mainly in the occipital cortex. (Figs. 23D-23E) Increased mobilization of newly generated neurons visualized with BrdU (orange) and DCX (green) immunostaining, in the RMS of EAE+GA mice, in comparison to EAE mice and naive controls, in RMS segment adjacent to the SVZ (Fig. 23D) and in a more medial section of the RMS arc (Fig. 23E). Sagital sections. Scale bar indicates: 1000 µm in (Fig. 23B), 25 in (Fig. 23C), 500 in and (Fig. 23D), 50 µm in (Fig. 23E). LV, lateral ventricle; Ctx, cortex; St, striatum; OB, olfactory bulb; AC, anterior commissure; cc, corpus callossum; Hip, hippocampus; L-5 and L-6, layer five and six of the cerebral cortex. (Figs. 23F-23H), Quantitative analysis of BrdU (co-expressing DCX) or DCX in the RMS, one day (Figs. 23F, 23G), and one month (Fig. 23H) after termination of BrdU and GA injections, indicating significant increase of neuroprogenitors in the RMS of EAE mice over control, and higher elevation in EAE+GA mice. Note that in one EAE mouse which exhibited slight, short-term disease and spontaneous recovery (EAE-rec, Fig. 23H), enhanced neuronal migration was observed. Quantification was performed by counting the BrdU⁺/DCX⁺ cells and measuring the DCX stained area (in 0.2² mm), along the striatal border. The amount of BrdU/DCX stained cells was averaged from 8 sections per mouse, 80 µm apart. Three mice counted per treatment group, except EAE rec, which shows a single mouse. Results are expressed as change fold from naive controls. Control values for BrdU incorporation: 146±31 BrdU⁺/DCX⁺ cells, one day after the last BrdU injection, and for DCX staining: 2193±305µm², averaged from 6 naïve mice. *p<0.05 versus naïve control. EAE mice in (Figs. 23B, 23C, 23H) were treated with GA subsequent to disease induction, one month before perfusion - prevention, in (Figs. 23D, 23E and 23F, 23G) EAE induced mice were injected with GA and BrdU 20 days post disease induction, 1-5 days before perfusion - suppression.

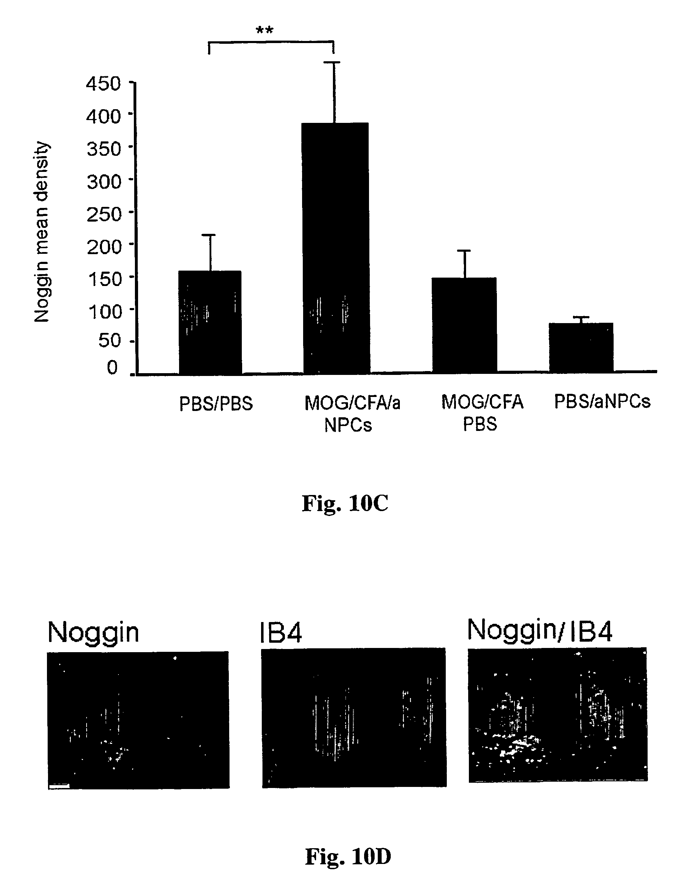

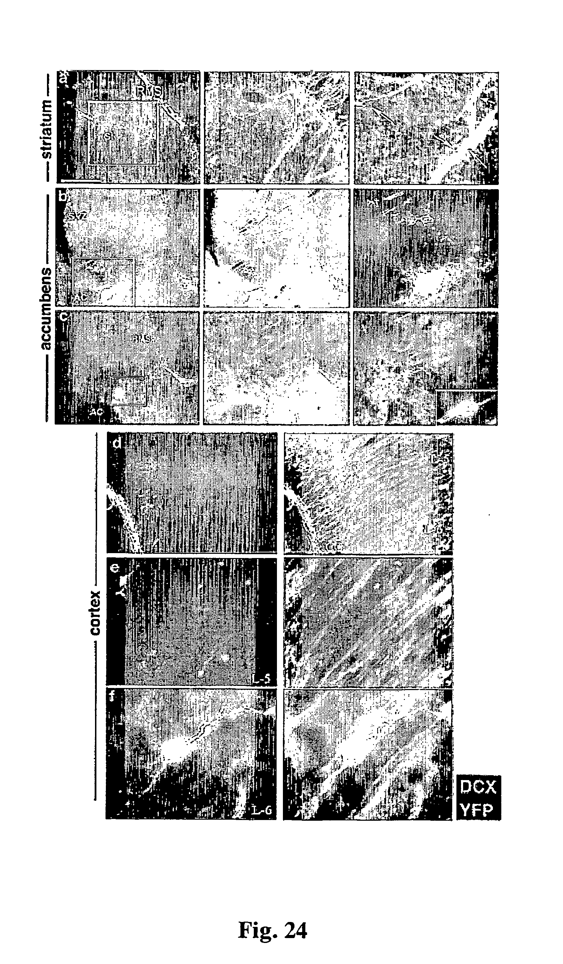

**Figs. 24A-24F** show migration of neuronal progenitor cells in EAE induced mice treated with GA. DCX expressing neuronal progenitors (orange) diverge from the classic neuroproliferative zones or the migratory streams and spread to atypical regions along YFP expressing fibers (green). (Fig. 24A) From the RMS into the in the striatum. (Figs. 24B, 24C) Towards the region of the nucleus accumbens, from the SVZ (Fig. 24B) and from the RMS (Fig. 24C). (Fig. 24D) From the RMS into the internal part of the cortex to - (Fig. 24E), layer 5, and (Fig. 24F), layer 6. Note the morphological features of the DCX expressing cells - fusiform somata with a leading and trailing processes (Fig. 24C insert and Figs. 24E, 24F), characteristic of migrating neurons, and their orientation - migration away from the migratory stream, along the nerve fibers (Figs. 24A 24D-24F). Sagital sections. Scale bar indicates: 200 µm in Figs, 24A-24D, 100 µm in Fig. 24E and 10 µm in Fig. 24F. In Figs. 24A-24C enlarged box area is depicted in the right panel. RMS, rostral migratory stream; SVZ, subventricular zone; St, striatum; AC, anterior commissure; cc, corpus callossum; L-5 and L-6, layer five and six of the cerebral cortex.

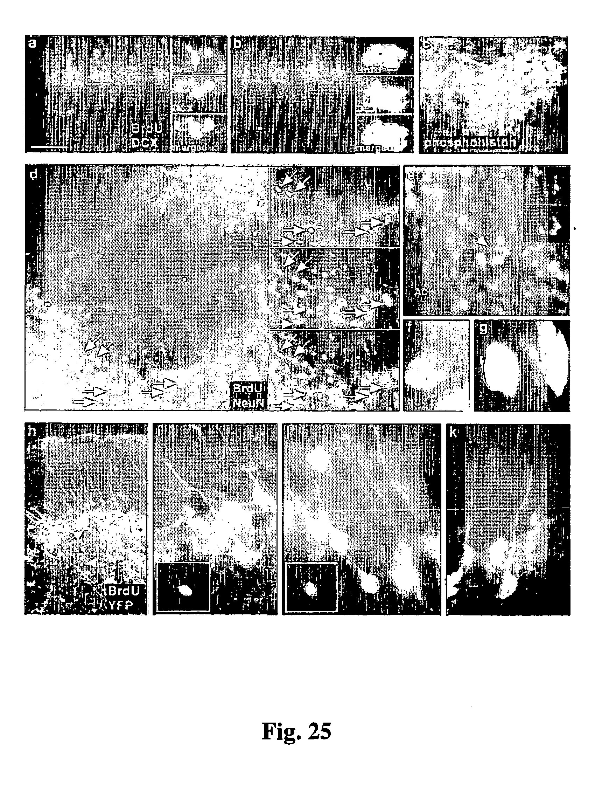

**Figs. 25A-25K** show Fate tracing of neuronal progenitor cells generated in the course of GA treatment in EAE mice. BrdU incorporated cells (red), born during the concurrent injections of BrdU and GA migrated to various brain regions and expressed neuronal markers. (Figs. 25A, 25B) BrdU positive cells co-expressing the immature neuronal marker DCX (green), 10 days after the last injection, in the striatum (Fig. 25A), and in the accombens nucleus (Fig. 25B). Note the clusters of double positive cells suggesting local divisions. (Fig. 25C) Staining of DCX expressing cells in the accombens nucleus with the endogenous proliferation marker phosphohiston (blue), showing DCX positive cells that had proliferated in situ prior to sacrifice of the mouse. (Figs. 25D-25G) BrdU positive cells co-expressing the mature neuronal marker NeuN (green), one month after completion of GA/BrdU injections, in the striatum (Fig. 25D, 25F), in the nucleus accumbens (Fig. 25E), and in the cingulate cortex layer 5 confocal image (Fig. 25G). Arrows indicate representative BrdU/NeuN co-expressing cells. (Figs. 25H, 25K) BrdU positive cells, one month after GA/BrdU injection in YFP mice, co-expressing YFP (green) in the cingulate layer 5 (Figs. 25H, 25I), occipital layer 6 (Fig. 25J), and motor layer 5 (Fig. 25K) of the cortex. Pyramidal cells with characteristic elongated apical dendrites and axons, indicative of mature functional neurons can be seen. Fig. 25G and Fig. 25K are confocal images. Sagital sections. Scale bar indicates: 200 µm in (Figs. 25A, 25B, 25H), 100 µm in Figs. 25C, 25D 50 µm in Figs. 25E, 25I-25K, 15 µm in Figs. 25F, 25G.

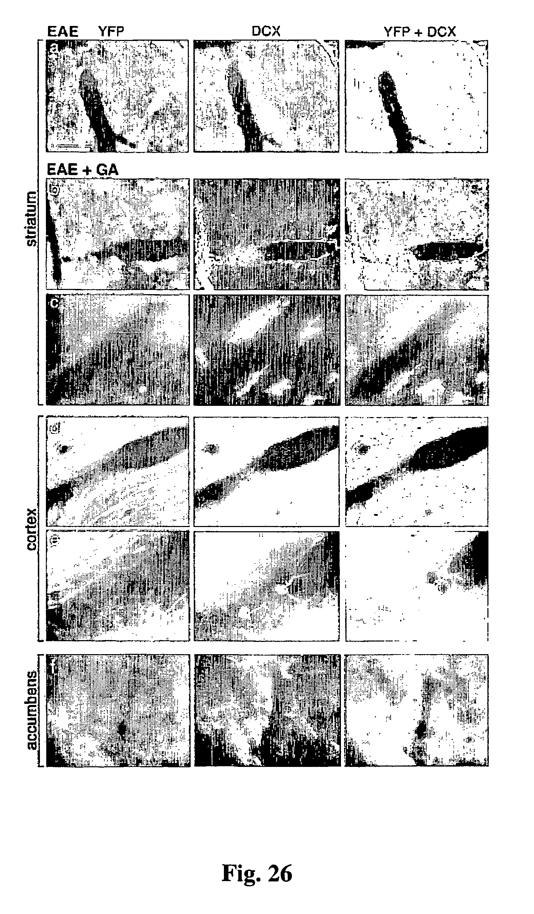

**Figs.26A-26F** show migration of neuronal progenitors to lesion sites. DCX expressing cells (orange) were found in injured regions with deterioration of YFP expressing fibers (green). (Fig. 26A), In EAE mice (not treated by GA), 35 days after disease induction, in the striatum. (Figs. 26B-26F), In EAE mice, 35 days after disease induction, treated by GA (8 daily injections, starting immediately after disease induction - prevention). DCX expressing cells diverging from the RMS towards a lesion in the striatum (Fig. 26B), surrounding a lesion in the striatum (Fig. 26C), inside a lesion in the frontal cortex layer 5/6 (Figs. 26D, 26E), and in a cluster surrounding a lesion in the accumbens nucleus (Fig. 26F). Lesions in GA-treated mice were less extensive than in untreated mice, yet the amount of progenitors adjoining these lesions was extensively higher. Note the YFP expressing fibers extending into the lesions and the axonal sprouting in lesions occupied by DCX expressing cells (Figs. 26D-26F). Sagital sections. Scale bar indicates: 100 µm in Figs. 26A, 26B, 50 µm in Figs. 26C, 26D, 26F, and 20 µm in Fig. 26E.

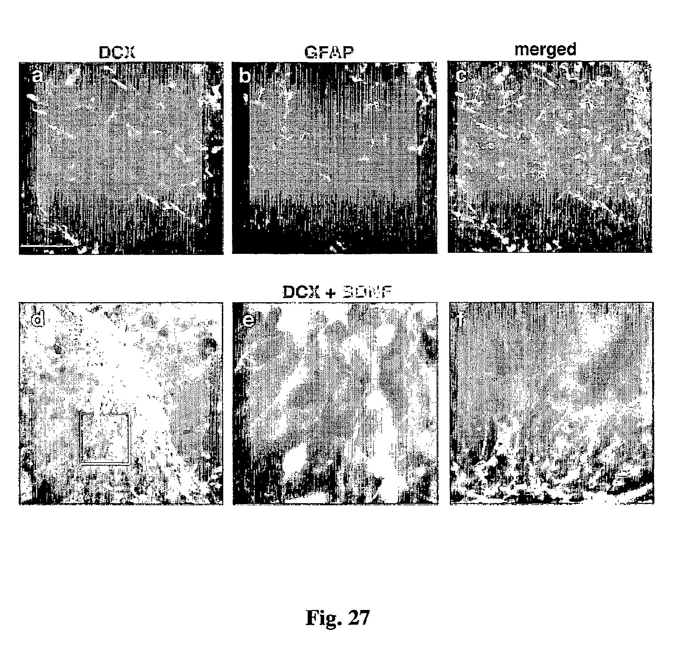

**Figs. 27A-27F** show that GA induced neuronal progenitors migrate to gliotic scar areas and express *in situ* BDNF. Figs. 27A-27C, DCX expressing cells (green), in regions populated with GFAP expressing astrocytes (red), in the striatum. Figs. 27D-27F, DCX expressing cells (green) manifest extensive expression of BDNF (orange) in the nucleus accombance (Fig. 27D, 27E) and the hippocampal dentate gyrus (Fig. 27F). Coronal sections. Scale bar indicates: 100 µm in Fig. 27A-27C, 50 µm in Fig. 27D, 12 µm in Fig. 27E and 30 µm in Fig. 27F.

### DETAILED DESCRIPTION OF THE INVENTION

While trying to elucidate the effect of EAE induction on neurogenesis and differentiation towards the neural lineage and to investigate whether peripheral immunomodulatory treatment with GA injection in various stages of disease has any effect on neurogenesis and neuroprotective processes, it was found by some of the inventors that in EAE mice neuroproliferation was elevated following disease appearance, but subsequently declined below that of naive mice. In contrast, GA treatment led to sustained reduction in the neuronal/axonal damage and augmented neuroprogenitor proliferation and mobilization. The newborn neuroprogenitors manifested massive migration through exciting and dormant migration pathways, into injury sites in brain regions, which do not normally undergo neurogenesis, and differentiated to mature neuronal phenotype, endorsing a direct linkage between immunomodulation, neurogenesis and therapeutic consequence in the CNS.

Research during the last decade has disclosed that the brain is potentially capable of cell renewal throughout life, albeit to a limited extent (Morshead et al., 1994). However, the mechanisms that might restrict or favor the renewal of adult neural cells are not known. Recent studies from the laboratory of the present inventors have shown that after an injury to the CNS a local immune response that is properly controlled in time, space, and intensity by the peripheral adaptive immunity is a pivotal requirement for posttraumatic neuronal survival (Moalem et al., 1999; Butovsky et al., 2001; Schwartz et al., 2003; Shaked et al., 2004). We therefore envisaged the possibility that the lack of neurogenesis and the restricted recovery might be attributable to a common factor, which might in turn be related to the local immune response.

The present invention is based on the assumption of some of the inventors that well-regulated adaptive immunity is needed for cell renewal in the brain. It was thus postulated that neurogenesis and oligodendrogenesis are induced and supported by microglia that encounter cytokines associated with adaptive immunity, but are not supported by naïve microglia and are blocked by microglia that encounter endotoxin.

In fact, it is shown herein that certain specifically activated microglia can induce and support neural cell renewal. Thus, both neurogenesis and oligodendrogenesis were induced and supported in NPCs co-cultured with microglia activated by the cytokines IL-4 and IFN-γ, both associated with adaptive immunity. In contrast, microglia exposed to LPS blocked both neurogenesis and oligodendrogenesis, in line with previous reports that MG_{(LPS)} block cell renewal (Monje et al., 2003).

Defense mechanisms in the form of activated microglia are often seen in acute and chronic neurodegenerative conditions, and the CNS is poorly equipped to tolerate them (Dijkstra et al., 1992). As a result, activated microglia have generally been viewed as a uniformly hostile cell population that causes inflammation, interferes with cell survival (Popovich et al., 2002), and blocks cell renewal (Monje et al., 2002, 2003).

Recent studies have shown, however, that the type of activation determines microglial activity, and that just as their effects can be inimical to cell survival in some circumstances, they can be protective in others. Thus, for example, microglia that encountered adaptive immunity (CD4⁺ T cells) were shown to acquire a protective phenotype (Butovsky et al., 2001). Among the cytokines that are produced by such T cells and can endow microglia with a neuroprotective phenotype are IFN-γ and IL-4, characteristic of Th1 and Th2 cells, respectively. Thus, microglia exposed to activated Th1 cells or to IFN-γ show increased uptake of glutamate, a key player in neurodegenerative disorders (Shaked et al., unpublished observation), while their exposure to IL-4 results in down-regulation of TNF-α, a common player in the destructive microglial phenotype, and up-regulation of insulin-like growth factor (IGF-1) (shown herein in the examples), which promotes differentiation of oligodendrocytes from multipotent adult neural progenitor cells (Hsieh et al., 2004). In addition, IGF-1 prevents the acute destructive effect of glutamate-mediated toxicity on oligodendrocytes *in vitro* (Ness et al., 2002) and inhibits apoptosis of mature oligodendrocytes during primary demyelination (Mason et al., 2000). These and other findings strongly suggest that the outcome of the local immune response (in terms of its effect on the microglia) in the damaged CNS will be either beneficial or harmful, depending on how the microglia interpret the threat.

In general, tissue repair is a process that is well synchronized in time and space, and in which immune activity is needed to clear the site of the lesion and create the conditions for migration, proliferation, and differentiation of progenitor cells for renewal. In light of the well-known fact that constitutive cell renewal is limited in the CNS, as well as the reported observations that treatment with MG_{(LPS)} causes neuronal loss (Boje et al., 1992) and interferes with the homing and differentiation of NPCs (Monje et al., 2003), and that adaptively activated microglia can support neuronal survival, it is not surprising to discover that immune conditions favoring neuronal survival will also support cell renewal. MG_{(LPS)} produce excessive amounts of NO (causing oxidative stress) and TNF-α, as well as other cytotoxic elements, leading to a spiral of worsening neurotoxicity (Boje et al., 1992). NO was found to act as an important negative regulator of cell proliferation and neurogenesis in the adult mammalian brain (Packer et al., 2003), and TNF-α has an inhibitory effect on oligodendrogenesis (Cammer et al., 1999).

The results of the present invention show that MG_{(LPS)} are indeed detrimental to NPC survival and differentiation, but that when microglia are activated by cells or cytokines possessing adaptive immune function, not only are they not cytotoxic but they even exert a positive effect on NPC proliferation, inducing and supporting their differentiation into neurons or oligodendrocytes. *In vivo,* injection of MG_{(IL-4)} into rat brain lateral ventricles resulted in no neuronal loss, minimal migration of microglia to the CNS parenchyma, and the appearance of new neurons and oligodendrocytes (indicated by the double-staining of BrdU⁺ cells with markers of neurons or oligodendrocytes). Staining for microglia revealed significant invasion of the healthy CNS by MG_{(LPS)}, with consequent massive tissue loss, unlike in the case of MG_{(IL-4)} or MG₍₋₎. Interestingly, in the non-injected hippocampus, resident microglia were found adjacent to the subventricular zone. It is tempting to speculate that these might be the cells responsible for controlling neurogenesis, restraining it when in their resting state (as found in the present work using MG₍₋₎), but inducing and supporting it when suitably activated.

Our findings are supported by the observation that in mice with experimental autoimmune encephalomyelitis (EAE), NPCs migrate to sites of CNS damage (Pluchino et al., 2003). They are also in line with the common experience that cell renewal is favored by injury, since they imply that in the absence of injury the conditions that might favor renewal do not exist.

Renewal of cells and their replenishment by new growth is the common procedure for tissue repair in most tissues of the body. It was thought that in the brain those processes do not occur, and therefore that any loss of neurons, being irreplaceable, results in functional deficits that range from minor to devastating. Since an insult to the CNS, whether acute or chronic, is often followed by the postinjury spread of neuronal damage, much research has been devoted to finding ways to minimize this secondary degeneration by rescuing as many neurons as possible.

The results of the present invention lead us to an intriguing conclusion. First, under pathological conditions (when cell renewal is critical), not only do the microglia not favor cell renewal, but they interfere with it. Secondly, this paradoxical situation can be remedied by well-controlled adaptive immunity, which shapes the microglia in such a way that their activity is not cytotoxic but is both protective and conducive to renewal. This indicates that in those cases in which protective autoimmunity leads to improved recovery, both neurogenesis and gliogenesis are likely to occur. These data can also explain the lack of cell renewal in autoimmune diseases; in such cases, it is likely that the quantity of circulating autoimmune T cells exceeds the threshold above which TNF-α production, due to an excess of IFN-γ, does not allow the microglia to acquire a protective phenotype. They can also explain why steroids are not helpful, as their anti-inflammatory activity masks not only the destructive but also the beneficial adaptive immunity. The therapy of choice for both autoimmune diseases and neurodegenerative conditions would therefore appear to be immunomodulation in which, after the acute phase of disease, the surviving tissue can be maintained by relatively small quantities of T cells.

The findings of the present invention indicate that the limitation of spontaneous, endogenous neurogenesis and oligodendrogenesis in the adult brain is, at least in part, an outcome of the local immune activity, and that harnessing of adaptive immunity rather than immunosuppression is the path to choose in designing ways to promote cell renewal in the CNS.

Cell renewal in the adult mammalian CNS is limited. Recent studies suggest that it is arrested by inflammation. That view is challenged by the findings of the present invention that microglia, depending on environmental stimulation, can either induce and support or block such renewal. *In vitro,* neurogenesis and oligodendrogenesis from neural progenitor cells were shown herein to be promoted by mouse microglia that encountered T-cell-associated cytokines (IFN-γ, IL-4), but were blocked by microglia that encountered endotoxin. Anti-IGF-1 antibodies neutralized the IL-4 effect, while anti-TNF-α antibodies augmented the effect of IFN-γ. Injection of IL-4-activated microglia into cerebral ventricles of adult rats induced significant hippocampal neurogenesis and cortical oligodendrogenesis, whereas endotoxin-activated microglia caused neuronal loss and blocked neurogenesis and oligodendrogenesis. These results strengthen our assumption that controlled adaptive immunity, unlike uncontrolled (e.g. endotoxin-induced) inflammation, activates microglia to induce and support neuronal and oligodendrocyte survival and renewal. Thus, to promote cell renewal in the CNS, well-controlled immunity is needed and should not be suppressed.

It has been reported that the controlled activity of T cells directed to autoantigens in the CNS is needed for postinjury survival and repair (Moalem et al., 1999; Yoles et a., 2001; Kipnis et al., 2002). These results led us to suspect that a fundamental role of autoimmune T cells, known to be present in healthy individuals, is to help maintain the integrity of the CNS, and that their remedial effect in a neurodegenerative environment is a manifestation of the same restorative role under extreme conditions. Moreover, accumulating evidence attesting to the participation of such autoimmune T cells in postinjury neuronal survival led us to postulate that if they have a similar role in the healthy CNS, it might well have to do with neurogenesis in adult life, possibly by maintaining the conditions needed for such cell renewal.

According to the present invention, we examined how the nature of microglial activation affects neurogenesis in the adult rat hippocampus under physiological and pathological conditions associated with brain inflammation. Transient inflammatory conditions associated with transient accumulation of myelin-specific Th1 cells promoted neurogenesis. Injection of microglia (MG) activated by IFN-γ (MG_{(IFN-γ)}) or by IL-4 (MG_{(IL-4)}) into the lateral ventricles of the brains of healthy rats promoted neurogenesis. In rats that developed monophasic (transient) EAE, the induced neurogenesis was further promoted by MG_{(IL-4)}. Our results *in vitro* showed that MG_{(IFN-γ)} supported neurogenesis from adult rat NPCs as long as the IFN-γ concentration was low. The impediment to neurogenesis imposed by high-dose IFN-γ could be counteracted by IL-4. Neurogenesis induced by IL-4 was weaker, however, than that induced by low-dose or by high-dose administered in combination with IL-4.

We have identified herein cellular elements in the CNS that can respond to local environmental changes and needs, and consequently can support the formation of new cells from adult aNPCs. We demonstrated that once the microglia become suitably activated by circulating T cell-derived cytokines, they can induce neuronal and oligodendroglial differentiation from aNPCs. In view of that observation, and our previous demonstration in rodents that a T cell-based vaccination promotes recovery from contusive spinal cord injury (SCI), we postulated that translation of those findings into a therapeutic approach might benefit the repair process by creating a niche-like neurogenic/gliogenic environment at the injured site. Thus, we expected to find that supplementing the vaccination by transplantation of homologous aNPCs would further promote functional recovery after SCI. In the present invention we in fact demonstrated, using a mouse model, synergistic interaction between T cell-based immune activation and transplanted aNPCs in promoting functional motor recovery after contusive injury of the spinal cord.

Previous studies by the inventor M. Schwartz have shown that systemic manipulations of the immune system, based on increasing the numbers of T cells directed to weak agonists of autoantigens, beneficially affect neurodegenerative conditions by promoting neuronal survival (Moalem et al., 1999; Hauben et al., 2001; Schwartz and Kipnis, 2002). The same manipulations, for example, T cell-based vaccination, is proposed here for increasing neurogenesis, yielding novel ways to maintain the integrity of the aging brain and the diseased mind.

It thus seems that maintenance and repair of brain cells necessitate a dialog between CNS-autoreactive T cells and brain-resident microglia. This dialog cannot take place, however, unless the microglia are able to act as APCs, presenting the relevant antigens to the homing T cells. We therefore postulated that in order to halt the progression of Alzheimer disease (AD), T cells that recognize CNS-specific antigens other than aggregated amyloid-β (Aβ) must target sites of aggregated Aβ plaques in the brain. On reaching these sites they become activated by the encounter with their specific antigens, presented to them by microglia acting as APCs. Such activation enables these T cells to offset the negative effect of aggregated Aβ on locally resident microglia, thus preventing the latter from becoming cytotoxic to neurons and blocking neurogenesis. We tested this hypothesis by vaccinating AD mice with glatiramer acetate (GA, also known as copolymer 1 or Cop-1), a synthetic copolymer approved by the FDA for treatment of multiple sclerosis, and capable of weakly cross-reacting with a wide range of CNS-resident autoantigens (Kipnis et al., 2000). GA-activated T cells, after infiltrating the CNS, have the potential to become locally activated without risk of the overwhelming proliferation that is likely to cause an autoimmune disease. Studies by the present inventors and others have shown that GA can simulate the protective and reparative effects of autoreactive T cells (Kipnis et al., 2000; Benner et al., 2004).

In the present invention, APP/PS1 double-transgenic AD mice (which coexpress mutated human presenilin 1 and amyloid-β precursor protein) suffering from decline in cognition and accumulation of Aβ plaques, a T cell-based vaccination, by altering the microglial phenotype, ameliorated cognitive performance, reduced plaque formation, rescued cortical and hippocampal neurons, and induced hippocampal neurogenesis.

We show here that vaccination of Tg mice with GA reduced plaque formation, and prevented and even partially reversed cognitive decline, even if the vaccination was given after some loss of cognition and some plaque formation had already occurred. It should be noted that vaccination with GA was effective not only in preventing disease progression but also-when administered after onset of the clinical symptoms of learning/memory loss and pathological appearance of plaques-in promoting tissue repair. The above findings are in line with our observation that in mice deficient in CNS-autoreactive T cells the expression of brain-derived neurotrophic factor (BDNF), known to be associated with both cognitive activity and cell renewal, is impaired. They are also in accord with our observation that T cells are needed for the maintenance of ccognitive functioning in the healthy as well as in the diseased brain (Kipnis et al., 2004). Since aggregated Aβ evidently interferes with the ability of microglia to engage in dialog with T cells, its presence in the brain can be expected to cause loss of cognitive ability and impairment of neurogenesis. Homing of CNS-autoreactive T cells to the site of disease or damage in such cases is critical, but will be effective only if those T cells can counterbalance the destructive activity of the aggregated Aβ. Myelin-presenting microglia, with which myelin-specific T cells can readily hold a dialog, are likely to be present in abundance. Myelin-related antigens, or antigens (such as GA) that are weakly cross-reactive with myelin, are therefore likely to be the antigens of choice for the therapeutic vaccination. Myelin-specific T cells will then home to the CNS and, upon encountering their relevant APCs there, will become locally activated to supply the cytokines and growth factors needed for appropriate modulation of harmful microglia like those activated by aggregated Aβ. The resulting synapse between T cells and microglia will create a supportive niche for cell renewal by promoting neurogenesis from the pool of adult stem cells, thereby overcoming the age-related impairment induced in the inflammatory brain.

The known features of glatiramer acetate (GA, Copolymer 1) are also of essence in relation to stem cell transplantation for the treatment of neurological and other disorders. It is therefore proposed to use GA to improve the therapeutic outcome of stem cell transplantation for various disorders including neurological diseases, especially MS. The rationale for GA usage is for this purpose is based on previous results of the inventors R. Aharoni and R. Arnon concerning its mechanism of action as well on their findings of its efficacy in reducing graft and bone marrow rejection and self-neurogenerating effects. It is thus envisioned that GA will be effective not only in augmenting endogenous neurogenesis of self-neuroprogenitor cells but also exogenous neurogenesis of transplanted multipotent (stem) progenitor cells. These manifestations of GA function taken together with its very high safety profile, support its application in combination therapy for the improvement of progenitor stem cell transplantation for many clinical applications, in addition to those specifically related to neurological disorders.

There is thus disclosed a method for inducing and enhancing neurogenesis and/or oligodendrogenesis from endogenous as well as from exogenously administered stem cells, which comprises administering to an individual in need thereof an agent selected from the group consisting of Copolymer 1, a Copolymer 1-related polypeptide, a Copolymer 1-related peptide, and activated T cells which have been activated by Copolymer 1, a Copolymer 1-related polypeptide, or a Copolymer 1-related peptide.

The method as disclosed herein further includes proliferation, differentiation and survival of newly formed neurons or oligodendrocytes, and includes neuronal progenitor proliferation, neuronal migration, and/or neuronal differentiation of newly formed neurons into mature neurons.

There is also disclosed a method for inducing and enhancing neurogenesis from endogenous or exogenously applied stem cells.

It is also disclosed that the method is for inducing and augmenting self-neurogenesis in damaged or injured brain regions, both in brain regions that normally undergo neurogenesis and in brain regions that normally do not undergo neurogenesis such as striatum, nucleus accumbens and/or cortex.

There is also disclosed a method for inducing and augmenting self-neurogenesis including neuronal progenitor proliferation, neuronal migration, and/or neuronal differentiation of newly formed neurons into mature neurons, in the central nervous system (CNS), which comprises administering to an individual in need an agent selected from the group consisting of Copolymer 1, a Copolymer 1-related polypeptide and a Copolymer 1-related peptide.

There is also disclosed a method for inducing and enhancing oligodendrogenesis from endogenous or exogenously applied stem cells.

There is also disclosed a method for inducing and enhancing oligodendrogenesis from endogenous or exogenously applied stem cells, by immune modulation, which comprises administering to an individual in need a neuroprotective agent selected from the group consisting of of Copolymer 1, a Copolymer 1-related polypeptide, a Copolymer 1-related peptide, and activated T cells which have been activated by Copolymer 1, a Copolymer 1-related polypeptide, or a Copolymer 1-related peptide.

It is disclosed that the agent for use in the context of the present invention are T cells which have been activated by Copolymer 1, a Copolymer 1-related polypeptide, or a Copolymer 1-related peptide. The T cells can be endogenous and activated *in vivo* by administration of the antigen or peptide, thereby producing a population of T cells that accumulate at a site of injury or disease of the CNS or PNS, or the T cells are prepared from T lymphocytes isolated from the blood and then sensitized to the antigen. The T cells are preferably autologous, most preferably of the CD4 and/or CD8 phenotypes, but they may also be allogeneic T cells from related donors, e.g., siblings, parents, children, or HLA-matched or partially matched, semi-allogeneic or fully allogeneic donors. Methods for the preparation of said T cells are described in the above-mentioned WO 99/60021.

The methods as disclosed herein are useful for inducing and enhancing neurogenesis and/or oligodendrogenesis both from endogenous and exogenously administered stem cells and and may assist in solving the problems found today with the poor results of stem cell transplantation, particularly in the cases of injuries, diseases, disorders and conditions of the nervous system, both the CNS and PNS.

It is disclosed that the method as disclosed herein is applied to induce and enhance neurogenesis and/or oligodendrogenesis from endogenous pools of neural stem/progenitor cells. Thus, Copolymer 1, a Copolymer 1-related polypeptide, a Copolymer 1-related peptide, and T cells activated therewith will by themselves boost endogenous neurogenesis and oligodendrogenesis in damaged tissues, supporting also the survival of the new neurons and oligodendrocytes.

It is also disclosed that the method as disclosed herein is applied to induce and enhance neurogenesis and/or oligodendrogenesis from both endogenous and exogenous stem cells administered to the patient. The administration of said neuroprotective agent will assist to enhance the successful engraftment of the implanted stem cells, cell renewal and differentiation of the stem cells into neurons and/or oligodendrocytes, while at the same time inducing the endogenous neurogenesis and oligodendrogenesis in the damaged tissues and supporting the survival of the new neurons and oligodendrocytes.

Thus, there is also disclosed a method of stem cell therapy comprising transplantation of stem cells in combination with a neuroprotective agent to an individual in need thereof, wherein said neuroprotective agent is selected from the group consisting of Copolymer 1, a Copolymer 1-related polypeptide, a Copolymer 1-related peptide, and activated T cells which have been activated by Copolymer 1, a Copolymer 1-related polypeptide, or a Copolymer 1-related peptide.

It is disclosed that the methods as disclosed herein are applied to individuals suffering from an injury, disease, disorder or condition of the central nervous system (CNS) or peripheral nervous system (PNS).

The CNS or PNS injury to be treated according to the methods as disclosed herein, preferably by stem cell therapy, include spinal cord injury, closed head injury, blunt trauma, penetrating trauma, hemorrhagic stroke, ischemic stroke, cerebral ischemia, optic nerve injury, myocardial infarction or injury caused by tumor excision. The transplanted stem cells will migrate to the region of the injury where cells had died (for example, due to ischaemia) and will differentiate into neurons and/or oligodendrocytes.

The CNS or PNS diseases, disorders or conditions to be treated according to the methods as disclosed herein, preferably by stem cell therapy, include Parkinson's disease and Parkinsonian disorders, Huntington's disease, Alzheimer's disease, multiple sclerosis, or amyotrophic lateral sclerosis (ALS). Other diseases, disorders or conditions include facial nerve (Bell's) palsy, glaucoma, Alper's disease, Batten disease, Cockayne syndrome, Guillain-Barré syndrome, Lewy body disease, Creutzfeldt-Jakob disease, or a peripheral neuropathy such as a mononeuropathy or polyneuropathy selected from the group consisting of adrenomyeloneuropathy, alcoholic neuropathy, amyloid neuropathy or polyneuropathy, axonal neuropathy, chronic sensory ataxic neuropathy associated with Sjogren's syndrome, diabetic neuropathy, an entrapment neuropathy nerve compression syndrome, carpal tunnel syndrome, a nerve root compression that may follow cervical or lumbar intervertebral disc herniation, giant axonal neuropathy, hepatic neuropathy, ischemic neuropathy, nutritional polyneuropathy due to vitamin deficiency, malabsorption syndromes or alcoholism, porphyric polyneuropathy, a toxic neuropathy caused by organophosphates, uremic polyneuropathy, a neuropathy associated with a disease or disorder selected from the group consisting of acromegaly, ataxia telangiectasia, Charcot-Marie-Tooth disease, chronic obstructive pulmonary diseases, Fabry's disease, Friedreich ataxia, Guillain-Barré syndrome, hypoglycemia, IgG or IgA monoclonal gammopathy (non-malignant or associated with multiple myeloma or with osteosclerotic myeloma), lipoproteinemia, polycythemia vera, Refsum's syndrome, Reye's syndrome, and Sjogren-Larsson syndrome, a polyneuropathy associated with various drugs, with hypoglycemia, with infections such as HIV infection, or with cancer; epilepsy, amnesia, anxiety, hyperalgesia, psychosis, seizures, oxidative stress, opiate tolerance and dependence, and for the treatment of a psychosis or psychiatric disorder selected from the group consisting of an anxiety disorder, a mood disorder, schizophrenia or a schizophrenia-related disorder, drug use and dependence and withdrawal, and a memory loss or cognitive disorder.

It is disclosed that the method of cell therapy as disclosed herein is applied to injuries, diseases, disorders or conditions unrelated to the nervous system. In one preferred embodiment, the method is suitable for bone marrow-derived stem cell transplantation for treatment of an injury, disease, disorder or condition selected from diabetes, failure of tissue repair, myocardial infarction, kidney failure, liver cirrhosis, muscular dystrophy, skin burn, leukemia, arthritis injury, or osteoporosis injury.

The stem cells for use in the methods as disclosed herein include, but are not limited to, adult stem cells, embryonic stem cells with the proviso that said embryonic stem cells are not human embryonic stem cells , umbilical cord blood stem cells, hematopoietic stem cells, peripheral blood stem cells, mesenchymal stem cells, multipotent stem cells, neural stem cells, neural progenitor cells, stromal stem cells, progenitor cells, or precursors thereof, and genetically-engineered stem cells, and any other stem cells that may be found suitable for the purpose of the present invention. Examples of such cells include the CNS neural stems cells disclosed in US 6,777,233 and US 6,680,198; the neural stem cells and hematopoietic cells disclosed in US 6,749,850 for administration with neural stimulants; and the stromal cells disclosed in US 6,653,134 for treatment of CNS diseases.

As used herein, the term "neural stem cell" is used to describe a single cell derived from tissue of the central nervous system, or the developing nervous system, that can give rise *in vitro* and/or *in vivo* to at least one of the following fundamental neural lineages: neurons (of multiple types), oligodendroglia and astroglia as well as new neural stem cells with similar potential. "Multipotent" or "pluripotent" neural stem cells are capable of giving rise to all of the above neural lineages as well as cells of equivalent developmental potential.

It is disclosed that the neural stem cells may be human neural stem cells that can be isolated from both the developing and adult CNS, and can be successfully grown in culture, are self-renewable, and can generate mature neuronal and glial progeny. Embryonic human neural stem cells can be induced to differentiate into specific neuronal phenotypes. Human neural stem cells integrate into the host environment after transplantation into the developing or adult CNS. Human neural stem cells transplanted into animal models of Parkinson's disease and spinal cord injury have induced functional recovery. However, there are still problems with the engraftment of said cells and the present invention will enhance the successful engraftment, survival and further differentiation of the implanted cells. In a most preferred embodiment, the neural stem cells are autologous.

As used herein, the term "hematopoietic stem cells" refer to stem cells that can give rise to cells of at least one of the major hematopoietic lineages in addition to producing daughter cells of equivalent potential. Certain hematopoietic stem cells are capable of giving rise to many other cell types including brain cells.

The stem cells, once isolated, are cultured by methods known in the art, for example as described in US 5,958,767, US 5,270,191, US 5,753,506

The treatment regimen according to the present disclosure is carried out, in terms of administration mode, timing of the administration, and dosage, depending on the type and severity of the injury, disease or disorder and the age and condition of the patient. The immunomodulator may be administered concomitanly with, before or after the injection or implantation of the cells.

The administration of the cells may be carried out by various methods. In certain embodiments, the cells are preferably administered directly into the stroke cavity, the spinal fluid, e.g., intraventricularly, intrathecally, or intracisternally. The stem cells can be formulated in a pharmaceutically acceptable liquid medium, which can contain the Copolymer 1 or the T cells as well. Cells may also be injected into the region of the brain surrounding the areas of damage, and cells may be given systemically, given the ability of certain stem cells to migrate to the appropriate position in the brain.

It is disclosed that the method as disclosed herein comprises stem cell therapy by administration of stem cells in combination with Copolymer 1. It is disclosed that the stem cells are injected/transplanted to the patient, followed by vaccination with Copolymer 1. It is also disclosed that a combination of the stem cells with the Copolymer 1 may be injected/transplanted to the patient. It is also disclosed that the stem cells can be cultured *in vitro* (artificially) with the

### Copolymer 1 and differentiated prior to transplantation

As used herein in the application, the terms "Cop 1", "Copolymer 1", "glatiramer acetate" and "GA" are used interchangeably.

For the purpose of the present invention, " Copolymer 1 or a Copolymer 1-related peptide or polypeptide" is intended to include any peptide or polypeptide, including a random copolymer that cross-reacts functionally with MBP and is able to compete with MBP on the MHC class II in the antigen presentation.

The composition for use in the invention may comprise as active agent a Cop 1 or a Cop 1-related peptide or polypeptide represented by a random copolymer consisting of a suitable ratio of a positively charged amino acid such as lysine or arginine, in combination with a negatively charged amino acid (preferably in a lesser quantity) such as glutamic acid or aspartic acid, optionally in combination with a non-charged neutral amino acid such as alanine or glycine, serving as a filler, and optionally with an amino acid adapted to confer on the copolymer immunogenic properties, such as an aromatic amino acid like tyrosine or tryptophan. Such compositions may include any of those copolymers disclosed in WO 00/05250, the entire contents of which are herewith incorporated herein by reference.

More specifically, the composition for use in the present invention comprises at least one copolymer selected from the group consisting of random copolymers comprising one amino acid selected from each of at least three of the following groups: (a) lysine and arginine; (b) glutamic acid and aspartic acid; (c) alanine and glycine; and (d) tyrosine and tryptophan.

The copolymers for use in the present invention can be composed of L- or D-amino acids or mixtures thereof. As is known by those of skill in the art, L-amino acids occur in most natural proteins. However, D-amino acids are commercially available and can be substituted for some or all of the amino acids used to make the copolymers used in the present invention. The present invention contemplates the use of copolymers containing both D- and L-amino acids, as well as copolymers consisting essentially of either L- or D-amino acids.

In one embodiment of the invention, the copolymer contains four different amino acids, each from a different one of the groups (a) to (d).

In a more preferred embodiment, the pharmaceutical composition or vaccine of the invention comprises Copolymer 1, a mixture of random polypeptides consisting essentially of the amino acids L-glutamic acid (E), L-alanine (A), L-tyrosine (Y) and L-lysine (K) in an approximate ratio of 1.5:4.8:1:3.6, having a net overall positive electrical charge and of a molecular weight from about 2 KDa to about 40 KDa.

In one preferred embodiment, the Cop 1 has average molecular weight of about 2 KDa to about 20 KDa, more preferably of about 4,7 KDa to about 13 K Da, still more preferably of about 4 KDa to about 8.6 KDa, of about 5 KDa to 9 KDa, or of about 6.25 KDa to 8.4 KDa. In another preferred embodiment, the Cop 1 has average molecular weight of about 13 KDa to about 20 KDa, more preferably of about 13,5 KDa to about 18 KDa, with an average of about 15 KDa to about 16 KD, preferably of 16kDa. Other average molecular weights for Cop 1, lower than 40 KDa, are also encompassed by the present invention. Copolymer 1 of said molecular weight ranges can be prepared by methods known in the art, for example by the processes described in U.S. Patent No. 5,800,808

. The Copolymer 1 may be a polypeptide comprising from about 15 to about 100, preferably from about 40 to about 80, amino acids in length.

In one preferred embodiment of the invention, the agent is Cop 1 in the form of its acetate salt known under the generic name glatiramer acetate or its trade name Copaxone® (a trademark of Teva Pharmaceutical Industries Ltd., Petach Tikva, Israel).

The activity of Copolymer 1 for the composition disclosed herein is expected to remain if one or more of the following substitutions is made: aspartic acid for glutamic acid, glycine for alanine, arginine for lysine, and tryptophan for tyrosine.

In another embodiment of the invention, the Cop 1-related peptide or polypeptide is a copolymer of three different amino acids each from a different one of three groups of the groups (a) to (d). These copolymers are herein referred to as terpolymers.

In one embodiment, the Cop 1-related peptide or polypeptide is a terpolymer containing tyrosine, alanine, and lysine, hereinafter designated YAK, in which the average molar fraction of the amino acids can vary: tyrosine can be present in a mole fraction of about 0.05-0.250; alanine in a mole fraction of about 0.3 - 0.6; and lysine in a mole fraction of about 0.1-0.5. More preferably, the molar ratios of tyrosine, alanine and lysine are about 0.10:0.54:0.35, respectively. It is possible to substitute arginine for lysine, glycine for alanine, and/or tryptophan for tyrosine.

In another embodiment, the Cop 1-related peptide or polypeptide is a terpolymer containing tyrosine, glutamic acid, and lysine, hereinafter designated YEK, in which the average molar fraction of the amino acids can vary: glutamic acid can be present in a mole fraction of about 0.005 - 0.300, tyrosine can be present in a mole fraction of about 0.005-0.250, and lysine can be present in a mole fraction of about 0.3-0.7. More preferably, the molar ratios of glutamic acid, tyrosine, and lysine are about 0.26:0.16:0.58, respectively. It is possible to substitute aspartic acid for glutamic acid, arginine for lysine, and/or tryptophan for tyrosine.

In another preferred embodiment, the Cop 1-related peptide or polypeptide is a terpolymer containing lysine, glutamic acid, and alanine, hereinafter designated KEA, in which the average molar fraction of the amino acids can vary: glutamic acid can be present in a mole fraction of about 0.005-0.300, alanine in a mole fraction of about 0.005-0.600, and lysine can be present in a mole fraction of about 0.2 - 0.7. More preferably, the molar ratios of glutamic acid, alanine and lysine are about 0.15:0.48:0.36, respectively. It is possible to substitute aspartic acid for glutamic acid, glycine for alanine, and/or arginine for lysine.

In a preferred embodiment, the Cop 1-related peptide or polypeptide is a terpolymer containing tyrosine, glutamic acid, and alanine, hereinafter designated YEA, in which the average molar fraction of the amino acids can vary: tyrosine can be present in a mole fraction of about 0.005-0.250, glutamic acid in a mole fraction of about 0.005-0.300, and alanine in a mole fraction of about 0.005-0.800. More preferably, the molar ratios of glutamic acid, alanine, and tyrosine are about 0.21: 0.65:0.14, respectively. It is possible to substitute tryptophan for tyrosine, aspartic acid for glutamic acid, and/or glycine for alanine.

The average molecular weight of the terpolymers YAK, YEK, KEA and YEA can vary between about 2 KDa to 40 KDa, preferably between about 3 KDa to 35 KDa, more preferably between about 5 KDa to 25 KDa.

Copolymer 1 and related peptides and polypeptides may be prepared by methods known in the art, for example, under condensation conditions using the desired molar ratio of amino acids in solution, or by solid phase synthetic procedures. Condensation conditions include the proper temperature, pH, and solvent conditions for condensing the carboxyl group of one amino acid with the amino group of another amino acid to form a peptide bond. Condensing agents, for example dicyclohexylcarbodiimide, can be used to facilitate the formation of the peptide bond. Blocking groups can be used to protect functional groups, such as the side chain moieties and some of the amino or carboxyl groups against undesired side reactions.

For example, the copolymers can be prepared by the process disclosed in U.S. Patent 3,849,550, wherein the N-carboxyanhydrides of tyrosine, alanine, γ-benzyl glutamate and N ε-trifluoroacetyl-lysine are polymerized at ambient temperatures (20°C-26°C) in anhydrous dioxane with diethylamine as an initiator. The γ-carboxyl group of the glutamic acid can be deblocked by hydrogen bromide in glacial acetic acid. The trifluoroacetyl groups are removed from lysine by 1M piperidine. One of skill in the art readily understands that the process can be adjusted to make peptides and polypeptides containing the desired amino acids, that is, three of the four amino acids in Copolymer 1, by selectively eliminating the reactions that relate to any one of glutamic acid, alanine, tyrosine, or lysine.

The molecular weight of the copolymers can be adjusted during polypeptide synthesis or after the copolymers have been made. To adjust the molecular weight during polypeptide synthesis, the synthetic conditions or the amounts of amino acids are adjusted so that synthesis stops when the polypeptide reaches the approximate length that is desired. After synthesis, polypeptides with the desired molecular weight can be obtained by any available size selection procedure, such as chromatography of the polypeptides on a molecular weight sizing column or gel, and collection of the molecular weight ranges desired. The copolymers can also be partially hydrolyzed to remove high molecular weight species, for example, by acid or enzymatic hydrolysis, and then purified to remove the acid or enzymes.

In one embodiment, the copolymers with a desired molecular weight may be prepared by a process, which includes reacting a protected polypeptide with hydrobromic acid to form a trifluoroacetyl-polypeptide having the desired molecular weight profile. The reaction is performed for a time and at a temperature that is predetermined by one or more test reactions. During the test reaction, the time and temperature are varied and the molecular weight range of a given batch of test polypeptides is determined. The test conditions that provide the optimal molecular weight range for that batch of polypeptides are used for the batch. Thus, a trifluoroacetyl-polypeptide having the desired molecular weight profile can be produced by a process, which includes reacting the protected polypeptide with hydrobromic acid for a time and at a temperature predetermined by test reaction. The trifluoroacetyl-polypeptide with the desired molecular weight profile is then further treated with an aqueous piperidine solution to form a low toxicity polypeptide having the desired molecular weight.

In a preferred embodiment, a test sample of protected polypeptide from a given batch is reacted with hydrobromic acid for about 10-50 hours at a temperature of about 20-28°C. The best conditions for that batch are determined by running several test reactions. For example, in one embodiment, the protected polypeptide is reacted with hydrobromic acid for about 17 hours at a temperature of about 26°C.

As binding motifs of Cop 1 to MS-associated HLA-DR molecules are known (Fridkis-Hareli et al, 1999), polypeptides derived from Cop 1 having a defined sequence can readily be prepared and tested for binding to the peptide binding groove of the HLA-DR molecules as described in the Fridkis-Hareli et al (1999) publication. Examples of such peptides are those disclosed in WO 00/05249 and WO 00/05250 , and include the peptides of SEQ ID NOs. 1-32 hereinbelow.

| **SEQ ID NO.** | **Peptide Sequence** |
|---|---|
| 1 | AAAYAAAAAAKAAAA |
| 2 | AEKYAAAAAAKAAAA |
| 3 | AKEYAAAAAAKAAAA |
| 4 | AKKYAAAAAAKAAAA |
| 5 | AEAYAAAAAAKAAAA |
| 6 | KEAYAAAAAAKAAAA |
| 7 | AEEYAAAAAAKAAAA |
| 8 | AAEYAAAAAAKAAAA |
| 9 | EKAYAAAAAAKAAAA |
| 10 | AAKYEAAAAAKAAAA |
| 11 | AAKYAEAAAAKAAAA |
| 12 | EAAYAAAAAAKAAAA |
| 13 | EKKYAAAAAAKAAAA |
| 14 | EAKYAAAAAAKAAAA |
| 15 | AEKYAAAAAAAAAAA |
| 16 | AKEYAAAAAAAAAAA |
| 17 | AKKYEAAAAAAAAAA |
| 18 | AKKYAEAAAAAAAAA |
| 19 | AEAYKAAAAAAAAAA |
| 20 | KEAYAAAAAAAAAAA |
| 21 | AEEYKAAAAAAAAAA |
| 22 | AAEYKAAAAAAAAAA |
| 23 | EKAYAAAAAAAAAAA |
| 24 | AAKYEAAAAAAAAAA |
| 25 | AAKYAEAAAAAAAAA |
| 26 | EKKYAAAAAAAAAAA |
| 27 | EAKYAAAAAAAAAAA |
| 28 | AEYAKAAAAAAAAAA |
| 29 | AEKAYAAAAAAAAAA |
| 30 | EKYAAAAAAAAAAAA |
| 31 | AYKAEAAAAAAAAAA |
| 32 | AKYAEAAAAAAAAAA |

Such peptides and other similar peptides derived from Cop 1 would be expected to have similar activity as Cop 1. Such peptides, and other similar peptides, are also considered to be within the definition of Cop 1-related peptides or polypeptides and their use is considered to be part of the present invention.

The definition of "Cop 1-related peptide or polypeptide" according to the invention is meant to encompass other synthetic amino acid copolymers such as the random four-amino acid copolymers described by Fridkis-Hareli et al., 2002 (as candidates for treatment of multiple sclerosis), namely copolymers (14-, 35- and 50-mers) containing the amino acids phenylalanine, glutamic acid, alanine and lysine (poly FEAK), or tyrosine, phenylalanine, alanine and lysine (poly YFAK), and any other similar copolymer to be discovered that can be considered a universal antigen similar to Cop 1.

The dosage of Cop 1 to be administered will be determined by the physician according to the age of the patient and stage of the disease and may be chosen from a range of 1-80 mg, preferably 20 mg, although any other suitable dosage is encompassed by the invention. The treatment should be preferably carried out by administration of repeated doses at suitable time intervals, according to the neurodegenerative disease to be treated, the age and condition of the patient. In one embodiment, Cop 1 may be administered daily. In another embodiment, the administration may be made according to a regimen suitable for immunization, for example, at least once a month or at least once every 2 or 3 months, or less frequently, but any other suitable interval between the immunizations is envisaged by the invention according to the condition of the patient.

Pharmaceutical compositions for use in accordance with the present invention may be formulated in conventional manner using one or more physiologically acceptable carriers or excipients. The carrier(s) must be "acceptable" in the sense of being compatible with the other ingredients of the composition and not deleterious to the recipient thereof.

Methods of administration include, but are not limited to, parenteral, e.g., intravenous, intraperitoneal, intramuscular, subcutaneous, mucosal (e.g., oral, intranasal, buccal, vaginal, rectal, intraocular), intrathecal, topical and intradermal routes, with or without adjuvant. Administration can be systemic or local.

The invention will now be illustrated by the following non-limiting examples.

### EXAMPLE 1

### Microglia induce neural cell renewal - Microglia activated by IL-4 or IFN-γ differentially induce neurogenesis and oligodendrogenesis from adult stem/ progenitor cells

### Materials and Methods

*(i) **Animals.*** Tneonatal (P0-P1) C57B1/6J mice were supplied by the Animal Breeding Center of the Weizmann Institute of Science (Rehovot, Israel). All animals were handled according to the regulations formulated by the Weizmann Institute's Animal Care and Use Committee.
***(ii)*** ***Reagents.*** Lipopolysaccharide (LPS) (containing <1% contaminating proteins) was obtained from *Escherichia coli* 0127:B8 (Sigma-Aldrich, St. Louis, MO). Recombinant mouse tumor necrosis factor (TNF)-α and insulin-like growth factor (IGF)-I (both containing endotoxin at a concentration below 1 EU per µg of cytokine), recombinant rat and mouse interferon (IFN)-γ and interleukin (IL)-4 (both containing endotoxin at a concentration below 0.1 ng per µg of cytokine), goat anti-mouse neutralizing anti-TNF-α antibodies (ãTNF-α; containing endotoxin at a concentration below 0.001 EU per µg of Ab), and goat anti-mouse neutralizing anti-IGF-I (ãIGF-I; containing endotoxin at a concentration below 0.1 EU per µg of Ab) were obtained from R&D Systems (Minneapolis, MN).
***(iii)*** ***Neural progenitor cell (NPC) culture.*** Coronal sections (2 mm thick) of tissue containing the subventricular zone of the lateral ventricle were obtained from the brains of adult C57B16/J mice. The tissue was minced and then incubated for digestion at 37°C, 5% CO₂ for 45 min in Earle's balanced salt solution containing 0.94 mg/ml papain (Worthington, Lakewood, NJ) and 0.18 mg/ml of L-cysteine and EDTA. After centrifugation at 110 x g for 15 min at room temperature, the tissue was mechanically dissociated by pipette trituration. Cells obtained from single-cell suspensions were plated (3500 cells/cm²) in 75-cm² Falcon tissue-culture flasks (BD Biosciences, Franklin Lakes, NJ), in NPC-culturing medium [Dulbecco's modified Eagles's medium (DMEM)/F12 medium (Gibco/Invitrogen, Carlsbad, CA) containing 2 mM L-glutamine, 0.6% glucose, 9.6 µg/ml putrescine, 6.3 ng/ml progesterone, 5.2 ng/ml sodium selenite, 0.02 mg/ml insulin, 0.1 mg/ml transferrin, 2 µg/ml heparin (all from Sigma-Aldrich, Rehovot, Israel), fibroblast growth factor-2 (human recombinant, 20 ng/ml), and epidermal growth factor (human recombinant, 20 ng/ml; both from Peprotech, Rocky Hill, NJ)]. Spheres were passaged every 4-6 days and replated as single cells. Green fluorescent protein (GFP)-expressing neural progenitor cells (NPCs) were obtained as previously described (Pluchino et al., 2003).
***(iv) Primary microglial culture.*** Brains from neonatal (P0-P1) C57B1/6J mice were stripped of their meninges and minced with scissors under a dissecting microscope (Zeiss, Stemi DV4, Germany) in Leibovitz-15 medium (Biological Industries, Beit Ha-Emek, Israel). After trypsinization (0.5% trypsin, 10 min, 37°C/5% CO₂), the tissue was triturated. The cell suspension was washed in culture medium for glial cells [DMEM supplemented with 10% fetal calf serum (FCS; Sigma-Aldrich, Rehovot), L-glutamine (1 mM), sodium pyruvate (1 mM), penicillin (100 U/ml), and streptomycin (100 mg/ml)] and cultured at 37°C/5% CO₂ in 75-cm² Falcon tissue-culture flasks (BD Biosciences) coated with poly-D-lysine (PDL) (10 mg/ml; Sigma-Aldrich, Rehovot) in borate buffer (2.37 g borax and 1.55 g boric acid dissolved in 500 ml sterile water, pH 8.4) for 1 h, then rinsed thoroughly with sterile, glass-distilled water. Half of the medium was changed after 6 h in culture and every 2^{nd} day thereafter, starting on day 2, for a total culture time of 10-14 days. Microglia were shaken off the primary mixed brain glial cell cultures (150 rpm, 37°C, 6 h) with maximum yields between days 10 and 14, seeded (10⁵ cells/ml) onto PDL-pretreated 24-well plates (1 ml/well; Coming, Coming, NY), and grown in culture medium for microglia [RPMI-1640 medium (Sigma-Aldrich, Rehovot) supplemented with 10% FCS, L-glutamine (1 mM), sodium pyruvate (1 mM), β-mercaptoethanol (50 mM), penicillin (100 U/ml), and streptomycin (100 mg/ml)]. The cells were allowed to adhere to the surface of a PDL-coated culture flask (1 h, 37°C/5% CO₂), and non-adherent cells were rinsed off.
***(v) Co-culturing of neural progenitor cells* (*NPCs*) *and mouse Microglia.*** Microglia were treated for 24 h with cytokines (IFN-γ, 20 ng/ml; IL-4, 10 ng/ml) or LPS (100 ng/ml). Cultures of treated or untreated microglia were washed twice with fresh NPC-differentiation medium (same as the culture medium for NPCs but without growth factors and with 2.5% FCS) to remove all traces of the tested reagents, then incubated on ice for 15 min, and shaken at 350 rpm for 20 min at room temperature. Microglia were removed from the flasks and immediately co-cultured (5 × 10⁴ cells/well) with NPCs (5 × 10⁴ cells/well) for 5 or 10 days on cover slips coated with Matrigel (BD Biosciences) in 24-well plates, in the presence of NPC differentiation medium, with or without insulin. The cultures were then fixed with 2.5% paraformaldehyde in PBS for 30 min at room temperature and stained for neuronal and glial markers. Cell proliferation rates and cell survival *in vitro* were determined by staining with 5-bromo-2'-deoxyuridine (BrdU, 2.5 µM; Sigma-Aldrich, St. Louis). For quantification of live and dead cells, live cultures were stained with 1 µg/ml propidium iodide (Molecular Probes, Invitrogen, Carlsbad, CA) and 1 µg/ml Hoechst 33342 (Sigma-Aldrich, St. Louis), and cells were counted using Image-Pro (Media Cybernetics, Silver Spring, MD), as described (Hsieh et al., 2004)
***(vi) Immunocytochemistry.*** Cover slips from co-cultures of NPCs and mouse microglia were washed with PBS, fixed as described above, treated with a permeabilization/blocking solution containing 10% FCS, 2% bovine serum albumin, 1% glycine, and 0.1% Triton X-100 (Sigma-Aldrich, Rehovot) and stained with a combination of the mouse or rabbit anti-tubulin β-III-isoform C-terminus antibodies (β-III-tubulin; 1:500), rabbit anti-NG2 chondroitin sulfate proteoglycan (NG2; 1:500), mouse anti-RIP (RIP; 1:2000), mouse anti-galactocerebroside (GalC; 1:250), mouse anti-glutamic acid decarboxylase 67 (GAD; 1:1000), mouse anti-nestin (Nestin; 1:1000), rat anti-myelin basic protein (MBP; 1:300) (all from Chemicon, Temecula, CA), goat anti-double cortin (DCX; 1:400, Santa Cruz Biotechnology, Santa Cruz, CA) and mouse anti-glial fibrillary acidic protein (GFAP; 1:100, Sigma-Aldrich, St. Louis). For labeling of microglia we used either rat andi-CD11b (MAC1; 1:50, BD-Pharmingen, NJ) or FITC-conjugated *Bandeiraea simplicifolia* isolectin B4 (IB4; 1:50, Sigma-Aldrich, Rehovot). Expression of IGF-1 was detected by goat anti-IGF-1 (1:20, R&D Systems).
***(vii) RNA purification, cDNA synthesis***, ***and reverse-transcription PCR analysis***. Cells were lysed with TRI reagent (MRC, Cincinnati, OH), and total cellular RNA was purified from lysates using the RNeasy kit (Qiagen, Hilden, Germany) according to the manufacturer's instructions. Residual genomic DNA was removed during the purification process by incubation with RNase-free DNase (Qiagen). RNA was stored in RNase-free water (Qiagen) at -80°C. RNA (1 µg) was converted to cDNA using SuperScript II (Promega, Madison, WI), as recommended by the manufacturer. The cDNA mixture was diluted 1:5 with PCR-grade water.

We assayed the expression of specific mRNAs using semi-quantitative reverse transcription PCR (RT-PCR) with selected gene-specific primer pairs, using OLIGO v6.4 (Molecular Biology Insights, Cascade, CO).

The primers used were:
TNF-α, sense 5'-AGGAGGCGCTCCCCAAAAAGATGGG-3' (SEQ ID NO:33),
antisense 5'-GTACATGGGCTCATACCAGGGCTTG-3' (target size, 551 bp) (SEQ ID NO:34);
IGF-I, sense 5'-CAGGCTCCTAGCATACCTGC-3' (SEQ ID NO:35),
antisense 5'-GCTGGTAAAGGTGAGCAAGC-3' (target size, 244 bp) (SEQ ID NO:36); and
β-actin, sense 5'-TTGTAACCAACTGGGACGATATGG-3' (SEQ ID NO:37), antisense 5'-GATCTTGATCTTCATGGTGCTAGG-3' (target size, 764 bp) (SEQ ID NO:38).

The RT-PCR reactions were carried out using 1 µg of cDNA, 35 nmol of each primer, and ReadyMix PCR Master Mix (ABgene, Epsom, UK) in 30-µl reactions. PCR reactions were carried out in an Eppendorf PCR system with cycles (usually 25-30) of 95°C for 30 s, 60°C for 1 min, 72°C for 1 min, and 72°C for 5 min, and then kept at 4°C. As an internal standard for the amount of cDNA synthesized, we used β-actin mRNA. PCR products were subjected to agarose gel analysis and visualized by ethidium bromide staining. Signals were quantified using a Gel-Pro analyzer 3.1 (Media Cybernetics). In all cases one product was observed with each primer set, and the observed product had an amplicon size that matched the size predicted from published cDNA sequences.
***(viii) Quantification.*** For microscopic analysis we used a Zeiss LSM 510 confocal laser scanning microscope (40x magnification). For experiments *in vitro* we scanned fields of 0.053 mm² (n = 8-16 from at least two different coverslips) for each experimental group. For each marker, 500-1000 cells were sampled. Cells co-expressing GFP and β-III-tubulin, NG2, RIP, GalC, and GFAP were counted.
***(ix) Statistical analysis.*** The results were analyzed by the Tukey-Kramer multiple comparisons test (ANOVA) and are expressed as means ± SD (unless differently indicated).

### Example 1(1). Effect of microglia on neurogenesis in vitro - Microglia pretreated with IL-4 or IFN-γ induce and support neuronal differentiation from neural progenitor cells (NPCs) in vitro.

Adaptive immunity, in the form of a well-controlled Th1 or a Th2 response to a CNS insult, induces microglia (MG) to adopt a phenotype that facilitates neuronal protection and neuronal tissue repair (Butovsky et al., 2001). Here we examined the ability of adaptive immunity, via activation of microglia, to induce or support the differentiation of NPCs. Neurogenesis is reportedly blocked by the inflammation caused by microglia activated with endotoxin (such as lipopolysaccharide, LPS) (Ekdahl et al., 2003). We therefore compared the effects on NPCs of microglia exposed to LPS (MG_{(LPS)}) with the effects of microglia exposed to the low levels of characteristic Th1 (pro-inflammatory) and Th2 (antiinflammatory) cytokines, IFN-γ (MG_{(IFN-γ)}) and IL-4 (MG_{(IL-4γ)}), respectively, shown herein to be supportive of neural survival. We used NPCs expressing green fluorescent protein (GFP) to verify that any neural cell differentiation seen in the culture was derived from the NPCs rather than from contamination of the primary microglial culture.

Microglia were grown in their optimal growth medium (Zielasek et al., 1992) and were then treated for 24 h with IL-4, IFN-γ (low level), or LPS. Residues of the growth medium and the cytokines were washed off, and each of the treated microglial preparations, as well as a preparation of untreated microglia (MG₍₋₎), was freshly co-cultured with dissociated NPC spheres in the presence of differentiation medium. We examined the effects of both IFN-γ-activated and IL-4-activated microglia. After 5 days in culture, GFP⁺ cells that expressed the neuronal marker β-III tubulin were identified as neurons.

Since we recently showed that IL-4-activated microglia (MG_{(IL-4)}) produce a high level of IGF-1 (Butovsky et al., 2005), and because IGF-1 is reportedly a key factor in neural cell renewal (O'Kusky et al., 2000), we envisioned a situation in which IGF-1 might be one of the factors in the effect of IL-4-activated microglia. Therefore, the following experiments were carried out both in insulin-free (to allow detection, if exists, of the effect of insulin-related factors secreted by the activated microglia) and in insulin-containing differentiation media.

Quantitative analysis revealed that neurogenesis, in the absence of insulin, was only minimally supported by MG_{(IFN-γ)} and was impaired by MG_{(LPS)}, but was almost 3-fold higher in NPCs co-cultured with MG_{(IL-4)} than in controls **(****Fig. 1A****).** In the presence of insulin the picture was somewhat different: MG_{(IFN-γ)} were significantly more effective than MG₍₋₎ in inducing neurogenesis, whereas the inductive effect of MG_{(IL-4)} and the blocking effect of MG_{(LPS)} on neurogenesis were similar to their effects in the absence of insulin (compare **Fig. 1B****).** In the absence of microglia, addition of insulin (0.02 mg/ml) did not increase the numbers of GFP⁺/β-III-tubulin⁺ cells in NPC cultures **(****Fig. 1A****).**

In co-cultures of NPCs with MG₍₋₎, however, addition of insulin increased the percentage of GFP⁺/β-III-tubulin⁺ cells **(****Fig. 1B****)** relative to their percentage in such co-cultures without insulin **(****Fig. 1B****)** or in control (microglia-free) cultures in insulin-containing medium **(****Fig. 1B****).** In the presence of insulin, the number of neurons in NPCs co-cultured with MG_{(IFN-γ)} (Fig. 1B) was greater than in NPCs co-cultured with MG₍₋₎ (Fig. 1B), and even greater if the NPCs were co-cultured with MG_{(IFN-γ)} containing neutralizing anti-TNF-α antibodies (ãTNF-α) (Fig. 1B). To verify that the observed beneficial effect of ãTNF-α in the MG_{(IFN-γ)} co-cultures (Fig. 1B) was due to neutralization of the adverse effect of TNF-α on neurogenesis, we added recombinant mouse TNF-α (rTNF-α) to NPCs freshly co-cultured with MG_{(IFN-γ)}. **Fig. 1C** shows that in the presence of rTNF-α the numbers of GFP⁺/β-III-tubulin⁺ cells were similar to those in control (untreated) NPC cultures.

Morphological differences were observed between the newly differentiating neurons in NPCs co-cultured with MG_{(IFN-γ)} and those generated in co-cultures with MG_{(IL-4)} **(****Fig. 1D****).** Co-expression of GFP with β-III-tubulin is shown in **Fig**. **1E**. The newly differentiating neurons were positively labeled for GAD67 (glutamic acid decarboxylase 67), an enzyme responsible for the synthesis of GABA, the major inhibitory transmitter in higher brain regions, and were also found to be colabeled with GFP (β-III-tubulin⁺/GFP⁺/GAD⁺) **(****Fig. 1F**). In another set of experiments we prepared cultures similar to those described above and stained them with doublecortin (DCX; **Fig. 2****),** a marker of early differentiation of the neuronal lineage. This staining revealed a similar effect of the various microglial preparations to that seen with staining for β-III-tubulin. Striking differences in the morphology of newly differentiating neurons were seen between NPCs co-cultured with MG_{(IL-4)} and those co-cultured with MG_{(IFN-γ)} **(****Fig. 2A****);** the former showed significant branching, whereas in the latter the neurons were polarized and had long processes (Fig. 2A). These differences suggested that the mechanisms activated in microglia by the two cytokines are not identical. Co-expression of GFP with DCX is shown in **Fig. 2B****.** In cultures stained for both DCX and β-III-tubulin, these two neuronal markers were found to be co-localized **(****Fig. 2C****).** In all of the above experiments microglial viability, assayed by propidium iodide staining of live cells (Hsieh et al., 2004), was unaffected by the co-culturing conditions. Quantitative analysis of GFP⁺/DCX⁺-stained cells, shown in **Fig. 2D****,** yielded similar results to those obtained when β-III-tubulin was used as the neuronal marker (Figs. 1A, 1B).

### Example 1(2). Effect of microglia on oligodendrogenesis in vitro - Differentiation of NPCs into oligodendrocytes is induced by co-culturing with IL-4 pretreated microglia (MG_{(IL-4)})

Next we examined whether, under the same experimental conditions, microglia would also induce NPCs to differentiate into oligodendrocytes. Under high magnification, we were able to detect newly formed oligodendrocytes. In attempting to detect possible differentiation of NPCs to oligodendrocytes, we first looked for GFP-labeled cells co-expressing oligodendrocyte progenitor marker NG2. Quantitative analysis confirmed that both MG_{(IL-4)} and (to a lesser extent) MG_{(IFN-γ)} induced differentiation of NG2⁺ cells from co-cultured NPCs **(****Figs. 3A, 3B****).** In both MG₍₋₎ and MG_{(IFN-γ)} co-cultured with NPCs, significantly fewer NG2⁺-expressing cells were seen in the absence of insulin **(****Fig. 3A****)** than in its presence **(****Fig. 3B****).** Unlike in the case of neurogenesis **(****Fig. 1****),** MG_{(IFN-γ)}-even in the presence of insulin-was significantly less effective than MG_{(IL-4)} in inducing the appearance of newly differentiating oligodendrocytes (NG2⁺). A significant proportion of the NG2⁺ cells were also labeled for RIP [a monoclonal antibody that specifically labels the cytoplasm of the cell body and processes of premature and mature oligodendrocytes at the pre-ensheathing stage (Hsieh et al., 2004)] in both the MG_{(IFN-γ)} and the MG_{(IL-4)} co-cultures (Figs. 3A, 3B). In the absence of insulin, almost no GFP⁺/NG2⁺ cells were seen in control medium containing no microglia (Fig. 3A). A few GFP⁺/NG2⁺ cells were seen in co-cultures of NPCs with MG₍₋₎ (Fig. 3A), but none in co-cultures with MG_{(LPS)} (Fig. 3A). A dramatic increase in the numbers of these cells was seen in co-cultures with MG_{(IL-4)} **(****Figs. 3A****,** **3C****).**

Addition of insulin to the NPC cultures did not affect the incidence of NG2⁺ cells in the absence of microglia (control; Fig. 3B); it did, however, cause an increase in the numbers of NG2⁺ cells in NPCs co-cultured with MG₍₋₎ (Fig. 3B). Moreover, in the presence of insulin the blocking effect of MG_{(LPS)} on newly differentiating NG2⁺ cells was not altered (Fig. 3B), whereas the numbers of NG2⁺ cells in co-cultures with MG_{(IFN-γ)} were increased (Figs. 3B, 3C). In each of the co-cultures, all NG2⁺ cells were also found to be labeled with GFP **(****Fig. 3D****).** An interesting observation was the close spatial association between the microglia and the newly differentiating oligodendrocytes **(****Fig. 3E****).**

In light of the observed early differences between the effects of MG_{(IL-4)} and MG_{(IFN-γ)} on both neurogenesis and oligodendrogenesis, we examined NPCs co-cultured with the cytokine-activated microglia after 10 days in co-culture. As on day 5, few NG2⁺ cells were seen in the absence of microglia **(****Fig. 4A****).** Quantitative analysis of these cultures disclosed striking differences: while both of the cytokine-activated microglial preparations induced differentiation to both oligodendrocytes (NG2⁺, RIP⁺, GalC⁺) and neurons (β-III-tubulin), MG_{(IL-4)} showed a positive bias towards mature oligodendrocytes and MG_{(IFN-γ)} towards mature neurons. Analysis of the incidence of astrocytes (GFAP⁺ cells) in these cultures (after 10 days of co-culturing) disclosed no significant differences between co-cultures of NPCs with MG_{(IL-4)} and with MG_{(1FN-γ)}; in both, however, GFAP⁺ cells were more numerous than in NPC cultures without microglia **(****Fig. 4A****).** The results suggested that these two types of cytokine-activated microglia affect the three neural cell lineages, and that they have different effects on the neuronal and oligodendrocyte lineages but not on the astrocytic lineage **(****Fig. 4A****).**

In the presence of MG_{(IL-4)}, the GFP⁺/NG2⁺ cells were more branched at 10 days **(****Fig. 4B****)** than at 5 days (Fig. 3C). The branching cells appeared to be forming contacts with cells that looked like neurons (Fig. 4B). Staining of these cultures for galactocerebroside (GalC), a marker of mature oligodendrocytes, and for the neuronal marker β-III-tubulin, verified that the contact-forming cells were newly formed oligodendrocytes and neurons **(****Fig. 4C****).** Analysis of the same cultures for DCX and RIP **(****Fig. 4D****)** revealed that none of the newly differentiating cells expressed both of these markers together. Moreover, there was no overlapping in expression of the astrocyte marker glial fibrillary acid protein (GFAP) and NG2 **(****Fig. 4E****)** or of GFAP and DCX **(****Fig. 4F****).** Analysis of neurite length induced by the cytokine-activated microglia (after 10 days of co-culturing) revealed that neurites of the newly formed neurons in NPCs co-cultured with MG(_{IFN-γ}) were significantly longer than in NPCs co-cultured with MG_{(IL-4)} or in NPCs alone, with no significant differences between neurite lengths in the latter two **(****Fig. 4G****).** Interestingly, there were no significant differences between the absolute numbers of GFP⁺ cells counted in these three groups (NPCs alone: 90.2±32.0; co-cultured with MG_{(IFN-γ)}: 70.5±23.0; co-cultured with MG_{(IL-4)}: 66.1±10.4). This raises a question: do the activated microglia, besides affecting differentiation, also affect NPC proliferation and/or survival?

**Table 1** records the proliferation of NPCs co-cultured with non-activated, IL-4-activated, or IFN-γ-activated microglia. Cultures of untreated NPCs (control) or NPCs co-cultured with MG₍₋₎ or MG_{(IL-4)} or MG_{(IFN-γ)} or MG_{(LPS)}, with or without insulin, were analyzed for proliferation and cell death 24, 48, or 72 h after plating. For the proliferation assay, a pulse of BrdU was applied 12 h before each time point. Numbers of Brd*U⁺ cells are expressed as percentages of GFP⁺ cells (mean ± SEM from three independent experiments in duplicate) and analyzed by ANOVA. Cell death with and without insulin was determined by live staining with 1 µg/ml propidium iodide and 1 µg/ml Hoechst 33342 (mean ± SEM from two independent experiments in duplicate; * *P* < 0.05; ****P* < 0.001; ANOVA).

As shown in Table 1, comparisons of proliferation at 24 h and 48 h of culture revealed no differences. After 72 h a slight but non-significant difference was seen between NPCs alone and NPCs co-cultured with MG₍₋₎ or MG_{(IFN-γ)}, possibly because of decreased proliferation in the culture of NPCs alone rather than any increase in the co-cultures. In the absence of insulin there were no significant differences at any time in culture between NPCs alone and NPCs co-cultured with MG₍₋₎ or with MG_{(IL-4)}. A reduction in proliferation was observed in NPCs co-cultured with MG_{LPS}, with or without insulin. After 5 days, no proliferation was detectable in any of the co-cultures (data not shown). To identify dead or dying cells we stained live cultures with 1 µg/ml propidium iodide, which stains dead cells, and 1 µg/ml Hoechst 33342, which stains both live and dead cells (Hsieh et al., 2004). Significant cell death was observed in NPCs co-cultured with MG_{(LPS)}, both in the absence and in the presence of insulin, whereas in NPCs cultured alone or with MG_{(IFN-Y)} or MG_{(IL-4)} the percentage of cell death was low and did not differ significantly from that seen in cultures of NPCs alone (Table 1). These results suggested that the primary effect of the cytokine-activated microglia on the fate of NPCs in vitro occurs via a mechanism that is instructive rather than selective.

**Table 1. Proliferation and survival of neural progenitor cells (NPCs) in co-cultures with microglia**

| % BrdU⁺ cells out of GFP⁺ cells | | | | % PI⁺ cells out of GFP⁺ cells | | | |
|---|---|---|---|---|---|---|---|
| Treatment | +Insulin | | | Treatment | +Insulin | | |
| | 24h | 48h | 72h | | 24h | 48h | 72h |
| Control | 5.8±0.6 | 5.4±1.5 | 1.4±0.9 | Control | 3.3±0.9 | 1.7±0.4 | 1.7±0.7 |
| MG₍₋₎ | 6.2±0.5 | 6.9±1.9 | 5.6±1.4 | MG₍₋₎ | 4.0±0.5 | 2.8±0.7 | 2.8±0.9 |
| MG_{(IFN-γ)} | 5.8±1.5 | 7.1±2.5 | 5.6±1.1 | MG_{(IFN-γ)} | 5.9±0.6 | 4.2±1.5 | 3.6±2.1 |
| MG_{(LPS)} | 3.1±0.8 | 1.7±0.4 | 1.1±0.1 | MG_{(LPS)} | 14.0±0.1 *** | 7.3±1.9 | 4.1±0.2 |

| Treatment | -Insulin | | | Treatment | -Insulin | | |
|---|---|---|---|---|---|---|---|
| | 24h | 48h | 72h | | 24h | 48h | 72h |
| Control | 4.1±0.6 | 3.2±0.5 | 1.2±0.7 | Control | 3.7±0.2 | 2.3±0.8 | 2.2±1.1. |
| MG₍₋₎ | 6.5±1.5 | 3.5±0.7 | 3.3±2.5 | MG₍₋₎ | 5.2±3.0 | 3.7±0.3 | 3.0±1.0 |
| MG_{(IFN-4)} | 6.2±1.1 | 5.4±1.9 | 3.3±2.2 | MG_{(IFN-4)} | 3.7±2.0 | 2.9±0.4 | 2.5±0.3 |
| MG_{(LPS)} | 2.1±0.2 | 1.7±0.5 | 1.1±0.6 | MG_{(LPS)} | 15.8±1.9* | 7.0±5.0 | 4.8±0.8 |

Proliferation and survival of neural progenitor cells (NPCs) in co-cultures with microglia. Cultures of untreated NPCs (control) or NPCs co-cultured with MG₍₋₎ or MG_{(IL-4)} or MG_{(IFN-γ)} or MG_{(LPS)}, with or without insulin, were analyzed for proliferation and cell death 24, 48, or 72 h after plating. For the proliferation assay a pulse of BrdU was applied 12 h before each time point. Numbers of BrdU⁺ cells are expressed as percentages of GFP⁺ cells (mean ±SEM from three independent experiments in duplicate) and analyzed by ANOVA. Cell death with and without insulin was determined by live staining with 1 µg/ml propidium iodide and 1 µg/ml Hoechst 33342 (mean ±SEM from two independent experiments in duplicate; ** P <* 0.05; *** *P* < 0.001; ANOVA).

### Example 1(3). Possible mechanism of oligodendrogenesis induction by IL-4-and IFN-γ-activated microglia

Insulin-like growth factor (IGF)-I is reportedly a key factor in neurogenesis and oligodendrogenesis (Carson et al., 1993; Aberg et al., 2000; O'Kusky et al., 2000; Hsieh et al., 2004). To determine whether the beneficial effect of the cytokine-activated microglia on the differentiation of NPCs is mediated, at least in part, by the ability of the microglia to produce IGF-I, we added neutralizing antibodies specific to IGF-I (ãIGF-I) to the NPCs co-cultured with activated microglia. ãIGF-I blocked the MG_{(IL-4)}-induced effect on oligodendrogenesis **(****Fig.** 5A), indicating that the effect of IL-4-activated microglia on oligodendrogenesis is dependent on IGF-I. Direct addition of recombinant IGF-I (rIGF-I; 500 ng/ml) to NPCs resulted in their significant differentiation to NG2-expressing cells **(****Fig. 5B****).** Such differentiation, however, was less extensive than that observed in NPCs co-cultured with MG_{(IL-4)} (Fig. 5A), suggesting that the MG_{(IL-4)} effect is mediated through additional (possibly soluble) factors, or by cell-cell interaction, or both. ãIGF-I had no effect on oligodendrogenesis induced by MG_{(IFN-γ)} (data not shown). We also examined the effect of ãIGF-I on MG_{(IL-4)}-induced neurogenesis by assessing β-III-tubulin expression. The percentage of GFP⁺/β-III-tubulin⁺ cells was 21.9 ± 2.9% in NPCs co-cultured with MG_{(IL-4)} and 19.7 ± 4.5%, (P = 0.3) when ãIGF-I was added to those co-cultures. These results suggested that MG_{(IL-4)} produces additional potent neurogenic factors besides IGF-I.

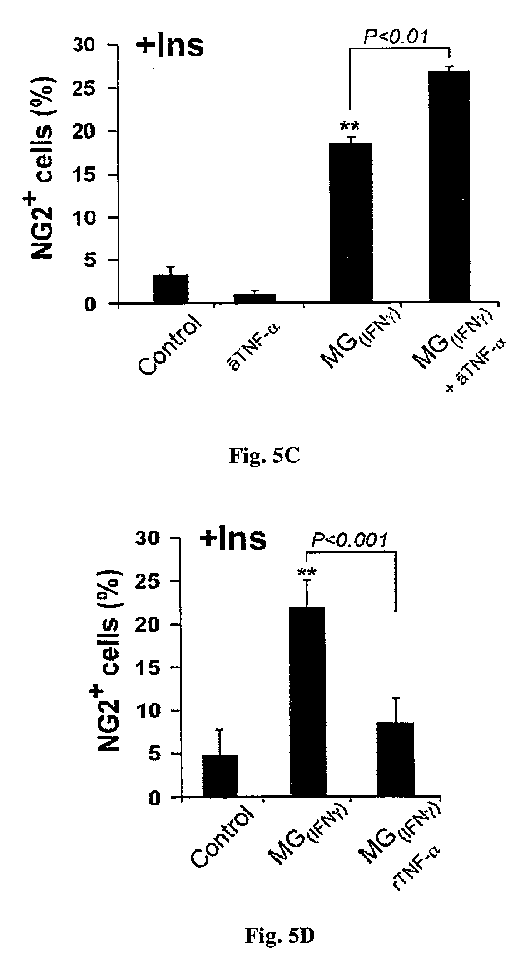

In light of the observed beneficial effect of ãTNF-α on the outcome of MG_{(IFN-γ)}-induced neurogenesis **(****Fig. 1****),** we examined whether neutralization of TNF-α, would promote MG_{(IFN-γ)}-induced oligodendrogenesis as well. Oligodendrogenesis was indeed enhanced by ãTNF-α in NPCs co-cultured with MG_{(IFN-γ)} **(****Fig. 5C****).** The implied negative effect of TNF-α was substantiated by direct addition of TNF-α to NPCs co-cultured with MG_{(IFN-γ)} **(****Fig. 5D****).**

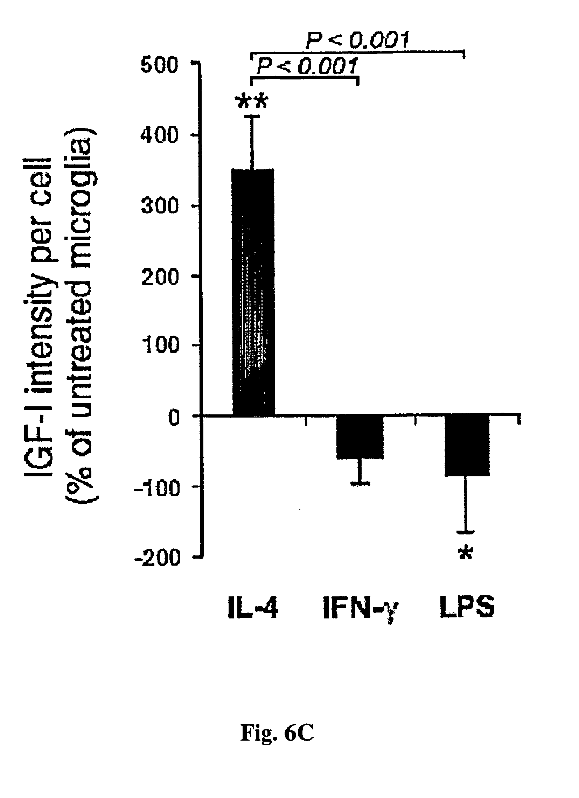

Comparative RT-PCR analyses of microglial mRNA disclosed that in the absence of activation the microglia produced both IGF-I and low levels of TNF-α. Analysis of TNF-α and IGF-I production as a function of time revealed that IFN-γ, unlike IL-4, caused a transient increase in TNF-α production and down-regulation of IGF-I **(****Figs. 6A, 6B****).** At the protein level, quantitative immunocytochemical analysis also disclosed up-regulation of the expression of IGF-I by MG_{(IL-4)}. LPS completely blocked the production of IGF-I **(****Fig. 6C****).**

### Discussion

The results of this study strongly suggest that certain specifically activated microglia can induce neural cell renewal in the adult CNS. The findings showed that microglia can determine the fate of differentiating adult NPCs. Both neurogenesis and oligodendrogenesis were induced in NPCs co-cultured with MG_{(1L-4)},and MG_{(IFN-γ)}, whereas both were blocked by MG_{(LPS)}, in line with reports that inflammation associated with LPS blocks adult neurogenesis (Ekdahl et al., 2003; Monje et al., 2003). NPCs co-cultured with MG_{(IL-4)} showed a bias towards oligodendrogenesis, whereas NPCs co-cultured with MG_{(IFN-γ)} were biased towards neurogenesis.

### EXAMPLE 2

### Synergy between T cells and adult neural progenitor cells promotes functional recovery from spinal cord injury

Recovery from spinal cord injury evidently necessitates a local immune response that is amenable to well-controlled boosting by immunization with T lymphocytes recognizing myelin-associated antigens at the injury site. The relevant T cells can activate local microglia to express a phenotype supportive of neuronal survival and renewal. We show that recovery of mice from spinal contusion is synergistically promoted by T-cell-based vaccination with a myelin-derived peptide and injection of adult neural stem/progenitor cells (aNPCs) into the cerebrospinal fluid. Significantly more aNPCs targeted the lesion site in vaccinated than in nonvaccinated mice. Synergistic interaction between aNPCs and T cells *in vitro* was critically dependent on T-cell specificity and phenotype. The results suggest that controlled immune activity underlies efficient regulation of the stem-cell niche, and that stem-cell therapy necessitates autologous or histocompatibility-matched donors instead of the immunosuppressive anti-rejection drugs that would eliminate any beneficial effect of immune cells on spinal cord repair.

### Materials and Methods

***(x) Animals.*** Inbred adult wild-type C57B1/6J mice were supplied by the Animal Breeding Center of The Weizmann Institute of Science. All animals were handled according to the regulations formulated by the Institutional Animal Care and Use Committee (IACUC).
**(xi) *Antigens.*** The following peptides were synthesized by the Synthesis Unit at the Weizmann Institute (Rehovot, Israel): MOG, residues 35-55 MEVGWYRSPFSRVVHLYRNGK (SEQ ID NO:39) and an altered MOG peptide (45D) MEVGWYRSPFDRVVHLYRNGK (SEQ ID NO:40), a peptide analog of MOG 35-55 containing a serine to aspartic acid substitution as shown. OVA was purchased from Sigma.
**(xii) *Immunization***. Adult mice were immunized with MOG, 45D, or OVA (all 100 µg), each emulsified in an equal volume of CFA (Difco, Detroit, MI) containing *Mycobacterium tuberculosis* (5 mg/ml; Difco), or IFA. The emulsion (total volume 0.15 ml) was injected s.c. at one site in the flank. Control mice were injected with PBS.
(xiii) ***Spinal cord injury.*** Mice were anesthetized, their spinal cords were exposed by laminectomy at T12, and a force of 200 kdyn was placed for 1 s on the laminectomized cord using the Infinite Horizon spinal cord impactor (Precision Systems and Instrumentation, Lexington, KY), a device shown to inflict a well-calibrated contusive injury of the spinal cord.
**(xiv) *Assessment of functional recovery from spinal cord contusion.*** Functional recovery from spinal cord contusion in mice was determined by hindlimb locomotor performance. Recovery was scored by the Basso Mouse Scale (BMS) open-field locomotor rating scale, a scale recently developed specifically for mice, with scores ranging from 0 (complete paralysis) to 9 (normal mobility) (Engesser-Cesar et al., 2005). Blind scoring ensured that observers were not aware of the treatment received by each mouse. Twice a week locomotor activities of mice in an open field were monitored by placing the mouse for 4 min in the center of a circular enclosure (90 cm in diameter, 7 cm wall height) made of molded plastic with a smooth, non-slip floor. Before each evaluation the mice were examined carefully for perineal infection, wounds in the hindlimbs, and tail and foot autophagia.
**(xv) *Stereotaxic injection of neural progenitor cells.*** Mice were anesthetized and placed in a stereotactic device. The skull was exposed and kept dry and clean. The bregma was identified and marked. The designated point of injection was at a depth of 2 mm from the brain surface, 0.4 mm behind the bregma in the anteroposterior axis, and 1.0 mm lateral to the midline. Neural progenitor cells were applied with a Hamilton syringe (5 × 10⁵ cells in 3 µl, at a rate of 1 µl/min) and the skin over the wound was sutured.
**(xvi) *Neural progenitor cell culture.*** Cultures of adult neural progenitor cells (aNPCs) were obtained as previously described in Example 1.
**(xvii) *Co-culturing of neural progenitor cells and T cells.*** CD4+ T cells were purified from lymph nodes of 8-week-old C57B16/J mice as previously described (Kipnis et al., 2004). T cells were activated in RPMI medium supplemented with L-glutamine (2 mM), 2-mercaptoethanol (5 × 10⁻⁵ M), sodium pyruvate (1 mM), penicillin (100 IU/ml), streptomycin (100 µg/ml), nonessential amino acids (1 ml/100 ml), and autologous serum 2% (v/v) in the presence of mouse recombinant IL-2 (mrIL-2; 5 ng/ml) and soluble anti-CD28 and anti-CD3 antibodies (1 ng/ml). T cells were co-cultured (5 × 10⁴ cells/well) with aNPCs (5 × 10⁴ cells/well) for 5 d on cover slips coated with Matrigel (BD Biosciences) in 24-well plates. The cultures were then fixed with 2.5% paraformaldehyde in PBS for 30 min at room temperature and stained for neuronal markers.
**(xviii) *Immunohistochemistry.*** Mice subjected to SCI were re-anesthetized 14 or 60 days later and perfused with cold PBS. Their spinal cords were removed, postfixed with Bouin's fixative (75% saturated picric acid, 25% formaldehyde, 5% glacial acetic acid; Sigma-Aldrich) for 48 h, and then transferred to 70% EtOH. The tissues were hydrated through a gradient of 70%, 95%, and 100% EtOH in xylene and paraffin, and were then embedded in paraffin. For each stain, five tissue sections, each 6 µm thick, were taken from each mouse. The paraffin was removed by successive rinsing of slides for 15 min with each of the following: xylene, EtOH 100%, 95%, 70%, 50%, and PBS. Exposure of the slides to antigen was maximized by heating them to boiling point in 10 mM sodium citrate pH 6.0 in a microwave oven, then heating them at 20% microwave power for a further 10 min. The slides were blocked with 20% normal horse serum for 60 min prior to overnight incubation at room temperature (GFAP, neurofilaments, and BDNF), or for 48 h at 4°C (CD3), with the monoclonal antibody in 2% horse serum. We used rabbit anti-mouse GFAP (1:200) (DakoCytomation, Glostrup, Denmark) for GFAP; rabbit anti-neurofilament (1:200), low and high molecular weight (Serotec, Oxford, UK) for neurofilaments; and rat anti-human CD3 (1:50) (Serotec) for CD3. For BDNF we used the monoclonal antibody chicken anti-human BDNF (1:100) (Promega, Madison, WI) with 0.05% saponin.

After rinsing, sections were incubated for 1 h at room temperature with the secondary antibody Cy3 donkey anti-rat (1:300) (Jackson ImmunoResearch Laboratories, West Grove, PA) (staining for CD3), Cy3 donkey anti-chicken (Jackson ImmunoResearch) (1:250) (staining for BDNF), or Cy3 donkey anti-rabbit (Jackson ImmunoResearch) (1:250) (staining for GFAP and neurofilaments). For IB4 staining, sections were blocked for 1 h with 20% horse serum and then incubated for 1 h at room temperature with Cy2-IB4 (1:50) (Sigma-Aldrich). All sections were stained with Hoechst (1:2000) (Molecular Probes-Invitrogen, Carlsbad, CA). They were then prepared for examination under a Nicon E-600 fluorescence light microscope. Results were analyzed by counting the cells (CD3-labeled) in the site of injury, or by determination of the density (IB4-labeled or BDNF-labeled), or by measurement of the unstained area (GFAP, neurofilaments). Each of the parameters was measured by an observer who was blinded to the treatment received by the mice.

### Example 2(1). Adult neural progenitor cells require local immune activity to promote motor recovery

Our working hypothesis in this study was that the protective immune response evoked at a site of injury by T-cell based immunization creates a niche that supports not only cell survival but also tissue repair. We further suspected that a local T-cell mediated immune response could attract exogenously delivered aNPCs and support their contribution to recovery. To test this hypothesis we vaccinated C57B1/6J mice, immediately after SCI, with the encephalitogenic peptide pMOG 35-55 (SEQ ID NO: 39) emulsified in CFA containing 0.5% *Mycobacterium tuberculosis* (MOG/CFA), and 1 week later administered aNPCs via the intracerebroventricular (i.c.v.) route. Mice subjected to this dual treatment protocol (MOG/CFA/aNPC) were compared to a control group of mice that were immunized with the same MOG peptide emulsified in the same adjuvant but were not transplanted with aNPCs and instead were injected i.c.v. with PBS (MOG/CFA/PBS), or to mice that were injected with PBS and CFA (0.5%) and transplanted i.c.v. with aNPCs (PBS/CFA/aNPC) or a control group of mice that were injected with PBS/CFA and then injected i.c.v. with PBS (PBS/CFA/PBS). To assess behavioral outcome after SCI we used the Basso motor score (BMS) rating scale (Engesser-Cesar et al., 2005), in which 0 indicates complete paralysis of the hindlimbs and 9 denotes full mobility. The mean motor recovery (BMS) scores of mice receiving the MOG/CFA/aNPC (4.21 ± 0.45; all values are mean ± SEM) were higher than those of mice treated with MOG/CF A/PBS. In mice treated with PBS/CFA/aNPC, recovery was not better than in control mice treated with PBS/CF A/PBS (1.5 ± 0.27). A BMS of 4.21 indicates extensive movement of the ankle and plantar placement of the paw (three animals showed, in addition, occasional weight support and plantar steps), whereas a score of 1.5 indicates ankle movement ranging from slight to extensive. Mice treated with MOG/CFA/PBS scored 2.71 ± 0.5, a, significantly higher score than that of control PBS/CFA/aNPC-treated mice (1.5 ± 0.4) or of control mice treated with PBS/CFA/PBS **(****Fig. 7A****).** These results thus demonstrated synergistic interaction between the administered aNPCs and the T cell-based immune response. Failure of the transplanted aNPCs to improve motor recovery by themselves (i.e., in the absence of MOG/CFA immunization) suggested that a site-specific immune response was necessary for aNPC activity. **Fig. 7B** shows the BMS of individual mice in all examined groups on day 28 postinjury. Because transplantation of aNPCs in the absence of immunization did not improve recovery from SCI, this control group (PBS/CFA/aNPC) was not included in subsequent experiments.

We have previously demonstrated that boosting of the amounts of T cells needed for promoting recovery from SCI does not necessitate the use of encephalitogenic peptides; weak agonists of encephalitogenic peptides are just as effective and do not carry the risk of inducing EAE. To test whether such 'safe' vaccination could be utilized in combination with aNPCs transplantation we used a MOG-derived altered peptide ligand (pMOG 35-55 APL; 45D peptide, (SEQ ID NO:40), in which aspartic acid is substituted for serine. Mice were vaccinated with the 45D peptide emulsified in CFA containing 2.5% *Mycobacterium tuberculosis.* One week later the immunized mice were subjected to contusive SCI, and after another week were transplanted i.c.v. with aNPCs. Increased motor activity (as expressed by the BMS, mean ± SEM) was seen in these mice than in control mice treated i.c.v with PBS/CFA/aNPC (4.11 ± 0.27 compared to 1.94 ± 0.22; **Fig. 7C****).** Without aNPC transplantation, immunization with peptide 45D in CFA resulted in only a slight increase in motor recovery relative to the PBS-treated control (2.57 ± 0.24). **Fig. 7D** shows BMS values for individual mice on day 28 postinjury. The above findings showed that the contribution of transplanted aNPCs to motor recovery after contusive SCI could also be promoted by the use of a weak agonist of the encephalitogenic peptide.

To determine the phenotype and specificity of the T cells needed for synergistic interaction with aNPCs we repeated the above experiments using different immunization protocols. Incomplete Freund's adjuvant (IFA), unlike CFA, is free of bacteria and is known to elicit a Th2-like response to encephalitogenic peptides. We found that although immunization with the MOG analog (peptide 45D) emulsified in IFA had some beneficial effect, it showed no synergy with subsequent transplantation of aNPCs (BMS of 3.2 ± 0.76 for MOG/IFA/aNPC-treated mice compared to 2.93 ± 1.03 for MOG/IFA/PBS-treated mice and 2.07 ± 0.53 in the PBS/CFA/PBS-treated mice; **Fig. 7E****).** These findings suggest that for MOG/IFA/aNPC-treated mice, the preferred T cells for synergistic interaction at the injury site between vaccinated T cells and aNPCs are Th1.

To verify that specificity to CNS-antigens is required for a synergistic effect of vaccination and stem-cell transplantation, mice were immunized with the nonself protein ovalbumin (OVA) emulsified in CFA (containing 2.5% *Mycobacterium tuberculosis),* and 1 week later were injected i.c.v. with aNPCs or with PBS as control. Immunization with OVA/CFA resulted in a slight, nonsignificant increase in BMS, and implantation of aNPCs did not increase the BMS any further (2.43 ± 1.78 for the OVA/CFA/aNPC-treated mice compared to 2.2 ± 0.68 for OVA/CFA/PBS-treated mice and 1.5 ± 0.29 for PBS/CFA/PBS-treated control group, **Fig. 7F****).** Taking all of the above results together, the absence of a beneficial effect after aNPC transplantation suggests that synergy between T cells and aNPCs is a function of both the antigenic specificity and the phenotype of the T cells.

Immune activation in the injured CNS, and specifically in the spinal cord, has been a major focus of research attention in recent years (Schwartz and Hauben, 2002). In some of the studies, T cell-based immune responses were shown to be protective only if their intensity and duration were well regulated. Overly strong immunization yielded excessive immune activity, which neutralized the potential benefit of the immune response for the injured spinal cord and even had a detrimental effect. We considered the possibility that if aNPCs home to the site of damage they can offset the negative effect of excessive immune activity and thus contribute to recovery. We therefore set out to determine whether administration of aNPCs can contribute to functional recovery even when the local immune activity is excessive. One week prior to SCI we immunized mice with MOG peptide 35-55 emulsified in CFA containing 2.5% *Mycobacterium tuberculosis.* Under conditions in which motor recovery from SCI was worse after immunization with MOG/CFA than after injection with PBS/CFA (BMS of 0.35 ± 0.2 and 1.94 ± 0.32, respectively), we found that recovery was improved upon administration of aNPCs (BMS of 2.68 ± 0.51; **Figs. 7G, 7H****).** It thus appears that i.c.v. administration of aNPCs can contribute to functional recovery from SCI even when the local adaptive immune response is detrimental.

### Example 2(2). Tissue integrity correlates with local immune activity

In an attempt to gain an insight into the mechanism underlying the apparent synergy between aNPCs and resident immune cells, we examined whether any of the injected aNPCs find their way to the injured spinal cord. We repeated the experiment showed on Fig. 7A using GFP-labeled aNPCs. Staining with anti-GFP antibodies revealed GFP⁺ cells in the parenchyma of the injured spinal cord only in mice that were treated with MOG-CFA/aNPC **(****Fig. 8****).** Injected GFP⁺ aNPCs could be seen surrounding the epicenter of the lesion and laterally in the spinal cord parenchyma adjacent to the meninges as early as 7 days after SCI **(****Figs. 8A-8C****),** and could still be detected as late as 60 days after the injury, the last time point examined **(****Figs. 8D-8F****).**

One of the morphological features that characterize recovery from SCI is the size of the lesion. To delineate the site of injury we stained longitudinal sections of the spinal cord with antibodies to glial fibrillary acidic protein (GFAP). We assessed the lesion size by measuring the areas that were not stained by GFAP. This analysis disclosed that as early as 7 days after aNPCs were transplanted in the MOG/CFA-vaccinated rats, the averaged size of the site of injury was significantly smaller in mice that had received both vaccination and aNPC transplantation than in mice that had only been vaccinated or had received only aNPCs **(****Figs. 9A, 9B****).**

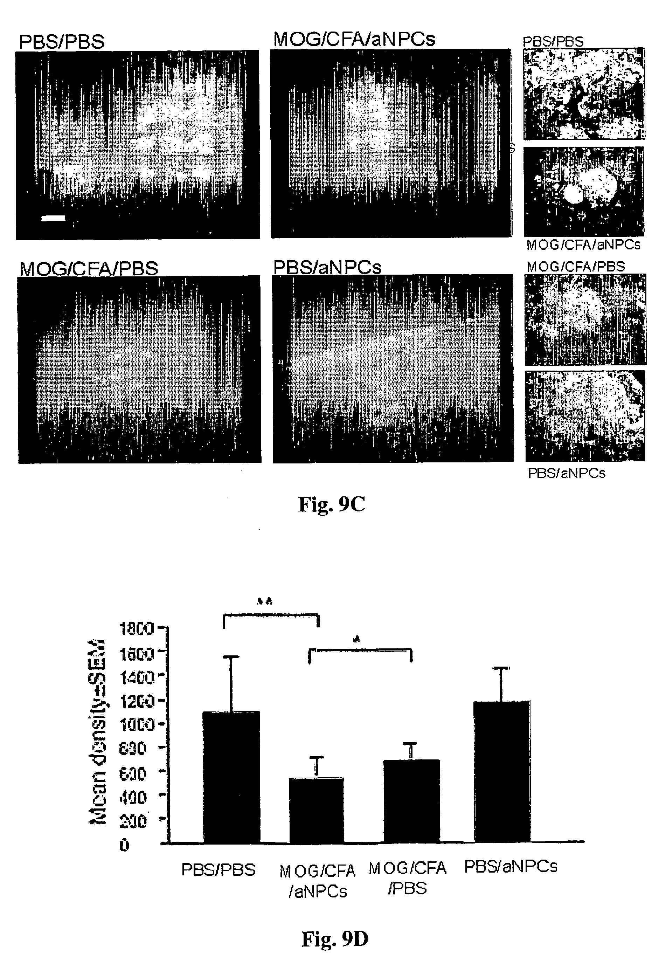

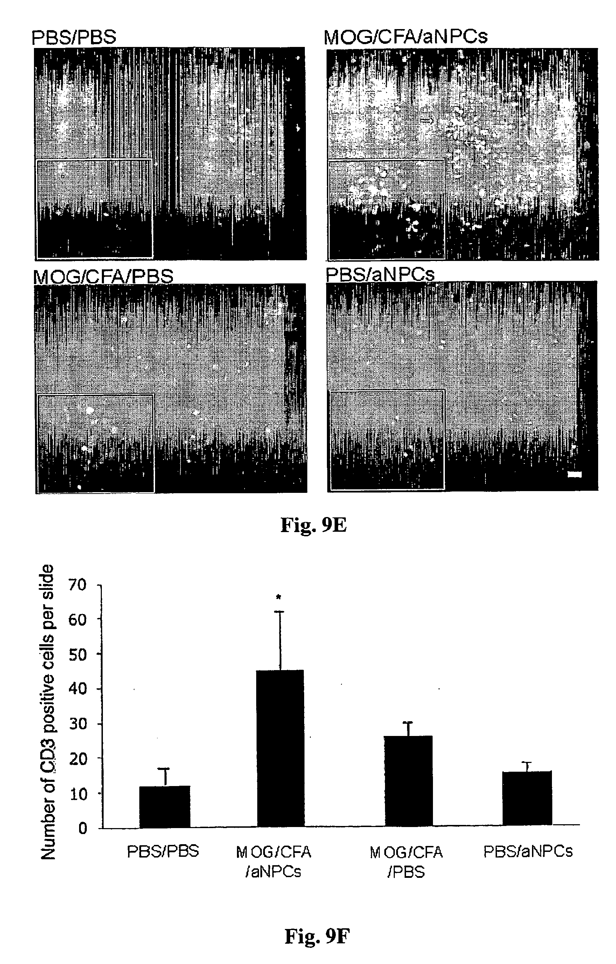

Next we examined whether the observed differences in the extent of recovery could be correlated with local immunological changes. Sections of spinal cord tissue were stained for markers of T cells (CD3) and accumulation of activated microglia/macrophages (IB4) **(****Figs. 9C-9F****).** All sections were also stained with Hoechst as a nuclear marker. Tissues were excised 7 days after cell transplantation. Staining with IB4 revealed fewer microglia/macrophages in mice that had received the dual treatment protocol (pMOG 35-55 in 0.5% CFA) than in the other experimental groups **(****Figs. 9C, 9D****).** Quantitative analysis revealed significantly more CD3⁺ T cells in areas surrounding the site of injury in mice that had received the dual treatment than in MOG/CFA/PBS-treated or in PBS/CFA/PBS-treated controls. Notably, immunization with the encephalitogenic pMOG 35-55 without aNPC transplantation resulted in only slightly more CD3⁺ cells at the injured site than in the controls **(****Figs. 9E, 9F****).** It thus seems that transplantation of aNPCs modulated the local immune response at the injured site.

Both activated T cells and T cell-activated microglia can serve as sources of growth factors such as brain-derived neurotrophic factor (BDNF). BDNF immunoreactivity was more intense in the spinal cords of mice treated with MOG/CFA/aNPC than in the other groups **(****Fig. 10A****).** Double staining for BDNF and IB4 showed that the cellular source of BDNF in the injured site was the microglial/macrophage population **(****Fig. 10B**).

Recent studies have shown that noggin, a bone morphogenesis protein (BMP) inhibitor, can induce neuronal differentiation from aNPCs in the injured spinal cord (Setoguchi et al., 2004). This protein was also shown to be needed to provide a neurogenic environment in the subventricular zone. We therefore assayed noggin immunoreactivity in the various experimental groups, and found that it was significantly increased in mice that received the dual treatment protocol, but was unaffected by MOG immunization alone and was slightly decreased by aNPC transplantation alone **(****Fig. 10C****).** As in the case of BDNF produced in the injured site, noggin was also localized to IB4+ cells **(****Fig. 10D****).**

### Example 2(3). Local differentiation of endogenous stem cells

The above results raised an important question: can a T cell-based vaccination, when given in combination with aNPC transplantation, create conditions favorable for neuronal differentiation of endogenous or exogenous aNPCs? To examine this possibility, we repeated the experiment described in Fig. 7, while also injecting the cell-proliferation marker BrdU twice daily for 3 days, starting on day 7 after aNPC transplantation (i.e., 14 days after SCI). Staining for BrdU and the early differentiation marker doublecortin (DCX) 7 days after the last BrdU injection disclosed significantly more newly formed neurons in the mice that had received the dual treatment **(****Figs. 11A-11E****).** **Fig. 11B** demonstrates the distribution of DCX+ cells in the environment/vicinity of the injured site. Staining for the combination of BrdU and GFP and of GFP and DCX revealed virtually no double-positive cells, indicating that most of the DCX+ cells in the injured spinal cord had originated from endogenous aNPCs. Notably, vaccination without aNPC transplantation did not increase the formation of new neurons in the injured spinal cord.

### Example 2(4). Interaction of adult neural progenitor cells and T cells in vitro

The above results indicated that cross-talk between immune cells and aNPCs was taking place at the site of injury. We showed hereinabove that microglia preactivated with the Th1- and Th2-associated cytokines, IFN-γ and IL-4, respectively, can induce neuronal differentiation from aNPCs. We therefore sought to determine whether direct interaction between aNPCs and T cells would also result in an altered pattern of aNPC differentiation. To address this question, we activated CD4+ T cells *in vitro* by a cognate protocol (with anti-CD3 antibodies, anti-CD28 antibodies, and IL-2) for 24 h and then allowed their activation to continue in co-cultures with aNPCs in a transwell culture system. As controls we used cultures of aNPCs alone (in the presence of anti-CD3 antibodies, anti-CD28 antibodies and IL-2) or aNPCs cultured with CD4+ T cells in a resting state (supplemented with IL-2 only). After 5 days in culture the aNPCs in the lower chamber were fixed and analyzed for the appearance of newly formed neurons. Staining for the early neuronal marker β-III-tubulin revealed a dramatic effect of T cells on neuronal differentiation **(****Fig. 12A****).** Compared to control cultures of aNPCs alone, in which only about 7% of the cells were positive for β-III-tubulin, approximately 90% of the cells expressed β-III-tubulin in co-cultures of aNPCs with activated T cells **(****Figs. 12A, 12B****).** Notably, β-III-tubulin staining showed a 3-fold increase (18%) relative to the control in co-cultures of aNPCs with nonactivated T cells. It thus seems that T cells induce neuronal differentiation via a soluble factor. **Fig. 12B** shows representative images of β-III tubulin-stained cultures of aNPCs alone (control) or of co-cultures with activated T cells. These findings imply that T cell-derived soluble factors might trigger one of the pathways of neuronal differentiation.

To exclude the possibility that the T cells had affected NPC differentiation as a consequence of encountering NPC-derived compounds, and to further substantiate our finding that the observed effect was caused by T cell-derived soluble substances, we allowed aNPCs to differentiate in the presence of medium conditioned by activated T cells. After 5 days, staining of these cultures for β-III-tubulin revealed similar results to those obtained in the co-culture system **(****Fig 12C****),** confirming that neuronal differentiation was induced by resting T cells and even more by the activated T cells. In addition to their effect on the numbers of differentiating cells, the T cell-derived soluble factors also evidently affected the cellular morphology, as manifested in the branched, elongating β-III tubulin-labeled fibers **(****Fig. 12D****).**

We next sought to determine whether the T cell-induced neurogenesis was mediated by cytokines secreted by activated T cells. aNPCs were cultured in the presence of different concentrations of the characteristic T-cell derived cytokines IFN-γ and IL-4. Analysis revealed that IFN-γ, at concentrations as low as 1 ng/ml, could induce an increase in β-III-tubulin expression after 5 days in culture **(****Fig 12E****).** In experiments described herein, we found that brief exposure to IFN-γ (24 h) was not sufficient to attain such an effect. It should be noted, however, that the morphology of the β-III-tubulin-expressing cells in IFN-γ-supplemented cultures was less developed than that seen in aNPCs cultured with activated T cells or in T cell-conditioned medium. In contrast to the effect of IFN-γ, no change in β-III-tubulin expression was observed in aNPCs treated with IL-4.

These findings suggested that IFN-γ, unlike IL-4, could account in part for the T cell-induced neurogenesis. Even the effect of IFN-γ, however, was limited relative to that of the T cells or to the T cell-derived soluble factors. PCR analysis of expression of the IFN-γ receptor-1 on aNPCs disclosed that this receptor is expressed by aNPCs under all of the conditions examined here (data not shown).

Activation of the Notch pathway is essential for maintenance of aNPCs, and blockage of this pathway and its downstream transcription factors of the Hes gene family underlie the first events in neuronal differentiation. To determine whether T cell-mediated neuronal differentiation induces changes in Notch signaling, we looked for possible changes in expression of Hes genes in aNPCs following their interaction with T cell-derived substances. Real-time PCR disclosed that relative to control cultures, aNPCs cultured for 24 h in the presence of T cell-conditioned medium underwent a five-fold decrease in Hes-5 expression **(****Fig. 12F****).** Thus, differentiation induced by T cells appears to involve inhibition of the Notch pathway. Expression of Notch 1-4 by aNPCs was not altered in the presence of T cell-conditioned medium, indicating that the inhibition could not be attributed to changes in Notch expression (data not shown).

Our observation that aNPCs express an IFN-γ receptor, taken together with recent studies showing that these cells express immune-related molecules such as B-7 (Imitola et al., 2004b) and CD44 (Pluchino et al., 2003), known to participate in the dialog between T cells and antigen-presenting cells (APCs), prompted us to examine whether aNPCs could affect T-cell function. First we examined the effects of aNPCs on proliferation of CD4+ T cells by assaying [³H]thymidine incorporation by the T cells. Co-culturing of T cells with aNPCs and APCs (lethally irradiated splenocytes) for 3 days resulted in a significant dose-dependent inhibition of T-cell proliferation **(****Fig. 13A****).** It is important to note that under these conditions there was only limited proliferation of aNPCs. To determine whether the inhibitory effect on T-cell proliferation is mediated by a soluble factor or requires cell-cell contact, we utilized the transwell system, plating aNPCs in the upper well. Co-culturing of T cells and aNPCs in the same well resulted in a two-fold reduction in T-cell proliferation, but this effect was diminished when the two cell populations were separated in the transwell. It thus seems that cell-cell contact is necessary for aNPCs to inhibit T-cell proliferation. To determine whether aNPCs could affect the production of cytokines by T cells, we measured the concentrations of six inflammatory cytokines in the media of co-cultured aNPCs and T cells. The concentrations of IL-12, IFN-γ, and TNF-α were similar in T-cell cultures with and without aNPCs, but the concentration of IL-10 was slightly increased (by 40%) in the co-culture. Relative to T-cell cultures alone, the concentration of IL-6 was twofold higher in the co-culture with aNPCs, and a remarkable difference was seen in MCP-1 concentration, which was higher by two orders of magnitude in the co-culture **(****Fig. 13C****).** Taken together, these results indicate that aNPCs can act directly on T cells, inhibiting their proliferative activity and changing their cytokine/ chemokine production profile.

### Discussion

Local interaction between immune cells and aNPCs underlies functional recovery. In the present study we combined two different therapeutic approaches for SCI: T cell-based vaccination and transplantation of neural progenitor cells into the CSF. Each of these approaches has been shown to be potentially capable of promoting functional recovery from SCI; we show here that when combined, they operate in synergy. Our experiments, both *in vivo* and *in vitro,* demonstrated tthat cross talk between immune cells and aNPCs can take place at the lesion site. The vaccination elicits a local immune response, which, if well controlled, provides the cellular and molecular elements needed to attenuate degeneration and promote repair. The same response also plays a role in recruiting aNPCs to the injured site and creating niche-like compartments that support neurogenesis from endogenous aNPCs. The interaction between aNPCs and immune cells was found to be reciprocal: aNPCs could modulate the postinjury immune activity, ensuring functional recovery even under conditions of excessive immune activity (which, in the absence of aNPCs, have a detrimental effect on recovery).

### EXAMPLE 3

### T-cell Based Vaccination Restores Cognition, Removes Plaques, and Induces Neurogenesis in a Mouse Model of Alzheimer's Disease

Accumulation of β-amyloid deposition (Aβ), neuronal loss, cognitive decline, and microglial activation, are characteristic features of Alzheimer's disease (AD). Using AD double-transgenic mice expressing mutant human genes encoding presenilin 1 and chimeric mouse/human amyloid precursor protein, we show that vaccination with glatiramer acetate prevented and restored cognitive decline, assessed by performance in a Morris water maze (MWM). The vaccination modulated microglial activation, eliminated plaque formation, and induced neuronal survival and neurogenesis. In vitro, Aβ-activated microglia impeded neurogenesis from adult neural stem/progenitor cells. This was counteracted by IL-4, and more so when IFN-γ was added, but not by IFN-γ alone.

### Materials and Methods

***(xix) Animals.*** Nineteen adult double-transgenic APP_{K670N, M671L} + PS1_{ΔE9} mice of the B6C3-Tg (APPswe, PSEN1dE9) 85Dbo/J strain were purchased from The Jackson Laboratory (Bar Harbor, ME) and were bred and maintained in the Animal Breeding Center of The Weizmann Institute of Science. All animals were handled according to the regulations formulated by the Weizmann Institute's Animal Care and Use Committee. Tg AD mice were produced by co-injection of chimeric mouse/human APPswe (APP695 [humanized Aβ domain] harboring the Swedish [K594M/N595L] mutation) and human PS1dE9 (deletion of exon 9) vectors controlled by independent mouse prion protein promoter (MoPrP) elements, as described (Borchelt et al., 1997).
***(xx) Reagents.*** Recombinant mouse IFN-γ and IL-4 (both containing endotoxin at a concentration below 0.1 ng/µg cytokine) were obtained from R&D Systems (Minneapolis, MN). β-amyloid peptides [amyloid protein fragment 1-40 and 1-42 (Aβ_{1-40/1-42})] were purchased from Sigma-Aldrich, St. Louis, MO. The Aβ peptides were dissolved in endotoxin-free water, and Aβ aggregates were formed by incubation of Aβ, as described (Ishii et al., 2000).
**(xxi) *Genotyping.*** All mice used in this experiment were genotyped for the presence of the transgenes by PCR amplification of genomic DNA extracted from 1-cm tail clippings (Jankowsky et al., 2004). Reactions contained four primers: one anti-sense primer-matching sequence within the vector that is also present in mouse genomic PrP (5'-GTG GAT ACC CCC TCC CCC AGC CTA GAC C) (SEQ ID NO:41); a second sense primer specific for the genomic PrP coding region (which was removed from the MoPrP vector) (5'-CCT CTT TGT GAC TAT GTG GAC TGA TGT CGG) (SEQ ID NO:42); and two sense and anti-sense primers specific for the PS1 transgene cDNA (PS1-a: 5'-AAT AGA GAA CGG CAG GAG CA (SEQ ID NO:43), and PS1-b: 5'-GCC ATG AGG GCA CTA ATC AT) (SEQ ID NO:44). All reactions give a 750-bp product of the endogenous PrP gene as a control for DNA integrity and successful amplification; PS1 transgene-positive samples have an additional band at approximately 608 bp.
**(xxii) *Glatiramer acetate vaccination.*** Each mouse was subcutaneously injected five times with a total of 100 µg of glatiramer acetate (GA (TV-5010), MW 13.5-18.5 kDa, average 16 kDa, Teva Pharmaceutical Industries Ltd., Petach Tikva, Israel), emulsified in 200 µl PBSxl, from experimental day 0 until day 24, twice during the first week and once a week thereafter.
**(xxiii) *Behavioral testing.*** Spatial learning/memory was assessed by performance on a hippocampus-dependent visuo-spatial learning task in the Morris water maze (MWM) (Morris, 1984). Mice were given four trials per day on 4 consecutive days, during which they were required to find a hidden platform located 1.5 cm below the water surface in a pool 1.4 m in diameter. Within the testing room, only distal visuo-spatial cues for location of the submerged platform were available. The escape latency, i.e., the time required by the mouse to find the platform and climb onto it, was recorded for up to 60 s. Each mouse was allowed to remain on the platform for 30 s and was then moved from the maze to its home cage. If the mouse did not find the platform within 60 s, it was placed manually on the platform and returned to its home cage after 30 s. The interval between trials was 300 s. On day 5 the platform was removed from the pool and each mouse was tested by a probe trial for 60 s. On days 6 and 7 the platform was placed at the quadrant opposite the location chosen on days 1-4, and the mice were then retrained in four sessions per day. Data were recorded using an EthoVision automated tracking system (Noldus).
***(xxiv) Administration of BrdU and tissue preparation.*** BrdU was dissolved by sonication in PBS and injected i.p. into each mouse (50 mg/kg body weight; 1.25 mg BrdU in 200 µl PBS×1). Starting from experimental day 22 after the first GA vaccination, BrdU was injected i.p. twice daily, every 12 h for 2.5 days, to label proliferating cells. Three weeks after the first BrdU injection the mice were deeply anesthetized and perfused transcardially, first with PBS and then with 4% paraformaldehyde. The whole brain was removed, postfixed overnight, and then equilibrated in phosphate-buffered 30% sucrose. Free-floating 30-µm sections were collected on a freezing microtome (Leica SM2000R) and stored at 4°C prior to immunohistochemistry.
***(xxv) Neural progenitor cell culture.*** Coronal sections (2 mm thick) of tissue containing the subventricular zone of the lateral ventricle were obtained from the brains of adult C57B1/6J mice. The tissue was minced and then incubated for digestion at 37°C, 5% CO₂ for 45 min in Earle's balanced salt solution containing 0.94 mg/ml papain (Worthington, Lakewood, NJ) and 0.18 mg/ml of L-cysteine and EDTA. After centrifugation at 110 × g for 15 min at room temperature, the tissue was mechanically dissociated by pipette trituration. Cells obtained from single-cell suspensions were plated (3500 cells/cm²) in 75-cm² Falcon tissue-culture flasks (BD Biosciences, San Diego, CA), in NPC-culturing medium [Dulbecco's modified Eagles's medium (DMEM)/F12 medium (Gibco/Invitrogen, Carlsbad, CA) containing 2 mM L-glutamine, 0.6% glucose, 9.6 µg/ml putrescine, 6.3 ng/ml progesterone, 5.2 ng/ml sodium selenite, 0.02 mg/ml insulin, 0.1 mg/ml transferrin, 2 µg/ml heparin (all from Sigma-Aldrich, Rehovot, Israel), fibroblast growth factor-2 (human recombinant, 20 ng/ml), and epidermal growth factor (human recombinant, 20 ng/ml; both from Peprotech, Rocky Hill, NJ)]. Spheres were passaged every 4-6 days and replated as single cells. Green fluorescent protein (GFP)-expressing NPCs were obtained as previously described (Pluchino et al., 2003).
***(xxvi) Primary microglial culture.*** Brains from neonatal (P0-P1) C57B1/6J mice were stripped of their meninges and minced with scissors under a dissecting microscope (Zeiss, Stemi DV4, Germany) in Leibovitz-15 medium (Biological Industries, Kibbutz Beit Ha-Emek, Israel). After trypsinization (0.5% trypsin, 10 min, 37°C/5% CO₂), the tissue was triturated. The cell suspension was washed in culture medium for glial cells [DMEM supplemented with 10% fetal calf serum (FCS; Sigma-Aldrich, Rehovot), L-glutamine (1 mM), sodium pyruvate (1 mM), penicillin (100 U/ml), and streptomycin (100 mg/ml)] and cultured at 37°C/5% CO₂ in 75-cm² Falcon tissue-culture flasks (BD Biosciences) coated with poly-D-lysine (PDL) (10 mg/ml; Sigma-Aldrich, Rehovot) in borate buffer (2.37 g borax and 1.55 g boric acid dissolved in 500 ml sterile water, pH 8.4) for 1 h, then rinsed thoroughly with sterile, glass-distilled water. Half of the medium was changed after 6 h in culture and every 2^{nd} day thereafter, starting on day 2, for a total culture time of 10-14 days. Microglia were shaken off the primary mixed brain glial cell cultures (150 rpm, 37°C, 6 h) with maximum yields between days 10 and 14, seeded (10⁵ cells/ml) onto PDL-pretreated 24-well plates (1 ml/well; Coming), and grown in culture medium for microglia [RPMI-1640 medium (Sigma-Aldrich) supplemented with 10% FCS, L-glutamine (1 mM), sodium pyruvate (1 mM), β-mercaptoethanol (50 mM), penicillin (100 U/ml), and streptomycin (100 mg/ml)]. The cells were allowed to adhere to the surface of a PDL-coated culture flask (30 min, 37°C/5% CO₂), and non-adherent cells were rinsed off.
***(xxvii) Co-culturing of mouse neural progenitor cells and mouse microglia***. Cultures of treated or untreated microglia were washed twice with fresh NPC-differentiation medium (same as the culture medium for NPCs but without growth factors and with 2.5% FCS) to remove all traces of the tested reagents, then incubated on ice for 15 min, and shaken at 350 rpm for 20 min at room temperature. Microglia were removed from the flasks and immediately co-cultured (5 × 10⁴ cells/well) with NPCs (5 × 10⁴ cells/well) for 10 days on cover slips coated with Matrigel™ (BD Biosciences) in 24-well plates, in the presence of NPC differentiation medium. The cultures were then fixed with 2.5% paraformaldehyde in PBS for 30 min at room temperature and stained for neuronal and glial markers.
***(xxviii) Immunocytochemistry and immunohistochemistry.*** Cover slips from co-cultures of NPCs and mouse microglia were washed with PBS, fixed as described above, treated with a permeabilization/blocking solution containing 10% FCS, 2% bovine serum albumin, 1% glycine, and 0.1% Triton X-100 (Sigma-Aldrich, Rehovot), and stained with a combination of the mouse anti-tubulin β-II-isoform C-terminus antibodies (β-III-tubulin; 1:500; Chemicon, Temecula, CA) and CD11b (MAC1; 1:50; BD-Pharmingen, Franklin Lakes, NJ).

For BrdU staining, sections were washed with PBS and incubated in 2N HCl at 37°C for 30 min. Sections were blocked for 1 h with blocking solution (PBS containing 20% normal horse serum and 0.1% Triton X-100, or PBS containing mouse immunoglobulin blocking reagent obtained from Vector Laboratories (Burlingame, CA)).

For immunohistochemistry, tissue sections were treated with a permeabilization/blocking solution containing 10% FCS, 2% bovine serum albumin, 1% glycine, and 0.05% Triton X-100 (Sigma-Aldrich, St. Louis). Tissue sections were stained overnight at 4°C with specified combinations of the following primary antibodies: rat anti-BrdU (1:200; Oxford Biotechnology, Kidlington, Oxfordshire, UK), goat anti-DCX [doublecortin] (1:400; Santa Cruz Biotechnology), and mouse anti-NeuN [neuronal nuclear protein] (1:200; Chemicon, Temecula, CA). Secondary antibodies were FITC-conjugated donkey anti-goat, Cy-3-conjugated donkey anti-mouse, and Cy-3- or Cy-5-conjugated donkey anti-rat (1:200; Jackson ImmunoResearch, West Grove, PA). For labeling of microglia we used either CD11b (MAC1; 1:50; BD-Pharmingen) or FITC-conjugated *Bandeiraea simplicifolia* isolectin B4 (IB4, 1:50; Sigma-Aldrich, Rehovot). To detect expression of cell-surface MHC-II proteins we used anti-MHC-II Abs (rat, clone IBL-5/22; 1:50; Chemicon, Temecula, CA). To detect expression of human Aβ we used anti-Aβ (human amino-acid residues 1-17) (mouse, clone 6E10, Chemicon, Temecula, CA). Expression of IGF-I was detected by goat anti-IGF-I Abs (1:20; R&D Systems). Expression of TNF-α, was detected by goat anti-TNF-α Abs (1:100; R&D Systems). T cells were detected with anti-CD3 polyclonal Abs (rabbit, 1:100; DakoCytomation, CA). Propidium iodide (1 µg/ml; Molecular Probes, Invitrogen, Carlsbad, CA) was used for nuclear staining.

Control sections (not treated with primary antibody) were used to distinguish specific staining from staining of nonspecific antibodies or autofluorescent components. Sections were then washed with PBS and coverslipped in polyvinyl alcohol with diazabicylo-octane as anti-fading agent.
***(xxix) Quantification and stereological counting procedure.*** For microscopic analysis we used a Zeiss LSM 510 confocal laser-scanning microscope (40x magnification). For experiments *in vitro* we scanned fields of 0.053 mm² *(n =* 8-16 from at least two different coverslips) for each experimental group. For each marker, 500-1000 cells were sampled. Cells co-expressing GFP and β-III-tubulin were counted.

For *in-vivo* experiments, the number of Aβ⁺ plaques and CD11b⁺/IB-4⁺ microglia in the hippocampus were counted at 300-µm intervals from 6-8 coronal sections (30 µm) from each mouse. Neurogenesis in the DG was evaluated by counting of premature neurons (DCX⁺), proliferating cells (BrdU⁺), and newly formed mature neurons (BrdU⁺/NeuN⁺) in six coronal sections (30 µm) from each mouse. Specificity of BrdU⁺/NeuN⁺ co-expression was assayed using the confocal microscope (LSM 510) in optical sections at 1-µm intervals/. Cell counts, numbers of Aβ⁺ plaques, plaque areas, and intensity of NeuN staining per unit area in the DG were evaluated automatically using Image-Pro Plus 4.5 software (Media Cybernetics).
***(xxx) Statistical analysis.*** MWM behavior scores were analyzed using 3-way ANOVA, with treatment group and trial block as sources of variation, was used to evaluate the significance of differences between mean scores during acquisition trial blocksin the MWM. When the *P*-value obtained was significant, a pairwise Fisher's least-significant-difference multiple comparison test was run to determine which groups were significantly different.

The *in-vitro* results were analyzed by the Tukey-Kramer multiple comparisons test (ANOVA) and are expressed as means ± SEM. *In-vivo* results were analyzed by Student's *t*-test or 1-way ANOVA and are expressed as means ± SEM.

### Example 3(1). T cell-based vaccination counteracts cognitive decline in AD

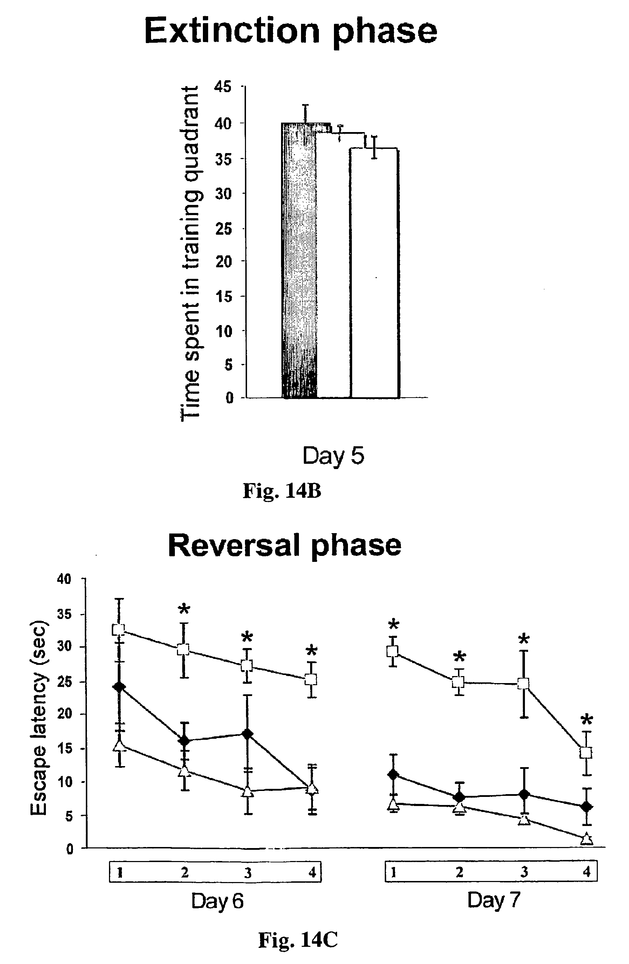

We examined the effect of GA in AD double-transgenic mice (Tg mice) expressing a mutant human presenilin 1 gene (*PS1dE9*) and a chimeric mouse/human amyloid precursor protein (*APPswe*), leading to learning/memory impairment and accumulation of Aβ plaques mainly in the cortex and the hippocampus, both characteristic features of early-onset familial AD (Borchelt et al., 1997). Expression of both transgenes in each mouse was verified by PCR amplification of genomic DNA. APP/PS1 Tg mice aged approximately 8 months were then vaccinated subcutaneously with GA (*n* = 6) twice during the first week and once a week thereafter. Age-matched Tg mice (*n* = 7) and non-Tg littermates that did not carry the transgenes (*n* = 6), were not treated and served as untreated Tg and wild-type non-Tg controls, respectively. Five weeks after the first GA injection all mice were assessed in a Morris water maze (MWM) for cognitive activity, as reflected by performance of a hippocampus-dependent spatial learning/memory task (reviewed in van Praag et al., 2000). The MWM performance of the untreated Tg mice was significantly worse, on average, than that of the age-matched non-Tg littermates **(****Fig. 14****).** However, the performance of Tg mice that were vaccinated with GA was superior to that of the untreated Tg mice and did not differ significantly from that of their non-Tg littermates **(****Fig. 14****),** suggesting that the GA vaccination had prevented further cognitive loss and even reversed part of the earlier functional deficit. Cognitive losses or improvements were manifested in both the acquisition and the reversal tasks **(****Figs. 14A-14C****).**

### Example 3(2). T cell-based vaccination modulates the immune activity of microglia, eliminates β-amyloid plaque formation, supports neuronal survival and induces neurogenesis

The above results prompted us to examine the possibility that the observed arrest and reversal of cognitive loss was related to reduction of Aβ plaques and survival of neurons in the hippocampus. Staining of brain cryosections from Tg mice with antibodies specific to human Aβ disclosed numerous plaques in the untreated Tg mice but very few in those vaccinated with GA **(****Fig. 15A****).** No plaques were seen in their respective non-Tg littermates **(****Fig. 15A****).** Examination of NeuN immunoreactivity disclosed loss of neurons in the untreated Tg mice but preservation of neurons in the GA-vaccinated Tg mice **(****Fig. 15A**).

Activated microglia are known to play a role in the pathogenesis of AD. We have shown in the Examples hereinbefore that, unlike microglia seen in association with inflammatory and neurodegenerative diseases, the microglia associated with neural tissue survival express MHC-II, produce IGF-I, and express little or no TNF-α. We therefore examined brain sections from GA-vaccinated and untreated Tg mice for the presence of microglia that stain positively for CD11b or TNF-α (markers of activation associated with a cytotoxic inflammatory phenotype). The presence of plaques was found to be correlated with the appearance of CD11b⁺ microglia **(****Fig. 15B****)** expressing TNF-α **(****Fig. 15C** and Movie S1 (prepared by the inventors but not shown here) that depicts a 3-D reconstruction of an Aβ plaque and CD11b⁺ microglia expressing TNF-α. This movie presents a representative 3-D confocal image of a microglial cell embedded within an Aβ plaque in the hippocampus of an untreated Tg mouse shown in Fig. 15C, in which the high TNF-α-immunoreactivity and engulfed Aβ in the cytoplasm can be noted), and was abundant in the untreated Tg mice. Significantly fewer CD11b⁺ microglia were detectable in the GA-vaccinated Tg mice **(****Fig. 15B****).** Staining with anti-MHC-II antibodies disclosed that in the untreated Tg mice almost no microglia expressed MHC-II (indicating their inability to act as APCs; data not shown), whereas in the GA-vaccinated Tg mice most of the microglia adjacent to residual Aβ⁺ plaques expressed MHC-II, and hardly any of them expressed TNF-α **(****Fig. 15D** and Movie S2 (prepared by the inventors but not shown here) that depicts 3-D reconstruction of an Aβ-immunoreactivity associated with MHC-II⁺ microglia in a GA-vaccinated Tg mouse. This movie also shows a representative 3-D confocal image of microglia shown in Fig. 15D, expressing marginal levels of TNF-α and high levels of MHC-II). These latter microglia also expressed IGF-I **(****Fig. 15E** and Movie S3 (prepared by the inventors but not shown here) that depicts a 3-D reconstruction of a microglial cell co-expressing IGF-1 and MHC-II⁺. This movie shows a representative 3-D confocal image of MHC-II⁺ microglia from the IGF-I-expressing GA-vaccinated Tg mouse shown in Fig. 15E.), indicating their potential for promoting neuroprotection and neurogenesis and for beneficially affecting learning and memory. All of the MHC-II⁺ cells were co-labeled with IB4, identifying them as microglia (data not shown). It is important to note that the CD11b⁺ microglia (seen mainly in the untreated Tg mice) showed relatively few ramified processes, whereas such processes were abundant in the MHC-II⁺ microglia in the GA-vaccinated Tg mice, giving them a bushy appearance (depicted in Movies S1 and S2, not shown).

In addition, unlike in the untreated Tg mice, in the GA-vaccinated Tg mice numerous T cells (identified by anti-CD3 antibodies) were seen in close proximity to MHC-II⁺ microglia. Any Aβ-immunoreactivity seen in these mice appeared to be in association with MHC-II⁺ microglia, creating an immune synapse with CD3⁺ T cells **(****Fig. 15F** and Movie S4 (prepared by the inventors but not shown here) that depicts a 3-D reconstruction of an Aβ plaque associated with CD3⁺ cells (T cells) in close proximity to MHC-II⁺ microglia. This movie depicts a representative 3-D confocal image of MHC-II⁴ microglia from the GA-vaccinated Tg mouse shown in Fig. 15F, with an immunological synapse between a CD3⁺ cell and a complex of MHC-II and Aβ.).

Quantitative analysis confirmed that mice vaccinated with GA showed significantly fewer plaques than untreated Tg mice when examined 6 weeks later **(****Fig. 15G****),** and that the area occupied by the plaques was significantly smaller than in their age-matched untreated counterparts **(****Fig. 15H****).** In addition, GA-vaccinated Tg mice showed significantly fewer CD11b⁺ microglia and significantly more intense staining for NeuN than their corresponding groups of untreated Tg mice **(****Figs. 15I****,** **15J****).**

Because MHC-II-expressing microglia are also associated with neurogenesis *in vitro,* we examined the same sections for the formation of new neurons in the dentate gyrus (DG) of the hippocampus. This was possible because all mice had been injected with BrdU, a marker of proliferating cells, 3 weeks before tissue excision. Quantitative analysis disclosed significantly more BrdU⁺ cells in GA-vaccinated Tg mice **(****Fig. 16A****)** than in their untreated counterparts. In addition, compared to the numbers of newly formed mature neurons (BrdU⁺/NeuN⁺) in their respective non-Tg littermates the numbers were significantly lower in the untreated Tg group, but were similar in the vaccinated group, indicating that the neurons had been at least partially restored by the GA vaccination **(****Fig. 16B****).** Analysis of corresponding sections for doublecortin (DCX), a useful marker for analyzing the absolute number of newly generated pre-mature neurons in the adult DG, disclosed that relative to the non-Tg littermates there were significantly fewer DCX⁺ cells in the DGs of untreated Tg mice, and slightly but significantly more in the DGs of Tg mice vaccinated with GA **(****Fig. 16C****).** Confocal micrographs illustrate the differences in the numbers of BrdU⁺/NeuN⁺ cells or of DCX⁺ cells and their dendritic processes between non-Tg littermates, untreated Tg mice, and GA-vaccinated Tg mice **(****Fig. 16D****).** The results showed that neurogenesis was indeed more abundant in the GA-vaccinated mice than in untreated Tg mice. Interestingly, however, in both untreated and GA-vaccinated Tg mice the processes of the DCX⁺-stained neurons in the subgranular zone of the DG were short, except in those GA-vaccinated mice in which the DCX⁺ cells were located adjacent to MHC-II⁺ microglia **(****Fig. 16E****).**

### Example 3(3). Aggregated β-amyloid induces microglia to express a phenotype that blocks neurogenesis, and the blocking is counteracted by IL-4

The *in-vivo* results presented above point to a relationship between arrested neurogenesis, aggregated Aβ, and the phenotype of the activated microglia. To determine whether aggregated Aβ-activated microglia block neurogenesis, and whether T cell-derived cytokines can counteract the inhibitory effect, we co-cultured GFP-expressing NPCs with microglia that had been pre-incubated for 48 h in their optimal growth medium in the presence or absence of aggregated Aβ peptide 1-40/1-42 (Aβ_{(1-40/1-42)}; 5 µM) and subsequently treated with IFN-γ (10 ng/ml), or with IL-4 (10 ng/ml) together with IFN-γ (10 ng/ml), for an additional 48 h. Growth media and cytokine residues were then washed off, and each of the treated microglial preparations was freshly co-cultured with dissociated NPC spheres on coverslips coated with Matrigel™ in the presence of differentiation medium **(****Fig. 17A****).** Expression of GFP by NPCs confirmed that any differentiating neurons seen in the cultures were derived from the NPCs rather than from contamination of the primary microglial culture. After 10 days we could discern GFP-positive NPCs expressing the neuronal marker β-III-tubulin βIIIT) **(****Figs. 17B, 17C****).** No βIIIT⁺ cells were seen in microglia cultured without NPCs. Significantly fewer GFP⁺/βIIIT⁺ cells were seen in control NPCs cultured without microglia (control). In co-cultures of NPCs with microglia previously activated by incubation with IFN-γ (10 ng/ml), however, the increase in numbers of GFP⁺/βIIIT⁺ cells was dramatic. In contrast, microglia activated by aggregated Aβ₍₁₋₄₀₎ (5 µM) blocked neurogenesis and decreased the number of NPCs. This negative effect was not exhibited by microglia activated by 5 µM Aβ₍₁₋₄₂₎ (data not shown). Interestingly, the addition of IL-4 (10 ng/ml) to microglia pre-treated with aggregated Aβ₍₁₋₄₀₎ partially counteracted the adverse effect of the aggregated Aβ on NPC survival and differentiation, with the result that these microglia were able to induce NPCs to differentiate into neurons **(****Fig. 17D****).** It thus appears that aggregated Aβ₍₁₋₄₀₎ impaired the ability to support neurogenesis, and that its effect could be counteracted to some extent by IL-4 and more strongly by the combination of IL-4 and IFN-γ.

### Discussion

In this study of APP/PS1 double-transgenic AD mice suffering from decline in cognition and accumulation of Aβ plaques, a T cell-based vaccination, by altering the microglial phenotype, ameliorated cognitive performance, reduced plaque formation, rescued cortical and hippocampal neurons, and induced hippocampal neurogenesis.

### EXAMPLE 4

### Combination of glatiramer acetate vaccination and stem cells in an animal model of amyotrophic lateral sclerosis (ALS)

### Materials and methods

***(xxxi) Animals.*** Transgenic mice overexpressing the defective human mutant SOD1 allele containing the Gly93→Ala (G93A) gene (B6SJL-TgN (SOD1-G93A)1Gur (herein "ALS mice") were purchased from The Jackson Laboratory (Bar Harbor, ME, USA).
***(xxxii) Immunization.*** Adult mice were immunized with Cop-1, 100 µg in 200µl PBS s.c.
***(xxxiii) Neural progenitor cell culture.*** Cultures of adult neural progenitor cells (aNPCs) were obtained as described in orevious examples
***(xxxiv) Stereotaxic injection of neural progenitor cells.*** Mice were anesthetized a week after the first immunization and placed in a stereotactic device. The skull was exposed and kept dry and clean. The bregma was identified and marked. The designated point of injection was at a depth of 2 mm from the brain surface, 0.4 mm behind the bregma in the anteroposterior axis, and 1.0 mm lateral to the midline. Neural progenitor cells were applied with a Hamilton syringe (5 x 10⁵ cells in 3 µl, at a rate of 1 µl/min) and the skin over the wound was sutured.
***(xxxv) Motor dysfunction.*** Motor dysfunction of the mice was evaluated using the rotarod task twice a week from 60 d of age onward. Animals were placed on a horizontal accelerating rod [accelerating rotarod (Jones and Roberts) for mice 7650] and time it took for each mouse to fall from the rod was recorded. We performed three trials at each time point for each animal and recorded the longest time taken. A cut-off time point was set to 180 sec and mice remaining on the rod for at least 180 sec were deemed asymptomatic. Onset of disease symptoms was determined as a reduction in rotarod performance between weekly time points. Animals were killed by euthanization when no longer able to right themselves within 30 seconds of being placed on their sides.

**Example 4.** The animals were treated with Cop-1 starting from day 59: in the first two weeks twice a week Cop-1, thereafter they received a weekly injection of Cop-1. The stem cells were given into the CSF: 500, 000 cells (single injection of adult neural stem cells).

The experiment was carried out in order to explore whether administration of a combination of Cop-1 vaccination and stem cells has beneficial effect in a mice model of ALS (herein "ALS mice"). For this purpose, 59 days old ALS mice were treated as follows: group 1 **(****Fig. 18****,** Cop-1+NPC) 4 males were immunized s.c. with 100µg/200µl Cop-1/PBS twice a week for 2 weeks (the first immunization was at age 59 days) and received thereafter one immunization per week until euthanization. With the third immunization, the mice received 100,000 NPC^{GFP} i.c.v (CSF) into the right cerebral ventricle; group 2 (**Fig.18****,** Cop-1) 5 males were immunized with 100µg/200µl Cop=1/PBS twice a week for 2 weeks and received thereafter one immunization per week until euthanization; group 3 **(****Fig.18****,** control) 5 males were immunized with 200µl PBS twice a week for 2 weeks and received thereafter one immunization per week until euthanization.

Mice from each of the groups were weighted (twice a week) and examined routinely for vital signs, and for signs of motor dysfunction. The results obtained in Fig. 18 show the probability of survival of each group of ALS mice. The results show that the combined treatment of Cop-1 vaccination and NPC results in increased survival of ALS mice.

### EXAMPLE 5

### Neurogenesis and neuroprotection induced by peripheral immunomodulatory treatment of EAE with glatiramer acetate

### Materials and Methods

*(xxxvi) Animals.* C57BL/6 mice were purchased from Harlan (Jerusalem, Israel). Yellow fluorescent protein (YFP) 2.2 transgenic mice, (originated from C57BL/6 and CBA hybrids), which selectively express YFP on their motor and sensory neuronal population (Feng et al., 2000), were kindly provided by J. R. Sanes (Washington University St. Louis, MO). Female mice, 8-10 weeks of age, were used in all experiments.
***(xxxvii) EAE.*** Disease was induced by immunization with the peptide p35-55 of rat MOG (SEQ ID NO:39), (Sigma, St. Louis, MO). Mice were injected subcutaneously at the flank, with a 200 µl emulsion containing 300 µg of MOG in CFA and 500 µg of heat-inactivated *Mycobacterium tuberculosis* (Sigma). An identical booster was given at the other flank one week later. Pertussis toxin (Sigma), 300 µg/mouse, was injected intravenously immediately after the first MOG injection and 48 h later. Mice were examined daily. EAE was scored as follows: 0, no disease; 1, limp tail; 2, hind limb paralysis; 3, paralysis of all four limbs; 4, moribund condition; 5, death.
***(xxxviii) Glatiramer acetate*** (GA, Copaxone®, Copolymer 1) consists of acetate salts of synthetic polypeptides containing four amino acids: L-alanine, L-glutamate, L-lysine, and L-tyrosine. GA from batch 242990599, with an average molecular weight of 7300 kDa, obtained from Teva Pharmaceutical Industries (Petach Tikva, Israel) was used throughout the study. GA treatment was applied by 5-8 consecutive daily subcutaneous injections (2 mg/mouse) in different stages of disease [i.e., (1) starting immediately after EAE induction (prevention treatment), (2) starting after appearance of disease manifestations at day 20 (suppression treatment), or (3) starting during the chronic phase 45 days after induction (delayed suppression).
***(xxxix) BrdU.*** 5-Bromo-2'-deoxyuridine (BrdU, Sigma), a thymidine analog incorporating into the DNA of dividing cells, was injected intraperitoneally (50 mg/kg), either concurrently with GA treatment (once each day), or immediately after completion of GA injections (twice each day).
***(xl) Perfusion.*** Animals were deeply anesthetized with Nembutal and perfused transcardially with 2.5% paraformaldehyde. Brains were removed, postfixed in 1% paraformaldehyde and cryoprotected with 15% sucrose solution in PBS. Free-floating sections (16 µm thick) were cut coronally or sagitaly with a sliding microtome (Leica SM 2000r) through the entire brain and collected serially in PBS.
***(xli) Immunohistochemistry.*** To detect BrdU-incorporated cells, sections were denatured in 2M HCl in PBS at 37°C for 30 min and then neutralized with 0.1M borate buffer (pH 8.5) for 10 min at room temperature. To detect specific cell types, sections were pre-incubated in PBS solution containing 20% serum and 0.5% Triton-X-100 for 1h, and then incubated overnight at room temperature with primary antibodies. The following primary antibodies were used: goat anti-doublecortin (DCX) C-18 (1:200, Santa Cruz Biotechnology, Santa Cruz, CA), mouse anti-NeuN (1:300, Chemicon, Temecula, CA), mouse anti-GFAP (1:100, Pharmingen, San Jose, CA), rabbit anti-phospho-histone (1:200, Upstate Biotechnology, Charlottesville, VA), rat anti-BrdU (1:200, Harlan, Indianapolis, IN), rat anti-CD11b (1:50, Pharmingen) and chicken anti-BDNF (1:50, Promega, Madison, WI). The second antibody step was performed by labeling with highly cross-absorbed Cy2- or Cy3-conjugated antibodies to rat, mouse, rabbit, goat or chicken (Jackson ImmunoResearch, West Grove, PA), to avoid cross-reactivity, (1:200; 20-40 min). Control slides were incubated with secondary antibody alone. In some cases, to enhance the signal, we used biotinylated secondary antibodies for 90 min, followed by Cy2- or Cy3-conjugated Streptavidin (Jackson ImmunoResearch). Sections were stained with Hoechst 33258 (Molecular Probes) for nuclear labeling. For detection of apoptosis, we used either rabbit anti-cleaved caspase-3 (1:75, Cell Signaling Technology, Beverly, MA) or TUNEL assay (Apoptag Fluorescein Detection Kit, Intergen, Purchase, NY). In addition, we used Fluro-Jade B derivate (Chemicon), which specifically binds to degenerating neurons.
***(xlii) Microscopy.*** Stained sections were examined and photographed by a confocal microscope (Axiovert 100M; Zeiss, Oberkochen, Germany), or by a fluorescence microscope (E600; Nikon, Tokyo, Japan), equipped with Plan Fluor objectives connected to CCD camera (DMX1200F, Nikon). Digital images were collected and analyzed using Image Pro+software. Images were assembled using Adobe Photoshop (Adobe Systems, San Jose, CA).
***(xliii) Quantification.*** Neuronal progenitor cells were quantified by counting the BrdU⁺ cells (those with BrdU/DCX dual staining) and by counting DCX⁺ cells (in the SGZ) or by measuring the DCX stained area (in the SVZ and RMS, where density did not permit counting of individual cells). Quantification was performed in coronal sections, in the SVZ starting at the level of the medial septum and 640 µm backward, and in the hippocampal DG (in both blades for BrdU/DCX or in the upper blade for DCX) through its septotemporal axis. Quantification in the RMS was done on sagital sections starting at 1 mm from the median line of the brain and 640 µm laterally. The Results for each brain structure were averaged from 8 unilateral levels per mouse (80 µm apart, 3-4 mice in each treatment group) and are expressed as change fold from naive controls. Quantification of BrdU/NeuN double positive cells in the cortex was performed in areas of 0.15 mm², selected at random (10 sections counted/mouse, 3 mice in each treatment group).
***(xliv) Statistical analysis.*** For BrdU and DCX analysis, the mean ± SEM (averaged from 8 unilateral levels per mouse, 3-4 mice in each treatment group) was subjected to one-way analysis of variance (ANOVA), followed by Fishers' least significant difference (LSD) at comparison-wise error rate of 0.05, where appropriate. Since control values for BrdU incorporation were reduced as a function of time, results were expressed as change fold from naive controls injected concurrently with BrdU. The number of BrdU/NeuN double-positive cells in the cortex was averaged from 10 sections per mouse (3 mice in each treatment group) and expressed as cells per mm³.
***(xlv) Preparation of stem cells.*** ROSA26 mice express lacZ in all tissues of the embryo and in most tissues of the adult mouse. Bone marrow (BM) cells were isolated from ROSA26 mice by flushing the femur and tibias with Hanks balanced salt containing 10% fetal bovine serum. A single cell suspension was prepared for transplantation.

### Example 5(1). Description of the experimental model

To study the manifestations of EAE as well as GA treatment in the CNS, we used the MOG 35-55 peptide-induced EAE model in two mice strains: the C57BL/6 susceptible strain and the YFP 2.2 transgenic mice, which selectively express YFP (yellow fluorescent protein) on their motor and sensory neuronal population, and thus provide a simple tool to follow axonal/neuronal damage (Feng et al., 2000). YFP 2.2 mice were susceptible to MOG-induced EAE similarly to C57BL/6 mice **(****Fig. 19A****).** In both strains, EAE induction resulted in chronic (non-remitting) disease, starting on days 16-20 (increasing in severity, reaching an average score of 3 by day 20-24), and maintained in chronic phase, grade 2-2.5, until perfusion. GA treatment was applied by 5-8 daily injections in different stages: (1) starting immediately after disease induction (prevention treatment); (2) starting after appearance of disease manifestations at day 20 (suppression treatment); or (3) starting during the chronic phase, 45 days after induction (delayed suppression). GA ameliorated the clinical manifestations of EAE regardless of the stage in which it was administered **(****Fig. 19B****).** The beneficial effect was stable over time and sustained until the mice were killed. The *in situ* manifestations in brains of EAE-inflicted mice (EAE mice) versus EAE-induced mice treated with GA (EAE+GA) were studied in comparison to brains of naive mice (control).

### Example 5(2). Characterization of neurological damage

In YFP 2.2 mice, YFP was expressed mainly by axons and dendrites. Partial population in the cerebral cortex and the hippocampus expressed YFP in cell bodies as well. YFP expression in brain sections from mice that had suffered grade 2-4 EAE revealed multiple neuronal malformations manifested in axonal transection, sparse processes and fiber deterioration **(****Fig. 20A****).** Multiple widespread lesions were frequently observed in various brain regions **(****Fig. 20B****),** indicative of considerable neuronal and axonal loss. An additional deformation in cell morphology in EAE mice was enlargement and swelling of neuronal cell body accompanied by margination of the nucleus as evident by distended hollow Hoechst-stained nuclei **(****Fig. 20C****).** These defects did not result from abnormality of the transgenic strain, since similar phenomena were observed in EAE-induced C57BL/6 mice stained by the neuronal marker NeuN (data not shown). Staining with Fluoro-jade B, which binds to degenerating neurons, revealed positively stained cells in the cortex, 25 days after disease induction, which is the peak of clinical manifestations **(****Fig. 20D****).** Yet, we could not see significant amount of apoptosis in the cortex and the striatum of both strains using either cleaved caspase-3 antibody or TUNEL assay, indicating that apoptotic mechanisms could not account for the damage extent in this model. Perivascular infiltrations of CD3-stained cells were found adjacent or inside aberrant regions, indicating the detrimental role of infiltrating T-cells **(****Fig. 20A****).** In naive controls as well as in mice injected with GA but not induced with EAE, we did not find neuronal malformations or perivascular infiltrations (not shown).

In brains of EAE+GA mice (either prevention or suppression treatment), considerably less damage was detected than in brains of EAE mice, revealing a smaller amount of deteriorating fibers **(****Fig. 20A****),** reduced number and size of lesions (Fig. 2B) and less swollen cell nuclei **(****Fig. 20C****).** A thin layer of YFP positive fibers was frequently found over the lesions in the GA treated animals (Fig. B), suggesting surviving filaments or axonal sprouting in the damaged areas. T-cells infiltrations were found also in brains of GA treated mice, yet, in smaller amount and their position was not associated with damage (Fig. 20A).

### Example 5(3). Microglia activation

Immunostaining for MAC-1 (CD11b, expressed on macrophages and microglia and up-regulated after their activation), correlated with the extent of neuronal injury in EAE mice (shown in the cerebellum, **Fig. 21A****).** Thus, in areas occupied with activated microglia, sparse fibers and axonal loss were generally evident (box I), whereas in adjacent areas of non-activated microglia, neuronal structure seemed intact (box II). Perivascular infiltration of activated MAC-1⁺ cells was found in injured areas suggesting that peripherally originated macrophages were also involved in the pathological process. As shown in **Fig. 21B****,** the vast increase in MAC-1 staining intensity found in EAE mice was demonstrated in additional brain regions e.g. striatum, thalamus and hippocampus. MAC-1⁺ cells in brains of control mice had relatively small cell body and long branched processes indicative of resting microglia. In contrast, brains of EAE mice, manifested rounding cell body with increased size and numerous retracted short processes, indicative of highly activated microglia (insert). In brains of EAE+GA mice, MAC-1 expression was significantly reduced, exhibiting moderate extent of activation. Cell morphology of MAC-1⁺ cells in GA-treated mice was similar to that of non-activated microglia in naive mice (insert). This arrest of microglial activation in EAE+GA mice was found at various times up to 30 days after termination of GA injections.

### Example 5(4). Proliferation of neuronal progenitor cells

To evaluate the generation and proliferation of neuronal progenitor cells following the pathological process of EAE, as well as after GA treatment, we used two markers: the immature neuronal marker DCX (associated with migrating and differentiating neurons of fetal and adult brain), and BrdU (thymidine analog incorporating into DNA of dividing cells) that had been injected concurrently with GA treatment. Hence, DCX expression indicated the amount of new neurons generated 10-14 days before animal sacrifice, and the number of BrdU incorporated cells (those with BrdU/DCX dual staining) indicated the number of neuroprogenitors emerging during the BrdU injection period. Neuroproliferation was studied in the neuroproliferative zones - the subventricular zone (SVZ) as well as in the subgranular zone (SGZ) and the granular cell layer (GCL) of the hippocampus. BrdU and DCX manifested overlapping patterns.

In the SVZ of EAE mice, neuroprogenitor proliferation was elevated following disease appearance (25 days after EAE induction, **1** day after last BrdU injection) in comparison to the controls **(****Figs. 22A****, 22BI).** This was evident by a 2.1 and a 1.4 fold increase in BrdU and DCX expression, respectively **(****Fig. 22D****,** SVZ, I, red columns). Still, 10 and 20 days later there was no significant difference in BrdU and DCX expression between EAE mice and naïve controls **(****Figs. 22B, 22D****, II, III).** Furthermore, in mice enduring disease for prolonged periods (35 and 60 days), proliferation manifested by BrdDU incorporation was lower than that of controls, either when BrdU was injected concurrently with EAE induction and tested one month later **(****Fig. 22D****,** first red column) or during the chronic stage before perfusion **(****Fig. 22D****,** last red column). GA treatment in EAE mice (administration schedules illustrated in **Fig. 22E****)** augmented neuronal proliferation in the SVZ in comparison to untreated EAE mice, as well as to controls **(****Figs. 22A****, 22B).** This elevation reached statistical significance over control and EAE for both BrdU and DCX by the suppression treatment, 1 and 10 days after termination of GA injection **(****Fig. 22D****).** Delayed suppression treatment resulted in significant elevation over EAE but not control. In the prevention treatment, substantial elevation over EAE and control was observed only for DCX (4-fold), though even BrdU incorporation was indicative of significant elevation compared to EAE.

In the SGZ of the hippocampus, neuronal proliferation was elevated following disease appearance, but subsequently declined below that of naive control **(****Figs. 22C, 22D****,** hippocampus, red columns). The effect of GA treatment in the hippocampus was similar to its effect in the SVZ, namely increased proliferation manifested by both BrdU incorporation and DCX expression that was not sustained after termination of the suppression treatments. Prevention as well as delayed suppression treatments resulted in higher neuroproliferation than in EAE mice, one month and one day after termination of GA/BrdU injection, respectively. Notably, in the hippocampus of EAE mice, and to a greater extent in EAE+GA mice **(****Fig. 22C****),** BrdU⁺/DCX⁺ cells were found in the SGZ and in the adjacent GCL. The DCX expressing cells manifested dense and branched dendritic tree with well-developed apical dendrites that crossed the inner molecular layer and extended into the outer molecular layer.

GA injection to naive mice (without EAE), either just prior to perfusion or a month earlier, did not result in significant elevation of BrdU or DCX expression, in both the SVZ and the DG **(****Fig. 22D****,** gray columns).

### Example 5(5). Migration of neuronal progenitor cells

To study the destiny of the induced progenitor cells, we first followed their mobilization into the route in which SVZ cells normally migrate in adult mice - the rostral migratory stream (RMS, illustrated in **Fig. 23A****).** As depicted in a segment adjacent to the SVZ **(****Fig. 23D****)** and in a more medial section **(****Fig. 23E****),** the amount of BrdU as well as DCX labeled cells migrating along the RMS of EAE mice (25 days post disease induction, 1 day after last BrdU injection), was elevated in comparison to controls. This was manifested by a 3.1 and a 1.6 fold elevation in the number of BrdU⁺/DCX⁺ cells and in the DCX stained area, respectively **(****Figs. 23F, 23G****,** red columns). After GA treatment (on days 20-25, suppression), the amount of neuronal progenitors in the RMS was even higher, exhibiting an extensive stream of BrdU/DCX expressing cells **(****Figs. 23B, 23D, 23E****).** Thus, an increase of 7.8 and 2.6 fold in BrdU and DCX expression over control, and 2.2 and 1.6 fold over EAE mice was obtained following GA treatment **(****Figs. 23F, 23G****,** blue columns). Notably, injection of GA alone also enhanced mobilization of progenitors into the RMS, but to a lower extent **(****Figs. 23F, 23G****,** gray columns).

Similar mobilization patterns of neuronal progenitors were found at a later time point (35 days after EAE induction, when GA treatment was given as prevention treatment, i.e., elevated DCX expression in the RMS of EAE vs. control mice, and even more robust migration in EAE+GA mice) **(****Fig. 23H****).** Interestingly, in one EAE mouse (out of 13 mice), we found enhanced neuronal migration similar to that of GA-treated mice. This mouse (denoted EAE-rec) exhibited only slight, short term disease (score 2, at days 24-26 after induction), and completely recovered by the day of perfusion.

Treatment of EAE mice with GA led not only to enhanced mobilization of neuronal progenitors through the RMS, but also to their migration into a region corresponding to the lateral cortical stream (LCS) of neuronal migration, naturally found in the developing forebrain (illustrated in **Fig. 23A****).** Hence, DCX⁺ cells appeared to travel from the SVZ caudally, in a chain along the corpus callosum and the hippocampo-callosal interface, towards various cortical regions mainly the occipital cortex **(****Fig. 23C****).** We could not trace such mobilization patterns in corresponding sections of EAE mice not treated with GA. Furthermore, in EAE+GA mice, neuronal progenitors diverged from the classic neuroproliferative zones, as well as the migratory streams and spread to atypical regions such as the striatum, nucleus accumbens and the cortex **(****Fig. 24****).** The DCX⁺ cells appeared to move away from the RMS in close proximity to YFP expressing filaments, suggesting their migration along nerve fibers **(****Fig. 24A****).** As seen by their direction and orientation, they migrated away from both the RMS and the SVZ, yet in some mice most cells extended from the SVZ **(****Fig. 24B****),** whereas in others the RMS seemed as their major origin **(****Fig. 24C****).** DCX⁺ cells appeared to reach into the frontal cortex from the RMS **(****Figs. 23B****,** **24D****)** and to the occipital cortex from the LCS **(****Fig. 23C****).** They manifested morphological features characteristic of migrating neurons, such as fusiform somata with a leading and trailing process (O'Rourke et al., 1995), and their orientation was consistent with migration away from the migratory stream into the internal part of the cortex, along nerve fibers (shown in layers 5 and 6, **Figs. 24E, 24F****).** We did not detect neuroprogenitors in areas remote from the neuroproliferative zones and the migratory streams such as the cerebellum and the pons.

At early time point following GA treatment and BrdU injection (1-10 days), neuronal progenitors that migrated away from the RMS manifested BrdU and DCX. co-expression as shown in the striatum **(****Fig. 25A****)** and the accombens nucleus **(****Fig. 25B****),** indicating that they underwent division concurrently with GA treatment. In some cases, these double positive cells appeared in small clusters, suggesting local divisions as well. Furthermore, staining with phosphorylated histone H³, an endogenous marker of cells in M phase, indicated that some DCX⁺ cells had proliferated just prior to perfusion, as seen for the neuroprogenitors accumulated in the nucleus accombens in **Fig. 25C****.**

At later time points (one month after completion of GA treatment), BrdU⁺ cells co-expressing the neuronal nuclear antigen (NeuN), were found in the striatum **(****Figs. 25D, 25F****),** nucleus accumbens **(****Fig. 25E****),** and cortex **(****Fig. 25G****,** cingulate cortex layer 5), indicating that some neuroprogenitor cells have differentiated further toward a mature neuronal phenotype. In the cortex **(****Fig. 25H, 25I****,** cingulate, **Fig. 25J****,** occipital, **Fig. 25K****,** motor) of YFP mice, pyramidal cells co-expressing BrdU and YFP with apical dendrites and axons were observed, indicative of mature functional neurons. An average of 128 ± 46/mm³ BrdU⁺/NeuN⁺ double-labeled cells were found in the cortex of EAE+GA mice, consisting of 1.3% of all NeuN⁺ cells. It should be noted that BrdU⁺/NeuN⁺ cells were found also in the cortex of EAE mice not treated with GA, though fewer (48 ± 25/mm³, 0.58% from NeuN⁺ cells). In the cortex of naive mice, BrdU⁺/NeuN⁺ cells were not found.

### Example 5(6). Migration to lesion sites

The newly generated neurons seemed to be attracted to damaged regions. Hence, clusters of DCXBrdU as well as NeuN/BrdU co-expressing cells, were situated in areas with deteriorating YFP expressing fibers and lesions **(****Figs. 24B, 24C****,** **25A-25D****).** Furthermore, DCX expressing cells, were found around the margins and inside lesions in the striatum **(****Figs. 26B, 26C****),** cortex **(****Figs. 26D, 26E****)** and the nucleus accombens **(****Fig. 26F****).** In EAE mice (not treated by GA), a few DCX⁺ cells surrounding lesions also observed **(****Fig. 26A****),** but in EAE+GA mice the amount of progenitors migrating to the lesions was much higher. The DCX⁺ neuroprogenitors localized into areas extensively occupied with astrocytes expressing GFAP **(****Figs. 27A-27C****),** suggesting their migration into gliotic scar areas.

In lesions occupied by DCX⁺ cells **(****Figs. 26D-26F****),** we observed YFP expressing fibers extending into lesions, suggesting the induction of axonal regeneration, or sprouting, by the neuroprogenitors. To find out if the newly generated neurons can actually induce a growth-promoting environment, we tested their ability to express BDNF. As shown in **Fig. 27****,** in the nucleolus accumbens **(****Figs. 27D, 27E****),** and the hippocampus **(****Fig. 27F****),** substantial proportion of the migrating DCX⁺ cells, in EAE+GA mice, manifested extensive expression of BDNF.

### Example 5 (7). Combination treatment of glatiramer acetate and progenitor stem cell in a mice model of EAE.

The following experiment was carried out in order to assess the effect of the combination of glatiramer acetate (GA) and stem cells administration in an EAE model. We employed the myelin oligodendrocyte glycoprotein (MOG) induced EAE mice model. Neuronal and axonal degeneration are extensively manifested in EAE mice. GA treatment was applied in EAE mice in combination with stem cells by a procedure, which was found to be effective in generating self- neurogenesis, namely by daily subcutaneous injections.

Multipotent stem cells were obtained from bone marrow of ROSA26 transgenic mice, which express Lac-z in most tissues of the adult mouse. Expression of Lac-z gene was detected by enzymatic activity of the gene product beta-galactosidase. These stem cells were transplanted into EAE C57BL/6 mice by local stereotactical inclusion into the lateral ventricle of the brain. Alternatively, it is possible to administer stem cells systemically by intravenous injection.

The effect of combined GA and stem cells administration was compared to administration of GA and stem cells separately. Untreated EAE mice and naive mice served as controls. After treatment, mice were inspected daily for neuronal symptoms and scored for disease severity and/or clinical improvement. The in situ effect of the treatments was evaluated by characterization of neuronal damage as described in 5(2) above. The fate of the transplanted cells was monitored using immunohistological methods as described in 5(4), 5(5) and 5(6) above. For example, proliferation was assessed by using markers such as BrdU injected concurrently with transplantation and differentiation was monitored by detecting DCX and NeuN markers. We also followed the migration of the transplanted cells and their ability to reach the lesion site.

Preliminary results obtained indicate that combined GA+stem cell treatment augmented the beneficial effect of each treatment separately, as evidenced by the above parameters inspected. The same beneficial effects can be obtained by the combined treatment of GA and stem cells in other experimental models. Thus, the combined treatment of GA and stem cells can be used in therapy of additional neurological diseases and other disorders.

### Discussion

The major finding reported here is that peripheral immunomodulatory treatment of an inflammatory autoimmune neurodegenerative disease induces neuroprotection as well as augmentation of the self-neurogenesis triggered by the pathological process. This results in massive migration of new neurons into injury sites, in brain regions that do not normally undergo neurogenesis, suggesting relevance to the beneficial effect of GA in EAE and MS.

The histopathological manifestations of MOG-induced EAE, in both C57BL/6 and YFP 2.2 strains were deteriorating fibers, axonal loss, widespread lesions, and nucleus margination, indicative of severe damage **(****Fig. 20****).** Perivascular infiltrations of T-cells **(****Fig. 20A****)** and macrophages **(****Fig. 21A****)** were found in close proximity to aberrant regions, in consistence with their detrimental role in this disease (Behi et al., 2005; Stollg and Jander 1999). The protective effect of GA was manifested in prevention of the typical axonal and neuronal damage as evidenced in less deteriorating fibers, reduced amount of lesions with smaller magnitude and less marginized cell nuclei. Additional prominent effect of GA was the reduction in microglia activation **(****Fig. 21B**), manifested in all time points (1-30 days after treatment termination), by the various schedules. Microglia function as antigen-presenting cells within the CNS and thereby activate encphalitogenic T-cells and produce inflammatory toxic mediators, though, dual function due to their capacity to express neurotrophic factors was also demonstrated (Stollg and Jander 1999). In the current model, microglia activation was markedly elevated in EAE inflicted mice in various brain regions, and this activation correlated with the amount of neuronal injury.

GA treatment resulted not only in decreased neuronal damage but also in increased neuronal proliferation. The combination of two detection markers allowed us to evaluate both the amount of new neurons generated 10-14 days before the animal was killed, by the overall expression of the immature neuronal marker DCX (Bayer et al., 1991) as well as the number of neuroprogenitors emerging during the concurrent BrdU/GA injection period (those that differentiated into the neuronal lineage and thus presented BrdU/DCX dual staining). Both systems gave comparable results as to the effect of the pathological process of EAE and that of GA treatment. Hence, EAE induction triggered increased neuroprogenitor proliferation in the neuroproliferative zones (the SVZ and the SGZ) following disease appearance (Fig. 22), in accordance with previous studies demonstrating increased cell proliferation in these zones following injury (Jin et al., 2003; Magavi et al., 2000; Picard-Riera et al., 2002). Still this neuroproliferation decreased gradually and subsequently declined below that of naive mice, indicative of the impairment inflicted by the disease and the failure of self-neurogenesis to compensate for the damage. GA treatment applied by various schedules to EAE mice augmented neuronal proliferation in both the SVZ and the SGZ over that of EAE mice and prolonged its duration. Of special significance is the neuroproliferative consequence of GA treatment initiated in the chronic phase of the disease (delayed suppression), as this phase in EAE/MS is regarded as the stage in which exhausted self-compensating neurogenesis fails, and extensive neurodegeneration overcomes (Bjartmar et al., 2003; Hobom et al., 2004).

Neuroprogenitors originated in the SVZ were mobilized into the route in which they normally migrate in adults, the RMS. This mobilization was increased in EAE mice, and GA augmented it even further (Fig. 23). The therapeutic relevance of this effect is implied by the enhanced neuronal migration found in the EAE mouse that exhibited slight, short-term disease and spontaneous recovery. Still, in GA treated mice neuroprogenitor, migration was not confined to the RMS. We found recurrence of the LCS - neuronal migratory route, naturally found in the embryonic forebrain (Francic et al., 1999), as DCX-expressing cells migrated along the corpus callosum and the hippocampo-callosal interface, towards various cortical regions mainly to the occipital cortex **(****Fig. 23C****).** Furthermore, neuronal progenitors diverged from the classic neuroproliferative zones, as well as the migratory streams and spread to adjacent atypical brain regions that do not normally undergo neurogenesis such as the striatum, nucleus accumbens and the cortex **(****Fig. 24****).** In the hippocampus of EAE mice, subsequent to disease appearance and to a greater extent and longer duration in EAE+GA mice, BrdU and DCX expressing cells migrated from the SGZ into the adjacent GCL, extending branched dendrites through the inner and outer molecular layer **(****Fig. 22C****).** However, mobilization of SGL originating cells was probably restricted to the hippocampus, as we found no evidence for migration beyond this region, in accord with previous studies identifying the SVZ rather than the SGZ as the source of neuroprecursor migration (Jin et al., 2003).

At early time points following GA and BrdU injection (1-10 days after their last injection), BrdU⁺ neuroprogenitors expressed the immature neuronal marker DCX characteristic to migrating and differentiating neurons (Bernier et al., 2002), and displayed migratory morphology, fusiform somata with a leading and a trailing process (O'Rouke et al., 1995) **(****Fig. 24****).** It has been doubted whether progenitors retain their ability to proliferate after leaving the neuroproliferative zones (Gould and Gross, 2002; Iwai et al., 2002). In EAE+GA mice, we found small clusters of BrdU/DCX co-expressing cells in the striatum and the nucleus accumbens, suggesting local divisions. Furthermore, staining with phosphorylated histone an endogenous marker of cells in M phase indicated that some DCX⁺ cells in these regions had divided just prior to perfusion **(****Fig. 25****),** suggesting *in situ* proliferation outside the classic neuroproliferative zones. At later time point (one month after completion of GA treatment), DCX⁺ cells with branching processes **(****Fig. 26****)** as well as BrdU⁺ cells expressing the mature neuronal marker NeuN and displaying mature morphology were observed **(****Fig. 25****).** The amount of new neurons in the cortex of EAE mice was comparable to those found in other cases of damage-induced neurogenesis (Magavi et al., 2000; Picard-Riera et al., 2002; Arlotta et al., 2003). GA treatment increased this number by 2.6 fold, indicating substantial elevation of newly generated neurons. BrdU/NeuN⁺ cells were not found in cortex of naive mice, confirming that neurogenesis does not normally occur in the adult rodent cortex (Iwai et al., 2002; Jin et al., 2003; Arlotta et al., 2003). Thus, three processes comprising neurogenesis: cell proliferation, migration and differentiation (Jin et al., 2003; Chen et al., 2004), were elevated after GA treatment.

These findings establish correlation between GA treatment and generation of neuroprotection and neurogenesis. It is possible that these effects result from suppression of inflammation - the insult initiating the pathological process, and thus the subsequent damage, as demonstrated for anti-inflammatory treatment after endotoxin administration (Monje et al., 2003). The ability of GA to shift cytokine secretion from the Th1 inflammatory to the Th2/3 anti-inflammatory pathway was demonstrated in the periphery of mice and humans (Aharoni et al., 1998; Duda et al., 2000). Moreover, GA induces specific Th2/3-cells that cross the BBB, accumulate in the CNS (Aharoni et al., 2000, 2002) and express *in situ* the anti-inflammatory cytokines IL-10 and TGF-β (Aharoni et al., 2003). In the present study as well, infiltrating T-cells were found in brains of EAE+GA mice, and in contrast to EAE mice, their location was not associated with damage **(****Fig. 19C****).** However, the effect of the GA-induced cells in the CNS goes beyond blockage of inflammation. Hence, IL-10 was shown to modulate glial activation (Ledeboer et. al., 2000), thus its *in situ* expression may account for the blockade of microglia activation in GA treated mice **(****Fig. 21****).** As for TGF-β, its neuroprotective activity has been shown in various species (Dhandapani and Brann, 2003) as well as its ability to induce neurproliferation and differentiation (Newmann eyal., 2000; Kawauchi et al., 2003). Furthermore, GA-specific cells in the brain were shown to express the potent neurotrophic factor BDNF (Aharoni et al., 2003), a key regulator of neuronal survival and neurogenesis in the adult brain (Lassmann et al., 2003). BDNF was shown to stimulate recruitment of SVZ cells, their migration through the RMS to structures, which do not exhibit neurogenesis in adulthood, and their differentiation into neurons (Pencea et al., 2001), similarly to the finding in this study. Of special relevance therefore are our previous finding that adoptive transfer of GA specific T-cells or GA injection as such, induced bystander effect on CNS resident cells e.g. astrocytes and neurons, to extensively express IL-10, TGF-β and BDNF, resulting in their significant elevation in various brain regions (Aharoni et al., 2003, 2004).

It is of special significance that the newly generated neurons were attracted or recruited to damaged regions, as evidenced by their migration into gliotic scarred areas **(****Fig. 27****)** and to regions exhibiting fiber deterioration, neuronal loss and lesions **(****Figs. 24B, 24C****,** **25A-25D****,** **26****).** Directed migration of new neurons towards injury sites has been demonstrated following cerebral ischemia (Jin et al., 2003), as well as in this study in EAE mice **(****Fig. 26A****).** However, although lesions in EAE mice treated by GA were less extensive, the amount of progenitors migrating into them was drastically larger. These new neurons could constitute a pool for the replacement of dead or dysfunctional cells and/or induce growth-promoting environment that supports neuroprotection and axonal growth. The latter activity was evidenced by BDNF expression of the new neurons **(****Figs. 27D-27F****).** Moreover, in lesions occupied by neuroprogenitors, YFP expressing fibers extending into the lesions were observed **(****Figs. 20B****,** **26D-26F****),** suggesting the induction of axonal regeneration, or sprouting. The cumulative results presented here support the notion that an immunomodulatory drug can induce neuroprotection and neurogenesis that counteract the neurodegenerative disease course.

### REFERENCES

Aberg, M. A., Aberg, N. D., Hedbacker, H., Oscarsson, J. & Eriksson, P. S. Peripheral infusion of IGF-I selectively induces neurogenesis in the adult rat hippocampus. J Neurosci 20, 2896-903 (2000).
Aharoni R, Schlegel PG, Teitelbaum D, Roikhel-Karpov O, Chen Y, Arnon R, Sela M, Chao NJ. (1997) Studies on the mehanism and specificity of the effect of synthetic random copolymer GLAT on graft-versus-host disease. Immunol. Lett. 58 (2):79-87.
Aharoni R, Teitelbaum D, Sela M, Arnon R (1998) Bystander suppression of experimental autoimmune encephalomyelitis by T cell lines and clones of the Th2 type induced by copolymer 1. J. Neuroimmunol. 2:135-146
Aharoni R, Teitelbaum D, Leitner O, Meshorer A, Sela M, Arnon R (2000) Specific Th2 cells accumulate in the central nervous system of mice protected against experimental autoimmune encephalomyelitis by copolymer 1. Proc. Natl. Acad. Sci. USA. 97:11472-11477.
Aharoni R., Teitelbaum D., Arnon R., Sela M. (2001). Copolymer 1 inhibits manifestations of graft rejection. Transplantation 27: 598-605.
Aharoni R, Meshorer A, Sela M, Arnon R (2002) Oral treatment of mice with copolymer 1 (glatiramer acetate) results in the accumulation of specific Th2 cells in the central nervous system. J. Neuroimmunol. 126:58-68.
Aharoni R, Kayhan B, Eilam R, Sela M, Arnon R (2003) Glatiramer acetate-specific T cells in the brain express T helper 2/3 cytokines and brain-derived neurotrophic factor in situ. Proc. Nat. Acad. Sci. USA. 100:14157-14162.
Aharoni R, Eilam R, Labunskay G, Sela M Arnon R (2004) Copaxone (glatiramer acetate) injection augment brain derived neurotrophic factor expression in the brain. Multiple Sclerosis 10:S256
Aharoni R., Yussim A., Sela M., Arnon R. (2005). Combined treatment of glatiamer acetate and low doses of immunosuppressive drugs is effective in the prevention of graft rejection. Int Immunopharmacol. 5(1):23-32.
Aharoni R, Kayhan B, Arnon R (2005) Therapeutic effect of the immunomodulator glatiramer acetate on trinitrobenzene sulfonic acid-induced experimental colitis. Inflamm Bowel Dis. 11(2):106-115.
Angelov DN, Waibel S, Guntinas-Lichius O, Lenzen M, Neiss WF, Tomov TL, Yoles E, Kipnis J, Schori H, Reuter A, Ludolph A, Schwartz M. (2003). Therapeutic vaccine for acute and chronic motor neuron diseases: implications for amyotrophic lateral sclerosis. Proc. Natl. Acad. Sci. U.S.A. 100(8):4790-4795.
Arnon, R, Sela M. (2003) Immunomodulation by the copolymer glatiramer acetate. J. Mol. Recognit. 16:412-421.
Bakalash S, Kessler A, Mizrahi T, Nussenblatt R, Schwartz, M. (2003) Antigenic specificity of immunoprotective therapeutic vaccination for glaucoma. Invest. Ophthalmol. Vis. Sci. 44: 3374-3381.
Bayer SA, Altman J, Russo RJ, Dai XF, Simmons JA (1991) Cell migration in the embryonic neocortex. I. Comp. Neurol. 307:499-516.
Behi ME, Dubucquoi S, Lefranc D, Zephir H, De Seze J, Vermersch P, Prin L (2005) New insights into cell responses involved in experimental autoimmune encephalomyelitis and multiple sclerosis. Immunology Letters 96:11-26.
Benner EJ, Mosley RL, Destache CJ, Lewis TB, Jackson-Lewis V, Gorantla S, Nemachek C, Green SR, Przedborski S, Gendelman HE. (2004) Therapeutic immunization protects dopaminergic neurons in a mouse model of Parkinson's disease. Proc Natl Acad Sci USA. 101:9435-40.
Bernier PJ, Bedard A, Vinet J, Levesque M, Parent A (2002) Newly generated neurons in the amygdala and adjoining cortex of adult primates. Proc. Nat. Acad. Sci. USA. 99:11464-11469.
Bjartmar C, Wujek JR, Trapp BD (2003) Axonal loss in the pathology of MS: consequences for understanding the progressive phase of the disease. J. Neurol. Sci. 15:165-171
Bliss, T.V. & Collingridge, G.L. A synaptic model of memory: long-term potentiation in the hippocampus. Nature 361, 31-9 (1993).
Boje, K. M. & Arora, P. K. Microglial-produced nitric oxide and reactive nitrogen oxides mediate neuronal cell death. Brain Res. 587, 250-6 (1992).
Borchelt, DR et al., Neuron 19, 939 (1997).
Brown, J. et al. Enriched environment and physical activity stimulate hippocampal but not olfactory bulb neurogenesis. Eur J Neurosci 17, 2042-6 (2003).
Brown JP, Couillard-Despres S, Cooper-Kuhn CM, Winkler J, Aigner L, Kuhn HG. (2003) Transient expression of doublecortin during adult neurogenesis. J. Comp Neuron. 467: 1-10.
Butovsky O, Talpalar AE, Ben-Yaakov K, Schwartz M. Activation of microglia by aggregated beta-amyloid or lipopolysaccharide impairs MHC-II expression and renders them cytotoxic whereas IFN-gamma and IL-4 render them protective. Mol Cell Neurosci 29:381-393 (2005).
Butovsky, O., Hauben, E. & Schwartz, M. Morphological aspects of spinal cord autoimmune neuroprotection: colocalization of T cells with B7--2 (CD86) and prevention of cyst formation. Faseb J. 15, 1065-7 (2001).
Cameron, H.A. & McKay, R.D. Restoring production of hippocampal neurons in old age. Nat Neurosci 2, 894-7 (1999).
Cameron, H.A. & McKay, R.D. Adult neurogenesis produces a large pool of new granule cells in the dentate gyrus. J Comp Neurol 435, 406-17 (2001).
Cammer, W. & Zhang, H. Maturation of oligodendrocytes is more sensitive to TNF alpha than is survival of precursors and immature oligodendrocytes. J Neuroimmunol 97, 37-42 (1999).
Chen J, Magavi SS, Macklis JD (2004) Neurogenesis od corticospinal motor neurons extending spinal projections in adult mice. Proc. Nat. Acad. Sci. USA. 101:16357-16362.
Cummings, B. J., Uchida, N., Tamaki, S. J., Salazar, D. L., Hooshmand, M., Summers, R., Gage, F. H. & Anderson, A. J. Proc Natl Acad Sci U S A 102, 14069-74 (2005).
Dawson, M. R., Polito, A., Levine, J. M. & Reynolds, R. NG2-expressing glial progenitor cells: an abundant and widespread population of cycling cells in the adult rat CNS. Mol Cell Neurosci 24, 476-88 (2003).
Dhandapani KM, Brann DW (2003) Transforming growth factor-beta: a neuroprotective factor in cerebral ischemia. Cell Biochem Biophs. 39:13-22.
Dijkstra, C. D., De Groot, C. J. & Huitinga, I. The role of macrophages in demyelination. J Neuroimmunol 40, 183-8 (1992).
Duda, P.W., Schmied, M.C., Cook, S.L., Krieger, J.I., and Hafler, D.A. Glatiramer acetate (Copaxone) induces degenerate, Th2-polarized immune responses in patients with multiple sclerosis. J Clin Invest 105:967-976 (2000).
Ekdahl, C. T., Claasen, J. H., Bonde, S., Kokaia, Z. & Lindvall, O. Inflammation is detrimental for neurogenesis in adult brain. Proc Natl Acad Sci U S A 100, 13632-7 (2003).
Engesser-Cesar, C., Anderson, A.J., Basso, D.M., Edgerton, V.R. & Cotman, C.W. Voluntary wheel running improves recovery from a moderate spinal cord injury. J Neurotrauma 22, 157-71 (2005).
Enzmann, G. U., Benton, R. L., Woock, J. P., Howard, R. M., Tsoulfas, P. & Whittemore, S. R. Exp Neurol 195, 293-304 (2005).
Feng G, Mellor RH, Bernstein M, Keller-Peak Cynthia, Nguyen QT, Wallace M, Nerbonne JM, Lichtman JW, Samce JR (2000) Imaging neuronal subsets in transgenic mice expressing multiple spectral variants of GFP. Neuron 28:41-51.
Francis F, Koulakoff A, Boucher D, Chafey P, Schaar B, Vinet MC, Friocourt G, McDonnell N, Reiner O, Kahn A, McConnell SK, Berwald-Netter Y, Denoulet P, Chelly J. (1999) Doublecortin is a developmentally regulated microtobule-associated protein expressed in migrating and differentiating neurons. Neuron 23:247-256.
Fridkis-Hareli M, Aharoni R, Teitelbaum D, Arnon R, Sela M, Strominger JL. (1999) Binding motifs of copolymer 1 to multiple sclerosis- and rheumatoid arthritis-associated HLA-DR molecules. J Immunol. 162(8):4697-4704.
Gould E, Gross CG (2002) Neurogenesis in adult mammals: some progress and problems. J. Neuroscience. 22:619-623.
Gould, E., Beylin, A., Tanapat, P., Reeves, A. & Shors, T.J. Learning enhances adult neurogenesis in the hippocampal formation. Nat Neurosci 2, 260-5 (1999).
Hauben E, Schwartz M. Therapeutic vaccination for spinal cord injury: helping the body to cure itself. Trends Pharmacol Sci 24:7-12 (2003).
Hauben, E., Gothilf, A., Cohen, A., Butovsky, O., Nevo, U., Smirnov, I., Yoles, E., Akselrod, S. & Schwartz, M. (2003) J Neurosci 23, 8808-19.
Hauben, E., Agranov, E., Gothilf, A., Nevo, U., Cohen, A., Smirnov, I., Steinman, L. & Schwartz, M. Posttraumatic therapeutic vaccination with modified myelin self-antigen prevents complete paralysis while avoiding autoimmune disease. J. Clin. Invest. 108, 591-9 (2001).
Hauben, E., Butovsky, O., Nevo, U., Yoles, E., Moalem, G., Agranov, G., Mor, F., Leibowitz-Amit, R., Pevsner, E., Akselrod, S., Neeman, M., Cohen, I. R. & Schwartz, M. Passive or active immunization with myelin basic protein promotes recovery from spinal cord contusion. J. Neurosci. 20, 6421-6430 (2000).
Hellings N, Raus, J. Stinissen P (2002) Insights into the immunopathogenesis of multiple sclerosis. Immunol Res. 25:27-51.
Hobom M, Storch MK, Weissert R, Maier K, Radhakrishnan A, Kramer B, Bahr M, Diem R. (2004) Mechanisms and time course of neuronal degeneration in experimental autoimmune encephalomyelitis. Brain Pathol. 14:148-157.
Hsieh, J., Aimone, J.B., Kaspar, B.K., Kuwabara, T., Nakashima, K., and Gage, F.H. 2004. IGF-I instructs multipotent adult neural progenitor cells to become oligodendrocytes. J Cell Biol 164, 111-22 (2004).
Imitola, J., Raddassi, K., Park, K. I., Mueller, F. J., Nieto, M., Teng, Y. D., Frenkel, D., Li, J., Sidman, R. L., Walsh, C. A., Snyder, E. Y. & Khoury, S. J. Proc Natl Acad Sci U S A 101, 18117-22 (2004a).
Imitola, J., Comabella, M., Chandraker, A. K., Dangond, F., Sayegh, M. H., Snyder, E. Y. & Khoury, S. J. Neural stem/progenitor cells express costimulatory molecules that are differentially regulated by inflammatory and apoptotic stimuli. Am J Pathol 164, 1615-25 (2004b).
Ishii, K. et al., Faseb J 14, 1485 (2000).
Iwai M, Sato K, Omori N, Nagano I, Manabe Y, Shoji M, Abe K. (2002) Three steps of neural stem cells development in gerbil dentate gurus after transient ischemia J. Cereb. Blood Flow Metab. 22:411-419.
Jankowsky, JL et al., Hum Mol Genet 13, 159 (2004).
Jin K, Sun Y, Xie L. Peel A, Mau XO, Batteur S, Greenberg DA (2003) Directed migration of neuronal precureors into the ischemic cerebral cortex and striatum. Molecular and Cellular Neuroscience 24:171-189.
Jones, L. L., Yamaguchi, Y., Stallcup, W. B. & Tuszynski, M. H. NG2 is a major chondroitin sulfate proteoglycan produced after spinal cord injury and is expressed by macrophages and oligodendrocyte progenitors. J Neurosci 22, 2792-803 (2002).
Kawauchi S, Beites CL, Crocker CE, Wu HH, Bonnin A, Murray R, Calof AL. (2004) Molecular signals regulating proliferation of stem and progenitor cells in mouse olfactory epithelium. Dev. Neurosci. 26:166-180.
Kempermann, G., Jessberger, S., Steiner, B. & Kronenberg, G. Milestones of neuronal development in the adult hippocampus. Trends Neurosci 27, 447-52 (2004).
Kempermann, G., Kuhn, H.G. & Gage, F.H. More hippocampal neurons in adult mice living in an enriched environment. Nature 386, 493-5 (1997).
Kipnis, J., Cohen, H., Cardon, M., Ziv, Y. & Schwartz, M. T cell deficiency leads to cognitive dysfunction: Implications for therapeutic vaccination for schizophrenia and other psychiatric conditions. Proc. Natl. Acad. Sci. U S A 101, 8180-8185 (2004).
Kipnis J, Nevo U, Panikashvili D, Alexandrovich A, Yoles E, Akselrod S, Shohami E, Schwartz M. (2003) Therapeutic vaccination for closed head injury. J. Neurotrauma 20(6):559-569.
Kipnis, J. and Schwartz, M. (2002) Dual Action of Glatiramer Acetate (Cop-1) as a Treatment for Autoimmune Diseases and a Vaccine for Protective Autoimmunity after CNS Injury. Trends Mol. Med. 8: 319-323.
Kipnis, J., Mizrahi, T., Yoles, E., Ben-Nun, A., Schwartz, M. & Ben-Nur, A. J Neuroimmunol 130, 78-85 (2002a).
Kipnis, J., Mizrahi, T., Hauben, E., Shaked, 1., Shevach, E. & Schwartz, M. Neuroprotective autoimmunity: naturally occurring CD4+CD25+ regulatory T cells suppress the ability to withstand injury to the central nervous system. Proc. Natl. Acad. Sci. USA 99, 15620-5 (2002b).
Kipnis J, Yoles E, Porat Z, Cohen A, Mor F, Sela M, Cohen IR, Schwartz M. (2000), T cell immunity to copolymer 1 confers neuroprotection on the damaged optic nerve: possible therapy for optic neuropathies, Proc Natl Acad Sci USA. 97:7446-7451.
Lafaille, J.J., Nagashima, K., Katsuki, M. & Tonegawa, S. High incidence of spontaneous autoimmune encephalomyelitis in immunodeficient anti-myelin basic protein T cell receptor transgenic mice. Cell 78, 399-408 (1994).
Ledeboer A, Breve JP, Poole S, Tilders FJ, Van Dam AM (2000) Interleukin-10, interleukin-4, and transforming growth factor-beta differentially regulate lipopolysaccharide-induced production of pro-inflammatory cytokines and nitric oxide in co-cultures of rat astroglial and microglial cells. Glia 30:134-141.
Lepore, A. C. & Fischer, I. Exp Neurol 194, 230-42 (2005).
Lessmann V, Gottmann K, Maclcangio M, (2003) Neurotrophin secretion: current facts and future prospect. Progress in Neurobiology 69:341-374.
Li, W.W., Setzu, A., Zhao, C., and Franklin, R.J. Minocycline-mediated inhibition of microglia activation impairs oligodendrocyte progenitor cell responses and remyelination in a non-immune model of demyelination. J Neuroimmunol 158:58-66 (2005).
Lu, P., Jones, L. L. & Tuszynski, M. H. Exp Neurol 191, 344-60 (2005). Magavi SS, Leavitt BR, Macklls J (2000) I nduction of Neurogenesis in the neocortex of adult mice. Nature, 405:951-955.
Magavi SS, Leavitt BR, Macklls J (2000) I nduction of Neurogenesis in the neocortex of adult mice. Nature, 405:951-955.
Mason, J. L., Ye, P., Suzuki, K., D'Ercole, A. J. & Matsushima, G. K. Insulin-like growth factor-1 inhibits mature oligodendrocyte apoptosis during primary demyelination. J Neurosci 20, 5703-8 (2000).
McDonald, J. W., Becker, D., Holekamp, T. F., Howard, M., Liu, S., Lu, A., Lu, J., Platik, M. M., Qu, Y., Stewart, T. & Vadivelu, S. J Neurotrauma 21, 383-93 (2004).
Merrill, J. E., Ignarro, L. J., Sherman, M. P., Melinek, J. & Lane, T. E. Microglial cell cytotoxicity of oligodendrocytes is mediated through nitric oxide. J Immunol 151, 2132-41 (1993).
Moalem, G., Leibowitz-Amit, R., Yoles, E., Mor, F., Cohen, IR. & Schwartz M Autoimmune T cells protect neurons from secondary degeneration after central nervous system axotomy. Nat. Med. 5, 49-55 (1999).
Moalem, G., Gdalyahu, A., Shani, Y., Otten, U., Lazarovici, P., Cohen, IR. & Schwartz, M.. Production of neurotrophins by activated T cells: implications for neuroprotective autoimmunity. J Autoimmun 15, 331-45 (2000).
Monje, M. L., Mizumatsu, S., Fike, J. R. & Palmer, T. D. Irradiation induces neural precursor-cell dysfunction. Nat Med 8, 955-62 (2002).
Monje, M. L., Toda, H. & Palmer, T. D. Inflammatory blockade restores adult hippocampal neurogenesis. Science 302, 1760-5 (2003).
Morris, R. JNeurosci Methods 11, 47 (1984).
Morshead, C. M. et al. Neural stem cells in the adult mammalian forebrain: a relatively quiescent subpopulation of subependymal cells. Neuron 13, 1071-82 (1994).
Nakatomi, H., Kuriu, T., Okabe, S., Yamamoto, S., Hatano, O., Kawahara, N., Tamura, A., Kirino, T. & Nakafuku, M. Cell 110, 429-41 (2002).
Ness, J. K. & Wood, T. L. Insulin-like growth factor I, but not neurotrophin-3, sustains Akt activation and provides long-term protection of immature oligodendrocytes from glutamate-mediated apoptosis. Mol Cell Neurosci 20, 476-88 (2002).
Neumann, H., Misgeld, T., Matsumuro, K., and Wekerle, H. Neurotrophins inhibit major histocompatibility class II inducibility of microglia: involvement of the p75 neurotrophin receptor. Proc. Natl. Acad. Sci. USA 95:5779-5784 (1998). Newman MP, Feron F, Mackay-Sim A (2000) Growth factor regulation of neurogenesis in adult olfactory epithelium. Neuroscience 99:343-350.
O'Kusky, J. R., Ye, P. & D'Ercole, A. J. Insulin-like growth factor-I promotes neurogenesis and synaptogenesis in the hippocampal dentate gyrus during postnatal development. J Neurosci 20, 8435-42 (2000). O'Rourke NA, Sullivan DP, Kaznowsky CE, Jacobs AA, McConnell SK (1995) Tangential migration of neurons in the developing cerebral cortex. Development 121:2165-2176.
Packer, M. A. et al. Nitric oxide negatively regulates mammalian adult neurogenesis. Proc Natl Acad Sci USA 100, 9566-71 (2003).
Pencea V, Bingaman KD, Wiegand SJ, Luskin MB (2001) Infusion of brain-derived neurotrophic factor into the lateral ventricle of the adult rat leads to new neurons in the parenchyma of the striatum, septum, thalamus, and hypothalamus. J Neurosci. 1:6706-6717.
Picard-Riera N, Decker L, Delarasse C, Goude K, Nait-Oumesmar B, Liblau R, Pham-Dinh D, Evercooren (2002) Experimental autoimmune encephalomyelitis mobilized neuronal progenitors from the subventricular zone to undergo oligodendrogenesis in adult mice. Proc. Natl. Acad. Sci. USA. 99:13211-13216.
Picard-Riera N, Nait-Oumesmar B, Baron-Van Evercooren A (2004) Endogenous adult neural stem cells: limits and potential to repair the injured central nervous system. J Neurosci. Res. 76:223-231.
Pluchino, S., Quattrini, A., Brambilla, E., Gritti, A., Salani, G., Dina, G., Galli, R., Del Carro, U., Amadio, S., Bergami, A., Furlan, R., Comi, G., Vescovi, A. L. & Martino, G. Injection of adult neurospheres induces recovery in a chronic model of multiple sclerosis. Nature 422, 688-94 (2003).
Popovich, P. G., Wei, P. & Stokes, B. T. Cellular inflammatory response after spinal cord injury in Sprague-Dawley and Lewis rats. J Comp Neurol 377, 443-64 (1997).
Popovich, P. G. et al. The neuropathological and behavioral consequences of intraspinal microglial/macrophage activation. J. Neuropathol. Exp. Neurol. 61, 623-33 (2002).
Rapalino, O., Lazarov-Spiegler, O., Agranov, E., Velan, G. J., Yoles, E., Fraidakis, M., Solomon, A., Gepstein, R., Katz, A., Belkin, M., Hadani, M. & Schwartz, M. Nat Med 4, 814-21 (1998).
Schori, H., Yoles, E. & Schwartz, M. T-cell-based immunity counteracts the potential toxicity of glutamate in the central nervous system. J. Neuroimmunol. 119, 199-204 (2001).
Schori H, Kipnis J, Yoles E, WoldeMussie E, Ruiz G, Wheeler LA, Schwartz M. (2001b) Vaccination for protection of retinal ganglion cells against death from glutamate cytotoxicity and ocular hypertension: implications for glaucoma. Proc Natl Acad Sci U S A 98:3398-3403.
Schlegel PG, Aharoni R, Chen Y, Chen J, Teitelbaum D, Arnon R, Sela M, Chao NJ. (1996) A synthetic random copolymer with promiscuous binding to class II MHC molecules inhibits T-cell proliferative response to major and minor histocompatibility antigens in vitro and confers the capacity to prevent murine graft-versus-host disease in vivo. Proc. Natl. Acad. Sci. USA. 93: 5061-6.
Schwartz, M. & Hauben, E. T cell-based therapeutic vaccination for spinal cord injury. Prog Brain Res 137, 401-6 (2002).
Schwartz, M. & Kipnis, J. Harm or heal - divergent effects of autoimmune neuroinflammation?. Response from Schwartz and Kipnis. Trends Immunol 24, 7-8 (2003).
Schwartz, M. & Kipnis, J. Autoimmunity on alert: naturally occurring regulatory CD4(+)CD25(+) T cells as part of the evolutionary compromise between a 'need' and a 'risk'. Trends Immunol. 23, 530-4 (2002).
Schwartz M, Kipnis J (2002) Multiple sclerosis as a by-product of the failure to sustain protective autoimmunity: a paradigm shift. Neuroscientist 8: 405-413.
Schwartz, M., Shaked, I., Fisher, J., Mizrahi, T. & Schori, H. Protective autoimmunity against the enemy within: fighting glutamate toxicity. Trends Neurosci. 26, 297-302 (2003).
Setoguchi, T., Nakashima, K., Takizawa, T., Yanagisawa, M., Ochiai, W., Okabe, M., Yone, K., Komiya, S. & Taga, T.Treatment of spinal cord injury by transplantation of fetal neural precursor cells engineered to express BMP inhibitor. Exp Neurol 189, 33-44 (2004).
Shaked, I., Porat, Z., Gersner, R., Kipnis, J. & Schwartz, M. Early activation of microglia as antigen-presenting cells correlates with T cell-mediated protection and repair of the injured central nervous system. J Neuroimmunol 146, 84-93 (2004).
Shaked, I., Tchoresh, D., Gersner, R., Meiri, G., Mordechai, S., Xiao, X., Hart, R. P. & Schwartz, M. J Neurochem 92, 997-1009 (2005).
Shors, T.J. et al. Neurogenesis in the adult is involved in the formation of trace memories. Nature 410, 372-6 (2001).
Shors, T.J., Townsend, D.A., Zhao, M., Kozorovitskiy, Y. & Gould, E. Neurogenesis may relate to some but not all types of hippocampal-dependent learning. Hippocampus 12, 578-84 (2002).
Snyder, J.S., Hong, N.S., McDonald, R.J. & Wojtowicz, J.M. A role for adult neurogenesis in spatial long-term memory. Neuroscience 130, 843-52 (2005).
Stollg G, Jander S (1999) The role of microglia and macrophages in the pathophsiology of the CNS. Progress in Neurology 58:233-247
van Praag, H., G. Kempermann, F. H. Gage, Nat Rev Neurosci 1, 191 (2000).
van Praag, H., Christie, B.R., Sejnowski, T.J. & Gage, F.H. Running enhances neurogenesis, learning, and long-term potentiation in mice. Proc Natl Acad Sci USA 96, 13427-31 (1999).
Yoles, E., Hauben, E., Palgi, O., Agranov, E., Gothilf, A., Cohen, A., Kuchroo, V., Cohen, 1. R., Weiner, H. & Schwartz, M. Protective autoimmunity is a physiological response to CNS trauma. J Neurosci 21, 3740-8 (2001a).
Yoles, E., Friedmann, I., Barouch, R., Shani, Y. & Schwartz, M. J Neurotrauma 18, 339-49 (2001b).
Zhao, X., Ueba, T., Christie, BR., Barkho, B., McConnell, MJ., Nakashima, K., Lein, ES., Eadie, BD., Willhoite, AR., Muotri, AR., Summers, RG., Chun, J., Lee, KF. & Gage, FH. Mice lacking methyl-CpG binding protein 1 have deficits in adult neurogenesis and hippocampal function. Proc Natl Acad Sci U S A 100, 6777-82 (2003).
Zielasek J, Tausch M, Toyka KV, Hartung HP. Production of nitrite by neonatal rat microglial cells/brain macrophages. Cell Immunol 141:111-120 (1992).

## Claims

1. A neuroprotective agent selected from Copolymer 1, a Copolymer 1-related polypeptide, a Copolymer 1-related peptide, and activated T cells which have been activated by Copolymer 1, a Copolymer 1-related polypeptide, or a Copolymer 1-related peptide, in combination with stem cell therapy with the proviso that said stem cells are not human embryonic stem cells, for use in treating an injury of the central nervous system (CNS) or peripheral nervous system (PNS), a Parkinsonian disorder such as Parkinson's disease, Huntington's disease, Alzheimer's disease, multiple sclerosis, or amyotrophic lateral sclerosis (ALS), wherein said Copolymer 1-related polypeptide/Copolymer 1-related peptide functionally cross-reacts with myelin basic protein (MBP) and is able to compete with MBP on the MHC class II in the antigen presentation.

2. The neuroprotective agent according to claim 1, for use according to claim 1, wherein said neuroprotective agent is for administration to a patient before, concomitantly or after transplantation of the stem cells to said patient.

3. The neuroprotective agent according to claims 1 or 2, for use according to claims 1 or 2, wherein said neuroprotective agent is Copolymer 1 or a pharmaceutically acceptable salt thereof.

4. The neuroprotective agent according to claims 1-3, for use according to any one of claims 1 to 3, wherein the stem cells are adult stem cells, embryonic stem cells with the proviso that said embryonic stem cells are not human embryonic stem cells, umbilical cord blood stem cells, hematopoietic stem cells, peripheral blood stem cells, mesenchymal stem cells, multipotent stem cells, neural stem cells, neural progenitor cells, stromal stem cells, progenitor cells, or precursors thereof, or genetically-engineered stem cells.

5. The neuroprotective agent according to claims 1-3 for use according to any one of claims 1 to 3, wherein said CNS injury is selected from spinal cord injury, closed head injury, blunt trauma, penetrating trauma, hemorrhagic stroke, ischemic stroke, cerebral ischemia, optic nerve injury, myocardial infarction or injury caused by tumor excision.

## Patentansprüche

1. Neuroprotektives Agens, das ausgewählt ist aus Copolymer-1, mit Copolymer-1 verwandtem Polypeptid, mit Copolymer-1 verwandtem Peptid und aktivierten T-Zellen, die durch Copolymer-1, mit Copolymer-1 verwandtem Polypeptid oder mit Copolymer-1 verwandtem Peptid aktiviert sind, in Kombination mit Stamzelltherapie, mit der Maßgabe, dass die Stammzellen keine humanen embryonalen Stammzellen sind, zur Verwendung bei der Behandlung einer Verletzung des zentralen Nervensystems (ZNS) oder peripheren Nervensystems (PNS), einer Parkinsonschen Krankheit wie Parkinson-Krankheit, Huntington-Krankheit, Alzheimer-Krankheit, Multiple Sklerose oder Amyotrophe Lateralsklerose (ALS), wobei das mit Copolymer-1 verwandte Polypeptid/mit Copolymer-1 verwandte Peptid funktionell mit Myelin-basischem Protein (MBP) kreuzreagiert und in der Lage ist, mit MBP um MHC Klasse II in der Antigenpräsentation zu konkurrieren.

2. Neuroprotektives Agens nach Anspruch 1 zur Verwendung nach Anspruch 1, wobei das neuroprotektive Agens für die Verabreichung an den Patienten vor, begleitend zu oder nach der Transplantation von Stammzellen an den Patienten ist.

3. Neuroprotektives Agens nach Anspruch 1 oder 2 zur Verwendung nach Anspruch 1 oder 2, wobei das neuroprotektive Agens Copolymer-1 oder ein pharmazeutisch verträgliches Salz davon ist.

4. Neuroprotektives Agens nach einem der Ansprüche 1 bis 3 zur Verwendung nach einem der Ansprüche 1 bis 3, wobei die Stammzellen adulte Stammzellen, embryonale Stammzellen, mit der Maßgabe, dass die embryonalen Stammzellen keine humanen embryonalen Stammzellen sind, Stammzellen aus Nabelschnurblut, hämatopoetische Stammzellen, Stammzellen des peripheren Bluts, mesenchymale Stammzellen, multipotente Stammzellen, neurale Stammzellen, neurale Vorläuferzellen, stromale Stammzellen, Progenitorzellen oder Vorläufer davon, oder genetisch modifizierte Stammzellen sind.

5. Neuroprotektives Agens nach einem der Ansprüche 1 bis 3 zur Verwendung nach einem der Ansprüche 1 bis 3, wobei die Verletzung des ZNS ausgewählt ist aus Rückenmarksverletzung, geschlossener Kopfverletzung, stumpfem Trauma, penetrierendem Trauma, hämorrhagischem Schlaganfall, ischämischem Schlaganfall, zerebraler Ischämie, Verletzung des Sehnervs, Myokardinfarkt oder einer Verletzung, die verursacht ist durch Entfernung eines Tumors.

## Revendications

1. Agent neuroprotecteur choisi parmi le Copolymère 1, un polypeptide apparenté au Copolymère 1, un peptide apparenté au Copolymère 1, et des cellules T activées qui ont été activées par le Copolymère 1, un polypeptide apparenté au Copolymère 1, ou un peptide apparenté au Copolymère 1, en combinaison avec un traitement par des cellules souches à condition que lesdites cellules souches ne soient pas des cellules souches embryonnaires humaines, pour une utilisation dans le traitement d'une lésion du système nerveux central (SNC) ou du système nerveux périphérique (SNP), d'un trouble parkinsonien comme la maladie de Parkinson, la maladie de Huntington, la maladie d'Alzheimer, la sclérose en plaques, ou la sclérose latérale amyotrophique (SLA), dans lequel ledit polypeptide apparenté au Copolymère 1/peptide apparenté au Copolymère 1 présente une réactivité croisée fonctionnelle avec la protéine basique de la myéline (PBM) et est capable d'entrer en compétition avec la PBM sur le CMH de classe II dans la présentation des antigènes.

2. Agent neuroprotecteur selon la revendication 1 pour une utilisation selon la revendication 1, dans lequel ledit agent neuroprotecteur est destiné à une administration à un patient avant, simultanément à ou après transplantation des cellules souches au dit patient.

3. Agent neuroprotecteur selon la revendication 1 ou 2 pour une utilisation selon la revendication 1 ou 2, dans lequel ledit agent neuroprotecteur est le Copolymère 1 ou un sel pharmaceutiquement acceptable de celui-ci.

4. Agent neuroprotecteur selon les revendications 1 à 3 pour une utilisation selon l'une quelconque des revendications 1 à 3, dans lequel les cellules souches sont des cellules souches adultes, des cellules souches embryonnaires à condition que lesdites cellules souches embryonnaires ne soient pas des cellules souches embryonnaires humaines, des cellules souches du sang du cordon ombilical, des cellules souches hématopoïétiques, des cellules souches du sang périphérique, des cellules souches mésenchymateuses, des cellules souches multipotentes, des cellules souches neuronales, des cellules progénitrices neuronales, des cellules souches stromales, des cellules progénitrices, ou des précurseurs de celles-ci, ou des cellules souches génétiquement modifiées.

5. Agent neuroprotecteur selon les revendications 1 à 3 pour une utilisation selon l'une quelconque des revendications 1 à 3, dans lequel ladite lésion du SNC est choisie parmi une lésion de la moelle épinière, une lésion crânienne fermée, un traumatisme contondant, un traumatisme pénétrant, un accident vasculaire cérébral hémorragique, un accident vasculaire cérébral ischémique, une ischémie cérébrale, une lésion du nerf optique, un infarctus du myocarde ou une lésion provoquée par une excision tumorale.


### SEQUENCE LISTING

<110> YEDA RESEARCH AND DEVELOPMENT CO. LTD. Eisenbach-schwartz, Michal Ruth, Arnon Butovsky, oleg Ziv, Yaniv Kipnis, Jonathan Ron , Noga Eilam, Raya Aharoni, Rina
<120> INDUCTION OF NEUROGENESIS AND STEM CELL THERAPY IN COMBINATION WITH
   COPOLYMER 1
<130> TEVA-008 PCT
<150> US 60/631,163
   <151> 2004-11-29
<150> US 60/690,498
   <151> 2005-06-15
<160> 44
<170> PatentIn version 3.1
<210> 1
   <211> 15
   <212> PRT
   <213> Artificial sequence
<220>
   <223> Synthetic
<400> 1
   Ala Ala Ala Tyr Ala Ala Ala Ala Ala Ala Lys Ala Ala Ala Ala
   1               5                   10                  15
<210> 2
   <211> 15
   <212> PRT
   <213> Artificial Sequence
<220>
   <223> Synthetic
<400> 2
   Ala Glu Lys Tyr Ala Ala Ala Ala Ala Ala Lys Ala Ala Ala Ala
   1               5                   10                  15
<210> 3
   <211> 15
   <212> PRT
   <213> Artificial Sequence
<220>
   <223> Synthetic
<400> 3
   Ala Lys Glu Tyr Ala Ala Ala Ala Ala Ala Lys Ala Ala Ala Ala
   1               5                   10                  15
<210> 4
   <211> 15
   <212> PRT
   <213> Artificial Sequence
<220>
   <223> Synthetic
<400> 4
   Ala Lys Lys Tyr Ala Ala Ala Ala Ala Ala Lys Ala Ala Ala Ala
   1 5 10 15
<210> 5
   <211> 15
   <212> PRT
   <213> Artificial Sequence
<220>
   <223> Synthetic
<400> 5
   Ala Glu Ala Tyr Ala Ala Ala Ala Ala Ala Lys Ala Ala Ala Ala
   1 5 10 15
<210> 6
   <211> 15
   <212> PRT
   <213> Artificial Sequence
<220>
   <223> Synthetic
<400> 6
   Lys Glu Ala Tyr Ala Ala Ala Ala Ala Ala Lys Ala Ala Ala Ala
   1 5 10 15
<210> 7
   <211> 15
   <212> PRT
   <213> Artificial Sequence
<220>
   <223> Synthetic
<400> 7
   Ala Glu Glu Tyr Ala Ala Ala Ala Ala Ala Lys Ala Ala Ala Ala
   1 5 10 15
<210> 8
   <211> 15
   <212> PRT
   <213> Artificial Sequence
<220>
   <223> Synthetic
<400> 8
   Ala Ala Glu Tyr Ala Ala Ala Ala Ala Ala Lys Ala Ala Ala Ala
   1 5 10 15
<210> 9
   <211> 15
   <212> PRT
   <213> Artificial Sequence
<220>
   <223> Synthetic
<400> 9
   Glu Lys Ala Tyr Ala Ala Ala Ala Ala Ala Lys Ala Ala Ala Ala
   1 5 10 15
<210> 10
   <211> 15
   <212> PRT
   <213> Artificial Sequence
<220>
   <223> Synthetic
<400> 10
   Ala Ala Lys Tyr Glu Ala Ala Ala Ala Ala Lys Ala Ala Ala Ala
   1 5 10 15
<210> 11
   <211> 15
   <212> PRT
   <213> Artificial Sequence
<220>
   <223> Synthetic
<400> 11
   Ala Ala Lys Tyr Ala Glu Ala Ala Ala Ala Lys Ala Ala Ala Ala
   1 5 10 15
<210> 12
   <211> 15
   <212> PRT
   <213> Artificial Sequence
<220>
   <223> Synthetic
<400> 12
   Glu Ala Ala Tyr Ala Ala Ala Ala Ala Ala Lys Ala Ala Ala Ala
   1 5 10 15
<210> 13
   <211> 15
   <212> PRT
   <213> Artificial Sequence
<220>
   <223> Synthetic
<400> 13
   Glu Lys Lys Tyr Ala Ala Ala Ala Ala Ala Lys Ala Ala Ala Ala
   1 5 10 15
<210> 14
   <211> 15
   <212> PRT
   <213> Artificial Sequence
<220>
   <223> synthetic
<400> 14
   Glu Ala Lys Tyr Ala Ala Ala Ala Ala Ala Lys Ala Ala Ala Ala
   1 5 10 15
<210> 15
   <211> 15
   <212> PRT
   <213> Artificial Sequence
<220>
   <223> Synthetic
<400> 15
   Ala Glu Lys Tyr Ala Ala Ala Ala Ala Ala Ala Ala Ala Ala Ala
   1 5 10 15
<210> 16
   <211> 15
   <212> PRT
   <213> Artificial Sequence
<220>
   <223> Synthetic
<400> 16
   Ala Lys Glu Tyr Ala Ala Ala Ala Ala Ala Ala Ala Ala Ala Ala
   1 5 10 15
<210> 17
   <211> 15
   <212> PRT
   <213> Artificial Sequence
<220>
   <223> Synthetic
<400> 17
   Ala Lys Lys Tyr Glu Ala Ala Ala Ala Ala Ala Ala Ala Ala Ala
   1 5 10 15
<210> 18
   <211> 15
   <212> PRT
   <213> Artificial Sequenece
<220>
   <223> Synthetic
<400> 18
   Ala Lys Lys Tyr Ala Glu Ala Ala Ala Ala Ala Ala Ala Ala Ala
   1 5 10 15
<210> 19
   <211> 15
   <212> PRT
   <213> Artificial Sequence
<220>
   <223> Synthetic
<400> 19
   Ala Glu Ala Tyr Lys Ala Ala Ala Ala Ala Ala Ala Ala Ala Ala
   1 5 10 15
<210> 20
   <211> 15
   <212> PRT
   <213> Artificial Sequence
<220>
   <223> Synthetic
<400> 20
   Lys Glu Ala Tyr Ala Ala Ala Ala Ala Ala Ala Ala Ala Ala Ala
   1 5 10 15
<210> 21
   <211> 15
   <212> PRT
   <213> Artificial Sequence
<220>
   <223> Synthetic
<400> 21
   Ala Glu Glu Tyr Lys Ala Ala Ala Ala Ala Ala Ala Ala Ala Ala
   1 5 10 15
<210> 22
   <211> 15
   <212> PRT
   <213> Artificial Sequence
<220>
   <223> Synthetic
<400> 22
   Ala Ala Glu Tyr Lys Ala Ala Ala Ala Ala Ala Ala Ala Ala Ala
   1 5 10 15
<210> 23
   <211> 15
   <212> PRT
   <213> Artificial Sequence
<220>
   <223> Synthetic
<400> 23
   Glu Lys Ala Tyr Ala Ala Ala Ala Ala Ala Ala Ala Ala Ala Ala
   1 5 10 15
<210> 24
   <211> 15
   <212> PRT
   <213> Artificial Sequence
<220>
   <223> Synthetic
<400> 24
   Ala Ala Lys Tyr Glu Ala Ala Ala Ala Ala Ala Ala Ala Ala Ala
   1 5 10 15
<210> 25
   <211> 15
   <212> PRT
   <213> Artificial Sequence
<220>
   <223> Synthetic
<400> 25
   Ala Ala Lys Tyr Ala Glu Ala Ala Ala Ala Ala Ala Ala Ala Ala
   1 5 10 15
<210> 26
   <211> 15
   <212> PRT
   <213> Artificial Sequence
<220>
   <223> Synthetic
<400> 26
   Glu Lys Lys Tyr Ala Ala Ala Ala Ala Ala Ala Ala Ala Ala Ala
   1 5 10 15
<210> 27
   <211> 15
   <212> PRT
   <213> Artificial sequence
<220>
   <223> synthetic
<400> 27
   Glu Ala Lys Tyr Ala Ala Ala Ala Ala Ala Ala Ala Ala Ala Ala
   1 5 10 15
<210> 28
   <211> 15
   <212> PRT
   <213> Artificial Sequence
<220>
   <223> Synthetic
<400> 28
   Ala Glu Tyr Ala Lys Ala Ala Ala Ala Ala Ala Ala Ala Ala Ala
   1 5 10 15
<210> 29
   <211> 15
   <212> PRT
   <213> Artificial Sequence
<220>
   <223> Synthetic
<400> 29
   Ala Glu Lys Ala Tyr Ala Ala Ala Ala Ala Ala Ala Ala Ala Ala
   1 5 10 15
<210> 30
   <211> 15
   <212> PRT
   <213> Artificial sequence
<220>
   <223> Synthetic
<400> 30
   Glu Lys Tyr Ala Ala Ala Ala Ala Ala Ala Ala Ala Ala Ala Ala
   1 5 10 15
<210> 31
   <211> 15
   <212> PRT
   <213> Artificial Sequence
<220>
   <223> Synthetic
<400> 31
   Ala Tyr Lys Ala Glu Ala Ala Ala Ala Ala Ala Ala Ala Ala Ala
   1 5 10 15
<210> 32
   <211> 15
   <212> PRT
   <213> Artificial Sequence
<220>
   <223> Synthetic
<400> 32
   Ala Lys Tyr Ala Glu Ala Ala Ala Ala Ala Ala Ala Ala Ala Ala
   1 5 10 15
<210> 33
   <211> 25
   <212> DNA
   <213> Artificial Sequence
<220>
   <223> Synthetic
<400> 33
   aggaggcgct ccccaaaaag atggg 25
<210> 34
   <211> 25
   <212> DNA
   <213> Artificial Sequence
<220>
   <223> synthetic
<400> 34
   gtacatgggc tcataccagg gcttg 25
<210> 35
   <211> 20
   <212> DNA
   <213> Artificial Sequence
<220>
   <223> Synthetic
<400> 35
   caggctccta gcatacctgc 20
<210> 36
   <211> 20
   <212> DNA
   <213> Artificial Sequence
<220>
   <223> Synthetic
<400> 36
   gctggtaaag gtgagcaagc 20
<210> 37
   <211> 24
   <212> DNA
   <213> Artificial Sequence
<220>
   <223> Synthetic
<400> 37
   ttgtaaccaa ctgggacgat atgg 24
<210> 38
   <211> 24
   <212> DNA
   <213> Artificial Sequence
<220>
   <223> Synthetic
<400> 38
   gatcttgatc ttcatggtgc tagg 24
<210> 39
   <211> 21
   <212> PRT
   <213> Artificial sequence
<220>
   <223> Synthetic
<400> 39
   Met Glu Val Gly Trp Tyr Arg Ser Pro Phe Ser Arg Val Val His Leu
   1 5 10 15
   Tyr Arg Asn Gly Lys
   20
<210> 40
   <211> 21
   <212> PRT
   <213> Artificial Sequence
<220>
   <223> Synthetic
<400> 40
   Met Glu Val Gly Trp Tyr Arg Ser Pro Phe Asp Arg Val Val His Leu
   1 5 10 15
   Tyr Arg Asn Gly Lys
   20
<210> 41
   <211> 28
   <212> DNA
   <213> Artificial Sequence
<220>
   <223> Synthetic
<400> 41
   gtggataccc cctcccccag cctagacc 28
<210> 42
   <211> 30
   <212> DNA
   <213> Artificial Sequence
<220>
   <223> Synthetic
<400> 42
   cctctttgtg actatgtgga ctgatgtcgg 30
<210> 43
   <211> 20
   <212> DNA
   <213> Artificial Sequence
<220>
   <223> Synthetic
<400> 43
   aatagagaac ggcaggagca 20
<210> 44
   <211> 20
   <212> DNA
   <213> Artificial Sequence
<220>
   <223> Synthetic
<400> 44 20
   gccatgaggg cactaatcat 20